(19) **Europäisches Patentamt**
**European Patent Office**
**Office européen des brevets**

(11) **EP 2 369 349 A2**

(12) **EUROPEAN PATENT APPLICATION**

(43) Date of publication:
**28.09.2011  Bulletin 2011/39**

(51) Int Cl.:
***G01N 33/68*** *(2006.01)*  ***G01N 33/574*** *(2006.01)*

(21) Application number: **11164150.2**

(22) Date of filing: **10.10.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **11.10.2005  EP 05384037**

(62) Document number(s) of the earlier application(s) in accordance with Art. 76 EPC:
**06792410.0 / 1 934 616**

(71) Applicant: **Laboratorios SALVAT, S.A.**
**08950 Esplugues de Llobregat, Barcelona (ES)**

(72) Inventors:
• **Soloaga Villoch, Ana**
**48013 Bilbao (ES)**
• **Castrillo Diez, José Luis**
**48993 Getxo (ES)**

• **Ramos Rodriguez, Inma**
**48006 Bilbao (ES)**
• **Escuredo Garcia-Galdeano, Kepa**
**48003 Bilbao (ES)**
• **Martinez Martinez, Antonio**
**48100 Laukariz (ES)**
• **Simón Buela, Laureano**
**20018 San Sebastiàn (ES)**

(74) Representative: **HOFFMANN EITLE**
**Patent- und Rechtsanwälte**
**Arabellastraße 4**
**81925 München (DE)**

Remarks:
•This application was filed on 28-04-2011 as a divisional application to the application mentioned under INID code 62.
•Claims filed after the date of filing of the application/ after the date of receipt of the divisional application (Rule 68(4) EPC).

(54) **Non-invasive in vitro method to detect transitional cell carcinoma of the bladder**

(57)    The present invention refers to a non-invasive in vitro method to detect a transitional cell carcinoma of the bladder in an individual via urine analysis, to determine the stage or severity of this cancer in an individual or to monitor the effect of treatment administered to an individual suffering from this cancer.

EP 2 369 349 A2

**Description**

FIELD OF THE INVENTION

The present invention refers to a non-invasive *in vitro* method to detect the presence of transitional cell carcinoma of the bladder in the urine of an individual via urine analysis, as well as to the use of peptide sequences derived from selected proteins and to a kit to perform the method.

BACKGROUND OF THE INVENTION

Bladder cancer is the most common cancer of the urinary tract; it is also the fourth most common cancer in men and the eighth most common in women. It includes a broad spectrum of tumors of various histological types including transitional cell carcinoma of the bladder (BTCC, 90%), squamous cell carcinoma (7%), adenocarcinoma (2%), and undifferentiated carcinoma (1%).

Tumor grade and stage are the best prognostic indicators of BTCC. Bladder tumors are graded cytomorphologically from G1 (low grade) to G3 (high grade) in decreasing state of differentiation and increasing aggressiveness of the disease according to the World Health Organization (WHO). With respect to stage or invasiveness, BTCCs are classified as superficial papillary (Ta and T1), muscle invasive (T2 to T4), or the uncommon carcinoma *in situ* or tumor *in situ* (TIS).

Low-grade tumors are usually confined to the mucosa or infiltrate superficial layers (stage Ta and T1). Most high-grade tumors are detected at least at T1 stage (invading lamina propria). Approximately 75% of the diagnosed bladder cancer cases are superficial. The remaining 25% are muscle invasive at the moment of diagnosis. Patients with superficial BTCC have a good prognosis but have a 70% risk of recurrence. In spite of this high risk, Ta tumors tend to be low grade and only 10-15% will progress to muscle invasion in 2 years. However, the percentage of T1 tumors that progresses to T2 stage is higher (30-50%).

Currently, the best diagnostic system for bladder cancer in individuals is established either by cystoscopy and transurethral biopsy or by resection, both representing invasive procedures.

Flexible cystoscopes make the technique less aggressive, but it remains invasive and highly unpleasant, still requiring some form of anaesthesia.

The prevailing non-invasive technique for diagnosis of BTCC is to identify neoplastic cells by morphological examination of the cells in urine. Thus, cytology is currently used to monitor patients diagnosed with and treated for bladder cancer. Although urine cytology can detect tumors *in situ* that are not detectable by cystoscopy, as well as tumors located in the upper end of the bladder or the upper urinary tract, i.e. ureter, pelvis and kidney, several studies have shown that cytology has a very low sensitivity in bladder cancer diagnosis, missing up to 50% of tumors. Findings from cytologies are subtle and can be confused with reactive or degenerative processes. Cytology studies require expert, individual evaluation, which delays the availability of the results and further introduces subjectivity and variance to the final results. Actually, there is no highly sensible and specific non-invasive method available to diagnose bladder cancer (Boman H. et al., J Urol 2002, 167:80-83).

Many efforts have been made to improve non-invasive cancer detection. Recent advances in expression profiling of cancer cells by proteomic technologies, high resolution two dimensional electrophoresis and mass spectrometry have made it possible to identify candidate proteins as tumor markers for bladder cancer, such as nuclear matrix protein NMP22 (Soloway M.S. et al., J Urol 1996, 156:363-367), hyaluronic acid and hyaluronidase (Pham H.T. et al., Cancer Res 1997, 57:778-783), basement membrane complexes (BTA, Pode D. et al., J Urol 1999, 161:443-446), carcinoembryonic antigen (CEA, Halim A.B. et al., Int J Biol Markers, 1992; 7:234-239), uroplakin II (Wu X.R. et al., Cancer Res 1998; 58:1291-1297), scatter factor/hepatocyte growth factor (SF/HGF, Gohji K. et al., J Clin Oncol 2000; 18:2963-2971), proteins of the keratin/cytokeratin family like cytokeratin 20 (Buchumensky V. et al., J Urol 1998, 160:1971-1974), cytokeratin 18 (Sánchez-carbayo M. et al., Clin Cancer Res 2000, 6:3585-3594), mammary tumor 8-Ka protein (MAT-8, Morrison B.W. et al., J Biol Chem 1995, 270:2176-2182) and telomerase (Lee D.H. et al., Clinical Cancer Research 1998, 4: 535-538).

Memon A. A. et al. (Cancer Detect Prev 2005, 29:249-255) identify seven differentially expressed proteins in bladder cancer from cell lines and biopsies. However no control is used for comparison. Langbein, S. et al (Technol Cancer Res Treat 2006, 67-71) disclose the protein profiling of biopsies from bladder cancer patients using the 2D-PAGE and SELDI-TOF-MS technique,

Rasmussen H. H. et al. (J Urol 1996, 155 :2113-2119) disclose a database of the most abundant proteins present in the urine of patients with bladder cancer. Celis J. E. et al (Electrophoresis 1999, 300-309) describe a database of proteins present in biopsies from bladder cancer patients. Nevertheless, no markers are identified in any of these references, since the authors do not show a quantified differential profile of the proteins in healthy samples vs tumoral samples.

Further, no marker to predict the prognosis and extent of bladder cancer has been proven useful in clinical trials (Miyake H. et al., J Urol 2002, 167:1282-1287).

DESCRIPTION OF THE INVENTION

The current invention provides a highly sensitive, efficient and rapid non-invasive *in vitro* method concerning proteins associated with BTCC. It further concerns a method for the diagnosis, prognosis and monitoring of BTCC via analysis of urine samples. The current invention surprisingly provides bladder cancer biomarkers for detection of BTCC via urine analysis. A preferred embodiment of the invention concerns 40 bladder cancer biomarkers for detection of BTCC via urine analysis that are of significant value for the detection, diagnosis, prognosis and/or monitoring of BTCC.

Aminoacylase-1 (ACY1) is a cytosolic, homodimeric, zinc-binding enzyme that catalyzes the hydrolysis of acylated L-amino acids to L-amino acids and acyl group, and has been postulated to function in the catabolism and salvage of acylated amino acids. ACY1 has been assigned to chromosome 3p21.1, a region reduced to homozygosity in small-cell lung cancer (SCLC), and its expression has been reported to be reduced or undetectable in SCLC cell lines and tumors, ACY1 is the first member of a new family of zinc-binding enzymes.

Aldehyde reductase (AKR1A1) belongs to the aldo/keto reductase family, it is involved in the reduction of biogenic and xenobiotic aldehydes and is present in virtually every tissue.

Aldoiase B (ALDOB), also called fructose 1-phosphate aldolase, is produced in the liver, kidneys and brain. It is needed for the breakdown of fructose.

Alpha-amylase (AMY) carcinoid, pancreatic alpha-amylase and alpha amylase 1A belong to the glycosyl hydrolase 13 family and hydrolyze 1,4-alpha-glucoside bonds in oligosaccharides and polysaccharides, and thus catalyze the first step in digestion of dietary starch and glycogen. The human genome has a cluster of several amylase genes that are expressed at high levels in either salivary gland or pancreas.

Annexin IV (ANXA4) belongs to the annexin family of calcium-dependent phospholipid binding proteins. Although their functions are still not clearly defined, several members of the annexin family have been implicated in membrane-related events along exocytotic and endocytotic pathways. ANXA4 is almost exclusively expressed in epithelial cells.

Apolipoprotein A-I (APOA1) belongs to the apolipoprotein A1/A4/E family and participates in the reverse transport of cholesterol from tissues to the liver for excretion by promoting chloesterol efflux from tissues and by acting as a cofactor for the lecithin cholesterol acyltransferase (LCAT).

Biliverdin reductase A (BIEA) belongs to the gfo/idh/moca family and biliverdin reductase subfamily and reduces the gamma-methene bridge of the open tetrapyrrole, biliverdin IX alpha, to bilirubin with the concomitant oxidation of NADH or NADPH.

Flavin reductase (BLVRB) catalyzes electron transfer from reduced pyridine nucleotides to flavins as well as to methylene blue, pyrroloquinoline quinone, riboflavin, or methemoglobin. BLVRB has a possible role in protecting cells from oxidative damage or in regulating iron metabolism. In the liver, it converts Biliverdin to bilirubin. It is predominantly expressed in liver and erythrocytes, and at lower levels in heart, lung, adrenal gland and cerebrum.

Cathepsin D (CATD) is an acid protease which belongs to the peptidase A1 family and activates intracellular protein breakdown. It is involved in the pathogenesis of several diseases such as bladder cancer (Ozer E., et al., Urology 1999, 54:50-5 and loachim E., et al., Anticancer Res 2002, 22:3383-8) breast cancer and possibly Alzheimer's disease. Cathepsin D is synthesized as an inactive precursor of 52 kDa, formed of two polypeptides of 14 kDa (CATD H) and one of 34 kDa (CATD K). The inactive peptide is activated by proteolysis of the N-terminus resulting in the enzymatically active protein of 48 kDa

Complement C3 precursor (CO3) plays a central role in the activation of the complement system. Its processing by C3 convertase is the central reaction in both complement pathways. CO3 has been related to urogenital tumors (Dunzendorfer U., et al., Eur Urol 1980, 6:232-6).

Cytosolic nonspecific dipeptidase (CPGL1) belongs to the peptidase M20A family and is also known as tissue camosinase and peptidase A. It is a nonspecific dipeptidase rather than a selective carnosinase.

Alpha enolase (ENOA) is a multifunctional enzyme that, as well as its role in glycolysis, plays a part in various processes such as growth control, hypoxia tolerance and allergic responses. It may also function in the intravascular and pericellular fibrinolytic system due to its ability to serve as a receptor and activator of plasminogen on the cell surface of several cell types such as leukocytes and neurones. Mammalian enolase is composed of 3 isozyme subunits, alpha, beta and gamma, which can form homodimers or heterodimers which are cell type and development-specific, ENOA interacts with PLG in the neuronal plasma membrane and promotes its activation. It is used as a diagnostic marker for many tumors and, in the heterodimeric form, alpha/gamma, as a marker for hypoxic brain injury after cardiac arrest. It has been related to bladder cancer (Iczkowski K.A., et al., Histopathology 1999, 35:150-6).

Ferritin light chain (FRIL) belongs to the ferritin family and is the major intracellular iron storage protein in prokaryotes and eukaryotes. It is composed of 24 subunits of the heavy and light ferritin chains. Variation in ferritin subunit composition may affect the rates of iron uptake and release in different tissues. A major function of ferritin is the storage of iron in a soluble and non-toxic state.

Rab GDP dissociation inhibitor beta (GDIB) belongs to the rab gdi family and regulates the GDP/GTP exchange reaction of members of the rab family, small GTP-binding proteins of the ras superfamily, that are involved in vesicular trafficking of molecules between cellular organelles. GDIB is ubiquitously expressed. Plasma glutathione peroxidase (GPX3) belongs to the glutathione peroxidase family and catalyzes the reduction of hydrogen peroxide, organic hydroperoxide, and lipid peroxides by reduced glutathione and functions in the protection of cells against oxidative damage. Human plasma glutathione peroxidase has been shown to be a selenium-containing enzyme. GPX3 expression appears to be tissue-specific.

Gluthatione synthetase (GSHB) is important for a variety of biologic functions, including protection of cells from oxidative damage by free radicals, detoxification of xenobiotics and membrane transport.

Gelsolin (GSN40 and GSN80) is a calcium-regulated, actin-modulating protein that binds to the plus ends of actin monomers or filaments, preventing monomer exchange (end-blocking or capping). It can promote the assembly of monomers into filaments (nucleation), as well as sever existing filaments. In addition, this protein binds to actin and to fibronectin. It has been related to bladder cancer, although it has not been described as a biomarker, that is, its expression level has not been compared and quantified in healthy individuals vs cancer patients (Rasmussen, H. H. et al., J Urol 1996, 155:2113-2119; Haga K, Hokkaido Igaku Zasshi 2003, 78:29-37; Celis A et al., Electrophoresis 1999, 20:355-61).

Glutathione S-transferase (GSTP1) belongs to a family of enzymes that play an important role in detoxification by catalysing the conjugation of many hydrophobic and electrophilic compounds with reduced glutathione.

Cytoplasmic isocitrate dehydrogenase (IDHC) belongs to the isocitrate and isopropylmalate dehydrogenase family and catalyzes the oxidative decarboxylation of isocitrate to form alpha-ketoglutarate.

Vesicular integral -membrane protein VIP36 precursor (LMAN2) plays a role as an intracellular lectin in the early secretory pathway. Interacts with N-acetyl-D-galactosamine and high-mannose type glycans and may also bind to O-linked glycans. It is involved in the transport and sorting of glycoproteins carrying high mannose-type glycans.

Lymphocyte antigen 6 complex, locus 6 G6E (LY6G6E) belongs to the Lymphocyte antigen-6 superfamily. Members of this family are cystein-rich, generally GPI-anchored cell surface proteins, which have definite or putative immune related roles. Its specific function is unknown. Mannan-binding lectin serine protease 2, precursor (MASP2) belongs to the peptidase s1 family and is a trypsin protease that presumably plays an important role in the initiation of the mannose binding lectin (MBL) complement activation pathway. After activation it cleaves C4 generating C4A and C4B.

Nicotinate-nucleotide pyrophosphorylase (NADC) belongs to the nadC/mod D family and is an intermediate in the *de novo* synthesis pathway of NAD from tryptophan and acts as a potent endogenous excitotoxin through hyperstimulation of N-methyl D-aspartate (NMDA) receptor in the neuron. Elevation of NADC levels in the brain has been linked to the pathogenesis of neurodegenerative disorders such as epilepsy, Alzheimer's disease and Huntington's disease. It has not been related to cancer yet.

Napsin A (NAPSA) belongs to the peptidase a1 family and it is expressed at the highest levels in the kidney, at a moderate level in the lung, and at low levels in the spleen and adipose tissue. It may be involved in processing of pneumocyte surfactant precursors.

Proliferation-associated protein 2G4 (PA2G4) is a single-stranded DNA-binding protein showing cell cycle specific variation in nuclear localization, which belongs to the peptidase m24c family. As the protein is expressed in response to mitogen stimulation, it may belong to a large family of cell cycle regulatory proteins or replication proteins that maintain the cell cycle activities of proliferating cells.

Oncogene DJ1 (PARK7) is highly expressed in pancreas, kidney, skeletal muscle, liver, testis and heart and acts as positive regulator of androgen receptor-dependent transcription. It may function as redox-sensitive chaperone and as sensor for oxidative stress. It prevents aggregation of SNCA and protects neurons against oxidative stress and cell death.

Poly(rc)-binding protein 1 (PCPB1) is a single stranded nucleic acid binding protein that binds preferentially to oligo dC. It may shuttle between the nucleus and the cytoplasm. It is abundantly expressed in skeletal muscle, thymus and peripheral blood leucocytes while a lower expression is observed in prostate, spleen, testis, ovary, small intestine, heart, liver, adrenal and thyroid glands.

Programmed cell death 6-interacting protein (PDC6l) is expressed in brain, heart, placenta, lung, kidney, pancreas, liver, skeletal muscle and cochlea and may play a role in the regulation of both apoptosis and cell proliferation. It interacts with PDCD6/ALG-2 and SH3KBP1.

Lysosomal acid phosphatase precursor (PPAL) belongs to the histidine acid phophatase family and hydrolyzes orthophosphoric monoesters to alcohol and phosphate. Peroxiredoxin 2 (PRDX2) belongs to the ahpc/tsa family and is involved in redox regulation of the cell. It reduces peroxides with reducing equivalents provided through the thioredoxin system. It is not able to receive electrons from glutaredoxin. It may play an important role in eliminating peroxides generated during metabolism. It might participate in the signalling cascade of growth factors and tumor necrosis factor-alpha by regulating the intracellular concentrations of $H_2O_2$. It enhances natural killer (NK) cells activity.

Glutaminyl-peptide cyclotransferase (QPCT) is responsible for the biosynthesis of pyroglutamyl peptides. It has a bias against acidic and tryptophan residues adjacent to a N-terminal glutamic acid.

Plasma retinol-binding protein (RETBP) belongs to the lipocalin family and is the specific carrier for retinol (vitamin A alcohol) in the blood. It delivers retinol from the liver stores to the peripheral tissues, In plasma, the RBP-retinol complex interacts with transthyretin, which prevents its loss by filtration through the kidney glomeruli. A deficiency of vitamin A blocks secretion of the binding protein posttranslationally and results in defective delivery and supply to the epidermal cells.

Selenium-binding protein (SBP1) belongs to the selenium-binding protein family. Selenium is an essential nutrient that exhibits potent anticarcinogenic properties; a deficiency in selenium may cause certain neurologic diseases. It has been proposed that the effects of selenium in preventing cancer and neurologic diseases may be mediated by selenium-binding proteins.

Stress-induced-phosphoprotein 1 (STIP1) is an adaptor protein that mediates the association of the molecular chaperones HSC70 and HSP90 (HSPCA and HSPCB).

Transaldolase (TALDO) belongs to the transaldolase family and is a key enzyme of the nonoxidative pentose phosphate pathway providing ribose-5-phosphate for nucleic acid synthesis and NADPH for lipid biosynthesis. This pathway can also maintain glutathione at a reduced state and thus protect sulfhydryl groups and cellular integrity from oxygen radicals.

Transthyretin (TTHY, formerly prealbumin) is one of 3 thyroid hormone-binding proteins found in the blood of vertebrates. It is produced in the liver and circulates in the bloodstream, where it binds retinol and thyroxine.

WD repeat protein 1 (WDR1) belongs to the wd-repeat aip1 family and induces dissassembly of actin filaments in conjunction with ADF/cofilin family proteins.

Cancer can be detected by analysing the expression pattern of at least one bladder cancer biomarker in a test urine sample. Thus, detection of at least one differentially expressed protein in a urine test sample in comparison to normal urine is indicative of BTCC.

Urine samples are very diverse in composition. Therefore, normalization is essential to account for differences in total protein concentration and to remove bias from sample to sample.

The expression levels of a control protein, whose content in urine is always constant, may be used to normalize signal levels. In the literature there are many examples of these non-variable proteins. In the present invention, transferrin was shown to be a non-variable protein.

Representative bladder cancer proteins or biomarkers identified in the present invention include, but are not limited to TTHY, ACY1, AKR1A1, ALDOB, ANXA4, BIEA, BLVRB, CATD H, CATD K, CO3 CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC6l, PPAL, PRDX2, PTD012, QPCT, SBP1, STIP1, TALDO, WDR1, AMY, APOA1, GSN40, GSN80 and RETBP (also referred as to the 40 bladder cancer biomarkers).

One aspect of the present invention relates to a non-invasive *in vitro* method that comprises: a) detecting and quantifying one or more biomarkers present in a urine test sample from an individual; and b) comparing the expression measurement obtained in a) in the test urine sample to the corresponding standard value in normal urine, wherein variations in the measurement obtained in a) compared to the corresponding standard value in normal urine are indicative of BTCC. This method can be adapted for screening to detect the presence of BTCC, to determine the stage or severity of this cancer.

In addition, this method may also be employed to assess the lack of disease after surgical resection, to establish the diagnosis and/or prognosis of this cancer and/or to monitor the effect of the treatment administered to an individual suffering from said cancer.

Another aspect of the invention relates to the use of one or more peptide sequences derived from biomarkers present in urine to detect the presence of BTCC via urine analysis, to determine the stage or severity of this cancer, to assess the lack of disease after surgical resection, to establish the diagnosis and/or prognosis of this cancer and/or to monitor the effect of the treatment administered to an individual suffering from said cancer.

Another aspect of the invention relates to the use of one or more nucleotides or peptide sequences derived from the 40 bladder cancer biomarkers or a transcriptional or a post-translational variant thereof, alone or in any combination, in methods to screen for, identify, develop and evaluate the efficacy of therapeutic agents to treat BTCC.

The invention additionally provides antibodies directed against such bladder cancer biomarkers. These antibodies may be provided in a variety of forms, as appropriate for a particular use, including, for example, in a soluble form, immobilized on a substrate, or in combination with a pharmaceutically acceptable carrier.

Another aspect of the invention relates to a kit to perform the method as previously defined comprising 1) at least one antibody that specifically recognises one cancer biomarker in urine and 2) a carrier in a suitable packaging.

Another aspect of the invention relates to a BTCC reference expression profile, comprising a pattern of expression of one or more bladder cancer biomarkers. Different BTCC reference expression profiles may be established and correlated to different cancer stages.

For the purposes of the present invention the following definitions have been used:

The term "cancer" refers to the disease that is typically characterised by abnormal or unregulated cell growth, capable of invading adjacent tissues and spreading to distant organs. The term "carcinoma" refers to the tissue resulting from abnormal or unregulated cell growth. The term "transitional cell carcinoma of the bladder" or its abbreviation "BTCC" refer to any malign proliferative disorder in bladder epithelial cells. The term "tumor" refers to any abnormal mass of tissue generated by a neoplastic process, whether this is benign (non cancerous) or malignant (cancerous). The term "bladder cancer proteins or biomarkers" refers to differentially expressed proteins in BTCC, that is, proteins which are expressed differently in the urine from a healthy individual as compared to the urine from a patient having BTCC. In the present invention the group of proteins comprised by differentially expressed proteins includes both the 40 biomarkers selected from the group comprising TTHY, ACY1, AKR1A1, ALDOB, ANXA4, BIEA, BLVRB, CATD H, CATD K, CO3 CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC6l, PPAL, PRDX2, PTD012, QPCT, SBP1, STIP1, TALDO, WDR1, AMY, APOA1, GSN40, GSN80 and RETBP, as well as any protein of a urine sample, whose ratio changes at least two-fold when comparing two different urine samples: one from a healthy individual and one from a patient of transitional cell carcinoma, this quantification being carried out by an image analyzer software, for example Progenesis PG220 software. Bladder cancer biomarkers as described herein may be of any length and further comprise additional sequences derived from the native protein and/or heterologous sequences, any transcriptional or post-translational variants, as well as any sequence with at least 95% identity with the described sequences, wherein identity is defined as the percentage of residues which are identical between two sequences. Those skilled in the art will appreciate that these other portions or variants may be also useful in the treatment and detection of cancer. The term "detecting one or more biomarkers" means determining the existence of one or more biomarkers, whereas the term "quantifying one or more biomarkers" means expressing the presence of one or more biomarkers as a value (for instance as an intensity value). The term "indicative of BTCC" means that variations found in the measurement of one or more biomarkers in a test urine sample as compared to the corresponding standard value in normal urine serve as a proof of the presence of BTCC. The term "variation" refers to a change in the level of expression of a protein.

The term "differentially expressed protein" refers to a protein, whose expression pattern varies (increases or decreases) in the urine from a patient having BTCC as compared to the urine from a healthy individual, The term "inversely expressed" refers to two differentially expressed proteins whose expression patterns are opposite (that is, in a sample one is overexpressed while the other is repressed or vice versa).

The term "protein extract" corresponds to the supernatant obtained after centrifugation of the urine sample.

The term "individual" refers to all species of animals classified as mammals and includes, but is not restricted to, domestic and farm animals, primates and humans, and preferably refers to a male or female human of any age or race. The term "healthy individual" refers to an individual who does not suffer from BTCC and may include patients having other urologic diseases. The term "previously diagnosed" relates to an individual who has already received a first positive diagnostic for BTCC. The term "not previously diagnosed" relates to an individual who has never received a first positive diagnostic for BTCC (de novo diagnostic).

The term "standard value in normal urine" relates to the quantification of the average expression level of one or more biomarkers detected in single urine samples of individuals known to not be suffering from BTCC.

The term "diagnosis" of BTCC relates to the process of identifying or determining the nature and cause of BTCC through evaluation of one or more BTCC biomarkers. The term "prognosis" refers to the likely outcome or course of a disease, that is the chance of recovery or recurrence. The term "monitoring" means to assess the presence or absence of BTCC in an individual at different times. The term "treatment" refers to any process, action, application or the like, wherein an individual is subject to medical aid with the object of improving his condition, directly or indirectly. The term "specificity" refers to the ability of a test to exclude the presence of a disease when it is truly not present. Specificity is expressed as the number of healthy individuals for whom there is a correct negative test (this group being called true negatives), divided by the sum of true negatives and the number of healthy individuals for whom there is an incorrect positive test (this group being called false positives). The term "sensitivity" refers to the ability of a test to detect a disease when it is truly present. Sensitivity is expressed as the number of diseased patients for whom there is a correct positive test (this group being called true positives), divided by the sum of true positives and the number of diseased patients for whom there is an incorrect negative test (this group being called false negatives). The term "robustness" defines the ability of the numerical method to provide the same result despite variabilities in the initial samples.

The term "gene" refers to a region of a molecular chain of deoxyribonucleotides that encodes a protein and may represent a portion of a coding sequence or a complete coding sequence. The term "protein" refers to at least one molecular chain of amino acids linked intermolecularly through either covalent or non-covalent bonds. The term includes all forms of post-translational protein modifications, for example glycosylation, phosphorylation or acetylation. The terms "peptide" and "polypeptide" refer to molecular chains of amino acids that represent a protein fragment. The terms "protein" and "peptide" are indistinguishable.

The term "antibody" refers to a Y-shaped protein (known as immunoglobulin) on the surface of B cells that is secreted into the blood or lymph in response to an antigenic stimulus, such as an exogenous protein, bacterium, virus, parasite, or transplanted organ, and that exhibits a specific binding activity for a target molecule called an "antigen". The antigen binding region of immunoglobulins can be divided into either F(ab')$_2$ or Fab fragments. The term "antibody" includes monoclonal and polyclonal antibodies, either intact or fragments derived from them; and includes human antibodies, humanized antibodies and antibodies of non-human origin. A "non-human antibody" is an antibody generated by an animal species other than *Homo sapiens.* A "humanized antibody" is a genetically engineered antibody in which the minimal mouse part from a murine antibody is fused to a human antibody. Generally, humanized antibodies are 5-10% mouse and 90-95% human. A "human antibody" is an antibody derived from a transgenic mice carrying human antibody genes or from human cells. The "monoclonal antibodies" are homogeneous, highly specific antibody populations directed against a single antigenic site or "determinant" of the target molecule. "Polyclonal antibodies" include heterogeneous antibody populations that are directed against different antigenic determinants of the target molecule. The term "specific antibody" refers to an antibody generated against a specific protein (in this case against a particular bladder cancer marker). The term "antibody-protein complex" refers to a complex formed by an antigen and its specific antibody. The term "combibody" (combinatorial antibody) refers to an antibody displayed on filamentous phages, which allows direct screening of cDNA libraries for expression of cell-surface-reactive antibodies, without the need for antibody production and purification using bacteria or eukaryotic cell systems. The term "Fab recombinant antibody" refers to a recombinant antibody that only contains the Fab fragment that is univalent and useful when the antibody has a very high affinity for its antigen. They can be recombinantly obtained if the protein sequence is known. The term "ScFv antibody fragment" refers to a single chain variable fragment (scFv) that can be expressed in bacterial cultures.

The term "epitope" refers to an antigenic determinant of a protein, which is the sequence of amino acids of the protein that a specific antibody recognises. Such epitopes may be comprised of a contiguous stretch of amino acids (linear epitope) or of non-contiguous amino acids that are brought into proximity with one another by virtue of the three dimensional folding of the polypeptide chain (discontinuous epitopes). The term "solid phase" refers to a non-aqueous matrix to which the antibody can be bound. Examples of materials for the solid phase include but are not limited to glass, polysaccharides (for example agarose), polyacrylamide, polystyrene, polyvinylic alcohol and silicons. Examples of solid phase forms are the well of a plate or a purification column.

The term "dipstick" refers to a device dipped into a liquid to perform some kind of test that may determine and/or quantify some properties of the liquid (chemical, physical, etc). This kind of dipstick is usually made of paper or cardboard and is impregnated with reagents whose colour changes indicate some feature of the liquid. The term "carrier" refers to a mechanism or device by which something is conveyed or conducted. The term "packaging" refers to the containment and packing prior to sale with the primary purpose of facilitating the purchase and use of a product.

The term "biochip" refers to a collection of miniaturized test sites (microarrays) arranged on a solid substrate that permits many tests to be performed at the same time in order to achieve higher throughput and speed.

In one embodiment of the invention, the first step of the method to assess bladder cancer comprises measuring one or more biomarkers selected from TTHY, ACY1, AKR1A1, ALDOB, ANXA4, BIEA, BLVRB, CATD H, CATD K, CO3, CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC6l, PPAL, PRDX2, PTD012, QPCT, SBP1, STIP1, TALDO, WDR1, AMY, APOA1, GSN40, GSN80 and RETBP (also referred as to the 40 bladder cancer biomarkers) or a transcriptional or a post-translational variant thereof in a urine test sample from an individual.

In another embodiment, the first step of the method to assess bladder cancer comprises measuring one or more biomarkers selected from TTHY, ACY1, AKR1A1, ALDOB, ANXA4, BIEA, BLVRB, CATD H, CATD K, CO3, CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC6l, PPAL, PRDX2, PTD012, QPCT, SBP1, STIP1, TALDO and WDR1 or a transcriptional or a post-translational variant thereof in a urine test sample from an individual.

In another embodiment, the first step of the method to assess bladder cancer comprises measuring at least two biomarkers or a transcriptional or a post-translational variant thereof in the test urine sample. In another embodiment, the first step of the method to assess bladder cancer comprises measuring at least three biomarkers or a transcriptional or a post-translational variant thereof in the test urine sample. In another embodiment, the first step of the method to assess bladder cancer comprises measuring at least four biomarkers or a transcriptional or a post-translational variant thereof in the test urine sample. In another embodiment, the first step of the method to assess bladder cancer comprises measuring at least five biomarkers or a transcriptional or a post-translational variant thereof in the test urine sample.

In another embodiment, the first step of the method to assess bladder cancer comprises measuring one or more biomarkers selected from TTHY, ACY1, AKR1A1, ANXA4, BIEA, BLVRB, CATD H, CATD K, CO3, CPGL1, ENOA, FRIL, GDIB, GPX3, GSHB, GSTP1, IDHC, LY6G6E, MASP2, NADC, NAPSA, PA2G4, PARK7, PCBP1, PRDX2, PTD012, QPCT, SBP1, STIP1, AMY, APOA1, GSN40, GSN80 and RETBP or a transcriptional or a post-translational variant thereof in the test urine sample.

In another embodiment, the first step of the method to assess bladder cancer comprises measuring one or more biomarkers selected from TTHY, ACY1, AKR1A1, ANXA4, BIEA, BLVRB, CATD H, CATD K, CO3, CPGL1, ENOA, FRIL, GDIB, GPX3, GSHB, GSTP1, IDHC, LY6G6E, MASP2, NADC, NAPSA, PA2G4, PARK7, PCBP1, PRDX2, PTD012, QPCT, SBP1 and STIP1 or a transcriptional or a post-translational variant thereof in the test urine sample.

In another embodiment, the first step of the method to assess bladder cancer comprises measuring one or more biomarkers selected from TTHY, CATD H, CATD K, ENOA, FRIL, GSHB, GSTP1, IDCH, MASP2, PRDX2, AMY, APOA1, GSN40, GSN80 and RETBP or a transcriptional or a post-translational variant thereof in the test urine sample.

In another embodiment, the first step of the method to assess bladder cancer comprises measuring one or more biomarkers selected from TTHY, CATD H, CATD K, ENOA, FRIL, GSHB, GSTP1, IDCH, MASP2 and PRDX2 or a transcriptional or a post-translational variant thereof in the test urine sample.

In another embodiment, the first step of the method to assess bladder cancer comprises measuring one or more biomarkers selected from TTHY, AMY, APOA1, GSN40 and GSN80 or a transcriptional or a post-translational variant thereof in the test urine sample,

In another embodiment, the first step of the method to assess bladder cancer comprises measuring APOA1 and RETBP or a transcriptional or a post-translational variant thereof in the test urine sample.

In another embodiment, the first step of the method to assess bladder cancer comprises measuring AMY, APOA1 and GSN40 or a transcriptional or a post-translational variant thereof in the test urine sample.

In another embodiment, the first step of the method to assess bladder cancer comprises measuring AMY, APOA1 and ENOA or a transcriptional or a post-translational variant thereof in the test urine sample.

In another embodiment, the first step of the method to assess bladder cancer comprises measuring APOA1, GSN40 and TTHY or a transcriptional or a post-translational variant thereof in the test urine sample.

In another embodiment, the first step of the method to assess bladder cancer comprises measuring CATD K, GSN80 and IDHC or a transcriptional or a post-translational variant thereof in the test urine sample.

In another embodiment, the first step of the method to assess bladder cancer comprises measuring AMY, APOA1, GSN40 and IDHC or a transcriptional or a post-translational variant thereof in the test urine sample.

In another embodiment, the first step of the method to assess bladder cancer comprises measuring APOA1, GSN40, GSN80 and TTHY or a transcriptional or a post-translational variant thereof in the test urine sample.

In another embodiment, the first step of the method to assess bladder cancer comprises measuring CATD H, ENOA, GSTP1 and PRDX2 or a transcriptional or a post-translational variant thereof in the test urine sample.

In another embodiment, the first step of the method to assess bladder cancer comprises measuring CATD K, ENOA, PRDX2 and TTHY or a transcriptional or a post-translational variant thereof in the test urine sample.

In another embodiment, the first step of the method to assess bladder cancer comprises measuring AMY, CATD K, ENOA, IDHC and PRDX2 or a transcriptional or a post-translational variant thereof in the test urine sample.

In another embodiment, the first step of the method to assess bladder cancer comprises measuring AMY, APOA1, GSN40, GSN80 and TTHY or a transcriptional or a post-translational variant thereof in the test urine sample.

In another embodiment, the first step of the method to assess bladder cancer comprises measuring CATD H, ENOA, GSTP1, MASP2, PRDX2 and TTHY or a transcriptional or a post-translational variant thereof in the test urine sample.

In another embodiment, the first step of the method to assess bladder cancer comprises measuring AMY, APOA1, CATD K, ENOA, IDHC, PRDX2 and TTHY or a transcriptional or a post-translational variant thereof in the test urine sample.

In another embodiment, the first step of the method to assess bladder cancer comprises measuring one or more biomarkers selected from AMY, APOA1, GSN40, GSN80 or a transcriptional or a post-translational variant thereof and one or more biomarkers selected from TTHY, ACY1, AKR1A1, ALDOB, ANXA4, BIEA, BLVRB, CATD H, CATD K, CO3, CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC6I, PPAL, PRDX2, PTD012, QPCT, SBP1, STIP1, TALDO, WDR1 and RETBP or a transcriptional or a post-translational variant thereof in the test urine sample.

In another embodiment, the first step of the method to assess bladder cancer comprises measuring one or more biomarkers selected from AMY, APOA1, GSN40, GSN80 or a transcriptional or a post-translational variant thereof and one or more biomarkers selected from TTHY, ACY1, AKR1A1, ALDOB, ANXA4, BIEA, BLVRB, CATD H, CATD K, CO3, CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC6I, PPAL, PRDX2, PTD012, QPCT, SBP1, STIP1, TALDO and WDR1 or a transcriptional or a post-translatianal variant thereof in the test urine sample.

In another embodiment, the first step of the method to assess bladder cancer comprises measuring one or more biomarkers selected from CATD H, CATD K, ENOA, GSHB, GSTP1, IDHC, PRDX2 and TTHY or a transcriptional or a post-translational variant thereof and one or more biomarkers selected from ACY1, AKR1A1, ALDOB, ANXA4, BIEA, BLVRB, CO3, CPGL1, FRIL, GDIB, GPX3, LMAN2, LY6G6E, MASP2, NADC, NAPSA, PA2G4, PARK7, PCBP1, PDC6I, PPAL, PTD012, QPCT, SBP1, STIP1, TALDO, WDR1, AMY, APOA1, GSN40, GSN80 and RETBP, or a transcriptional or a post-translational variant thereof.

In another embodiment, the first step of the method to assess bladder cancer comprises measuring one or more biomarkers selected from TTHY, CATD H, CATD K, ENOA, GSTP1, IDHC, MASP2, PRDX2, AMY, APOA1, GSN40, GSN80 and RETBP or a transcriptional or a post-translational variant thereof.

Another embodiment relates to a non-invasive *in vitro* method that comprises quantifying one or more ratios between two different biomarkers selected from the 40 bladder cancer biomarkers or a transcriptional or a post-translational variant thereof in a test urine sample from an individual and comparing the measurement obtained in the test urine sample to the corresponding standard value in normal urine, wherein variations are indicative of BTCC.

In another embodiment the method allows to determine the progression of the disease when the same protein or proteins is compared from different samples obtained from the same patient at different times within the BTCC evolution.

In another embodiment one or more of the biomarkers of the invention may be used to monitor the efficacy of pharmacological or surgical treatment.

In another embodiment the sample to be analyzed is obtained from an individual not previously diagnosed with BTCC.

In another embodiment the sample to be analyzed is obtained from an individual who has been previously diagnosed with BTCC.

In another embodiment the sample to be analyzed is obtained from an individual currently receiving treatment against BTCC.

In another embodiment the method comprises obtaining an extract of proteins of the sample.

The measuring typically comprises spectrometry or immunoassay. The spectrometry is typically surface enhanced laser desorption/ionization (SELDI) mass spectrometry or matrix assisted laser desorption/ionisation (MALDI) mass spectrometry. Immunoassays include western blot, ELISA (Enzyme-Linked Immunosorbent assay), RIA (Radioimmunoassay), Competitive EIA (Competitive Enzyme Immunoassay), DAS-ELISA (Double Antibody Sandwich-ELISA), immunocytochemical or immunohistochemical techniques, techniques based on the use of biochips or protein microarrays which include specific antibodies, assays based on the precipitation of colloidal gold in formats such as dipsticks; or by affinity chromatography techniques, ligand binding assays or lectin binding assays.

In another embodiment the method for detecting cancer comprises contacting a urine sample with a molecule that specifically binds a bladder cancer biomarker and quantifying this binding.

In another embodiment the detection and quantification of proteins comprises a first step, in which the protein extract of the sample is placed in contact with a composition of one or more specific antibodies against one or more epitopes of the protein or proteins, and a second step, in which the complexes formed by the antibodies and the proteins are quantified.

In another embodiment the specific antibodies used for the detection of proteins are of human origin, humanized or of non-human origin and selected from monoclonal or polyclonal antibodies, intact or recombinant fragments of antibodies, combibodies and Fab or scFv antibody fragments.

Another embodiment relates to the use of one or more peptide sequences derived from the biomarkers selected from TTHY, ACY1, AKR1A1, ALDOB, ANXA4, BIEA, BLVRB, CATD H, CATD K, CO3, CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC6I, PPAL, PRDX2, PTD012, QPCT, SBP1, STIP1, TALDO, WDR1, AMY, APOA1, GSN40, GSN80 and RETBP or a transcriptional or a post-translational variant thereof, wherein the biomarkers are present in urine.

Another embodiment of the invention relates to the use of one or more peptide sequences derived from the biomarkers selected from TTHY, ACY1, AKR1A1, ALDOB, ANXA4, BIEA, BLVRB, CATD H, CATD K, CO3, CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC6I, PPAL, PRDX2, PTD012, QPCT, SBP1, STIP1, TALDO and WDR1 or a transcriptional or a post-translational variant thereof, wherein the biomarkers are present in urine.

Another embodiment of the invention relates to the use of one or more peptide sequences derived from the cancer biomarkers, wherein variations in the measurement of these biomarkers in a urine test sample in comparison to the corresponding standard value in normal urine are indicative of BTCC.

In another embodiment, at least two biomarkers present in urine or a transcriptional or a post-translational variant thereof are used. In another embodiment, at least three biomarkers present in urine or a transcriptional or a post-translational variant thereof are used. In another embodiment, at least four biomarkers present in urine or a transcriptional or a post-translational variant thereof are used. In another embodiment, at least five biomarkers present in urine or a transcriptional or a post-translational variant thereof are used.

Another embodiment of the invention relates to the use of one or more peptide sequences derived from the biomarkers selected from TTHY, ACY1, AKR1A1, ANXA4, BIEA, BLVRB, CATD H, CATD K, CO3, CPGL1, ENOA, FRIL, GDIB, GPX3, GSHB, GSTP1, IDHC, LY6G6E, MASP2, NADC, NAPSA, PA2G4, PARK7, PCBP1, PRDX2, PTD012, QPCT, SBP1, STIP1, AMY, APOA1, GSN40, GSN80 and RETBP or a transcriptional or a post-translational variant thereof, wherein the biomarkers are present in urine.

Another embodiment of the invention relates to the use of one or more peptide sequences derived from the biomarkers selected from TTHY, ACY1, AKR1A1, ANXA4, BIEA, BLVRB, CATD H, CATD K, CO3, CPGL1, ENOA, FRIL, GDIB, GPX3, GSHB, GSTP1, IDHC, LY6G6E, MASP2, NADC, NAPSA, PA2G4, PARK7, PCBP1, PRDX2, PTD012, QPCT, SBP1 and STIP1 or a transcriptional or a post-translational variant thereof, wherein the biomarkers are present in urine.

Another embodiment of the invention relates to the use of one or more peptide sequences derived from the biomarkers selected from TTHY, CATD H, CATD K, ENOA, FRIL, GSHB, GSTP1, IDCH, MASP2, PRDX2, AMY, APOA1, GSN40, GSN80 and RETBP, or a transcriptional or a post-translational variant thereof, wherein the biomarkers are present in urine.

Another embodiment of the invention relates to the use of one or more peptide sequences derived from the biomarkers selected from TTHY, CATD H, CATD K, ENOA, FRIL, GSHB, GSTP1, IDCH, MASP2 and PRDX2 or a transcriptional or a post-translational variant thereof, wherein the biomarkers are present in urine.

Another embodiment of the invention relates to the use of one or more peptide sequences derived from the biomarkers selected from TTHY, AMY, APOA1, GSN40 and GSN80 or a transcriptional or a post-translational variant thereof, wherein the biomarkers are present in urine.

Another embodiment of the invention relates to the use of the peptide sequences derived from APOA1 and RETBP or a transcriptional or a post-translational variant thereof, wherein the biomarkers are present in urine.

Another embodiment of the invention relates to the use of the peptide sequences derived from AMY, APOA1 and GSN40 or a transcriptional or a post-translational variant thereof, wherein the biomarkers are present in urine.

Another embodiment of the invention relates to the use of the peptide sequences derived from AMY, APOA1 and ENOA or a transcriptional or a post-translational variant thereof, wherein the biomarkers are present in urine.

Another embodiment of the invention relates to the use of the peptide sequences derived from APOA1, GSN40 and TTHY or a transcriptional or a post-translational variant thereof, wherein the biomarkers are present in urine.

Another embodiment of the invention relates to the use of the peptide sequences derived from CATD K, GSN80 and IDHC or a transcriptional or a post-translational variant thereof, wherein the biomarkers are present in urine.

Another embodiment of the invention relates to the use of the peptide sequences derived from APOA1, GSN40, GSN80 and TTHY or a transcriptional or a post-translational variant thereof, wherein the biomarkers are present in urine.

Another embodiment of the invention relates to the use of the peptide sequences derived from AMY, APOA1, GSN40 and IDHC or a transcriptional or a post-translational variant thereof, wherein the biomarkers are present in urine.

Another embodiment of the invention relates to the use of the peptide sequences derived from CATD H, ENOA, GSTP1 and PRDX2 or a transcriptional or a post-translational variant thereof, wherein the biomarkers are present in urine.

Another embodiment of the invention relates to the use of the peptide sequences derived from CATD K, ENOA, PRDX2 and TTHY or a transcriptional or a post-translational variant thereof, wherein the biomarkers are present in urine.

Another embodiment of the invention relates to the use of the peptide sequences derived from AMY, CATD K, ENOA, IDHC and PRDX2 or a transcriptional or a post-translational variant thereof, wherein the biomarkers are present in urine.

Another embodiment of the invention relates to the use of the peptide sequences derived from AMY, APOA1, GSN40, GSN80 and TTHY or a transcriptional or a post-translational variant thereof, wherein the biomarkers are present in urine.

Another embodiment of the invention relates to the use of the peptide sequences derived from CATD H, ENOA, GSTP1, MASP2, PRDX2 and TTHY or a transcriptional or a post-translational variant thereof, wherein the biomarkers are present in urine.

Another embodiment of the invention relates to the use of the peptide sequences derived from AMY, APOA1, CATD K, ENOA, IDHC, PRDX2 and TTHY or a transcriptional or a post-translational variant thereof, wherein the biomarkers are present in urine.

Another embodiment of the invention relates to the use use of one or more peptide sequences derived from the biomarkers selected from AMY, APOA1, GSN40 and GSN80 or a transcriptional or a post-translational variant thereof and one or more peptide sequences derived from the biomarkers selected from TTHY, ACY1, AKR1A1, ALDOB, ANXA4, BIEA, BLVRB, CATD H, CATD K, CO3, CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC6I, PPAL, PRDX2, PTD012, QPCT, SBP1, STIP1, TALDO, WDR1 and RETBP or a transcriptional or a post-translational variant thereof, wherein the biomarkers are present in urine.

Another embodiment of the invention relates to the use use of one or more peptide sequences derived from the biomarkers selected from AMY, APOA1, GSN40 and GSN80 or a transcriptional or a post-translational variant thereof and one or more peptide sequences derived from the biomarkers selected from TTHY, ACY1, AKR1A1, ALDOB, ANXA4, BIEA, BLVRB, CATD H, CATD K, CO3, CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC6I, PPAL, PRDX2, PTD012, QPCT, SBP1, STIP1, TALDO and WDR1 or a transcriptional or a post-translational variant thereof, wherein the biomarkers are present in urine.

Another embodiment of the invention relates to the use use of one or more peptide sequences derived from the biomarkers selected from CATD H, CATD K, ENOA, GSHB, GSTP1, IDHC, PRDX2 and TTHY or a transcriptional or a post-translational variant thereof and one or more peptide sequences derived from the biomarkers selected from ACY1, AKR1A1, ALDOB, ANXA4, BIEA, BLVRB, CO3, CPGL1, FRIL, GDIB, GPX3, LMAN2, LY6G6E, MASP2, NADC, NAPSA, PA2G4, PARK7, PCBP1, PDC6I, PPAL, PTD012, QPCT, SBP1, STIP1, TALDO, WDR1, AMY, APOA1, GSN40, GSN80 and RETBP or a transcriptional or a post-translational variant thereof, wherein the biomarkers are present in urine.

Another embodiment of the invention relates to the use use of one or more peptide sequences derived from the biomarkers selected from TTHY, CATD H, CATD K, ENOA, GSTP1, IDHC, MASP2, PRDX2, AMY, APOA1, GSN40, GSN80 and RETBP or a transcriptional or a post-translational variant thereof, wherein the biomarkers are present in urine.

Another embodiment relates to a kit to perform a method as previously defined comprising 1) any combination of antibodies that specifically recognises one or more of these proteins and 2) a carrier in a suitable packaging, the kit being employed to detect the presence of BTCC, to determine the stage or severity of this cancer, to assess the lack of disease after surgical resection, to establish the diagnosis and/or prognosis of this cancer and/or to monitor the effect of the treatment administered to an individual suffering from said cancer.

The kit may comprise a container for housing at least one agent that binds a biomarker present in a urine sample; and instructions for use of the at least one agent for determining status of BTCC. Such kits may comprise a carrier, package or container that is compartmentalized to receive one or more containers such as vials, tubes, and the like, each of the container(s) comprising one of the separate elements to be used in the method. For example, the container(s) can comprise a probe that is or can be labelled and further detected. The probe can be an antibody or polynucleotide specific for a bladder cancer protein or a bladder cancer gene, respectively. Alternatively, the kit can comprise a mass spectrometry (MS) probe. The kit can also include containers containing nucleotide(s) for amplification or silencing of a target nucleic acid sequence, and/or a container comprising a reporter-means, such as a biotin-binding protein, e.g., avidin or streptavidin, bound to a detectable label, e.g., an enzymatic, florescent, or radioisotope label. The kit can include all or part of the amino acid sequence of the bladder cancer biomarkers, or a nucleic acid molecule that encodes such amino acid sequences. The kit of the invention will typically comprise the container described above and one or more other containers comprising materials desirable from a commercial and user standpoint, including buffers, diluents, filters, needles, syringes, and package inserts with instructions for use. In addition, a label can be provided on the container to indicate that the composition is used for a specific therapeutic or non-therapeutic application, and can also indicate directions for use, such as those described above. Directions and or other information can also be included on an insert which is included with the kit.

Another embodiment relates to a kit to perform a method as previously defined comprising a biochip.

Another embodiment relates to a kit to perform a method as previously defined comprising a biochip, wherein the biochip comprises antibodies for the detection of one or more biomarkers selected from the 40 bladder cancer biomarkers or a transcriptional or a post-translational variant thereof.

There is a wide range of immunological assays available to detect and/or quantify the formation of specific antigen-antibody complexes; numerous competitive or non-competitive protein-binding assays have been described previously and a large number of these are commercially available. Hence, proteins can be quantified with antibodies such as, for example: monoclonal antibodies, polyclonal antibodies, either intact or recombinant fragments of these, combibodies and Fab or scFv fragments of antibodies, specific for proteins. These antibodies may be of human origin, humanized or of animal origin. On the other hand, they can be labelled or unlabelled and can be used in a wide range of assays. Marker molecules that can be used to label antibodies include radionuclides, enzymes, fluorophores, chemoluminescent reagents, enzymatic substrates or cofactors, enzymatic inhibitors, particles, colorants and derivatives. The higher the antibody binding specificity, the lower the antigen concentration that can be detected.

There are a wide variety of assays well known to those skilled in the art that can be used in the present invention, which use unlabelled antibodies (primary antibodies) and labelled antibodies (secondary antibodies); these techniques include but are not limited to the western blot or western transfer, ELISA (Enzyme-Linked immunosorbent assay), RIA (Radioimmunoassay), Competitive EIA (Competitive enzyme immunoassay), DAS-ELISA (Double antibody sandwich-ELISA), immunocytochemical and immunohistochemical techniques, techniques based on the use of biochips or protein microarrays that include specific antibodies or colloidal precipitation in formats such as dipsticks. Other ways to detect and/or quantify proteins include affinity chromatography techniques, ligand binding assays or lectin binding assays. The preferred embodiments of this aspect of the invention are protein microarrays and double antibody sandwich ELISA (DAS-ELISA). In these immunoassays any antibody, or combination of antibodies, which are specific against one or more epitopes of the 40 proteins of the invention can be used. As an example of one of the many possible formats of this assay, a monoclonal or polyclonal antibody, or a fragment of this antibody, or a combination of these antibodies that recognise one or more epitopes of the 40 proteins of the invention are attached to the surface of a solid phase support and placed in contact with the sample to be analyzed and incubated for a specific time and in appropriate conditions to form the antigen-antibody complexes. After washing in appropriate conditions to eliminate non-specific complexes, an indicator reagent, consisting in a monoclonal or polyclonal antibody, or a fragment of this antibody, or a combination of these and which recognises one or more epitopes of the 40 proteins of the invention, bound to a signal generating molecule, is incubated with the antigen-antibody complexes in appropriate conditions of time and temperature. The presence of one or more proteins selected from the 40 proteins of the invention in the sample to be analyzed is detected and quantified and the signal generated measured. In order to prevent signal variation due to differences in total protein concentration among samples, all measurements are normalized.

As indicated above, the method of the invention involves monitoring the stage of bladder carcinoma by quantitating the differentially-expressed soluble proteins within a urine sample through specific antibodies. As will be appreciated by those skilled in the art, any means for specifically identifying and quantifying these proteins are contemplated.

In the description which follows, the generalized method for obtaining and analyzing the total protein content of human urine samples (herein referred to as the sample) is disclosed. The method involves the processing of the samples, the use of 2D electrophoresis to separate proteins within the sample, the selection of differentially expressed proteins by means of image analysis and statistics of different samples and the use of one or more of the differentially expressed proteins of the invention to generate protein-specific antibodies to be used as bladder cancer markers.

The comparative proteome analysis was performed between samples obtained from healthy individuals (controls) and patients diagnosed with BTCC (Ta, T1-low grade, T1-high grade and T2) in an attempt to identify differentially expressed proteins in the various stages of bladder cancer and during bladder cancer progression. Among all the proteins that showed differential expression, 40 proteins changed more than two-fold reproducibly. They were identified by peptide mass fingerprinting using mass spectrometry and database search.

The present invention will be further illustrated by the following examples. These examples are given by way of illustration only and are not to be construed as limiting.

1.Identification of differentially expressed proteins

To identify differentially expressed proteins along progression of bladder cancer, protein profiles of healthy urines were compared with those of early-stage and advanced bladder tumor patients using a proteomic approach.

Urine samples (150 in total) were collected from healthy individuals and from patients with transitional bladder cancer visiting the Urology units of Hospitals belonging to the Spanish Public Health Network. These samples were classified as follows:

a) No Carcinoma (63 samples) including:

- Controls (CV, 32 samples): patients that had had bladder cancer and had been resected from the tumor and were being controlled (cystoscopy showed no other affectations).
- Patients with other urological tract diseases (31 samples, including among others prostate benign hyperplasia, prostate cancer).

b) BTCC (87 samples): patients diagnosed with the disease at different stages of development including:

- Ta (21 samples)
- T1-low grade (29 samples)
- T1-high grade (19 samples)
- T2 (18 samples)

The BTCC group of samples was accompanied by a biopsy that was the key for their classification in the stages of development of bladder cancer.

A. Processing of urine samples and separation of proteins.

Urine samples were frozen at -80°C and shipped to the lab in dry ice without breaking the cold chain. Samples were kept at -80°C until they were processed. The samples were very heterogenic, ranging from light yellow urines to red ones with blood clumps, transparent or containing tissues in suspension. The total volume was also very variable, from 5 to 100 mL. The protein concentration of the samples ranged from 20 $\mu$g/mL to 2 mg/mL (150 $\mu$g/mL was the average concentration). To obtain the protein content, samples were thawed on ice and centrifuged at 2000xg for 5 min at 4°C. The supernatant was used to determine protein concentration. The volume necessary to precipitate 100 $\mu$g of

protein was calculated taking into account that the efficacy of protein precipitation with 15% w/v of trichloroacetic acid (TCA) is 75%. The rest of the urine sample was frozen again at -80°C and stored for further 2D electrophoresis (if needed). TCA and urine were mixed for 1 hour on ice, and then centrifuged at 16000xg for 20 min at 4°C to obtain the pellet of precipitated proteins. This pellet was washed with acetone, stored at -20°C and dried by solvent evaporation.

To perform the two dimension (2D) electrophoresis experiments, the first dimension was IEF (isoelectric focusing), where proteins separate by their charge (pl); the second dimension was SDS-PAGE where proteins separate by their molecular weight. To carry out the first dimension, the dried pellet of proteins was resuspended with 450 $\mu$L of rehydratation buffer (Urea 7M, thiourea 2M, CHAPS 2%, IPG buffer 2%, bromophenol blue 0.002%) for 1 hour at room temp. IPG buffer (Amersham, ref# 17-600-88) was used so that the IEF ranged from pH 3-10. For the IEF, the Ettan™ IPGphor™ Isoelectric Focusing System from Amersham was used following the manufacturer's directions. The IEF was performed in immobilized pH gradients, named IPG strips, purchased from Amersham (ref# 17-6002-45). The solubilized proteins focused in the first dimension in the strips after 16 hours of active rehydratation of the gel at 30 V. Then the voltage was increased to 8000 V, the intensity never being higher than 50 $\mu$A per gel. The IEF was finished when the voltage reached 90000 Vhour.

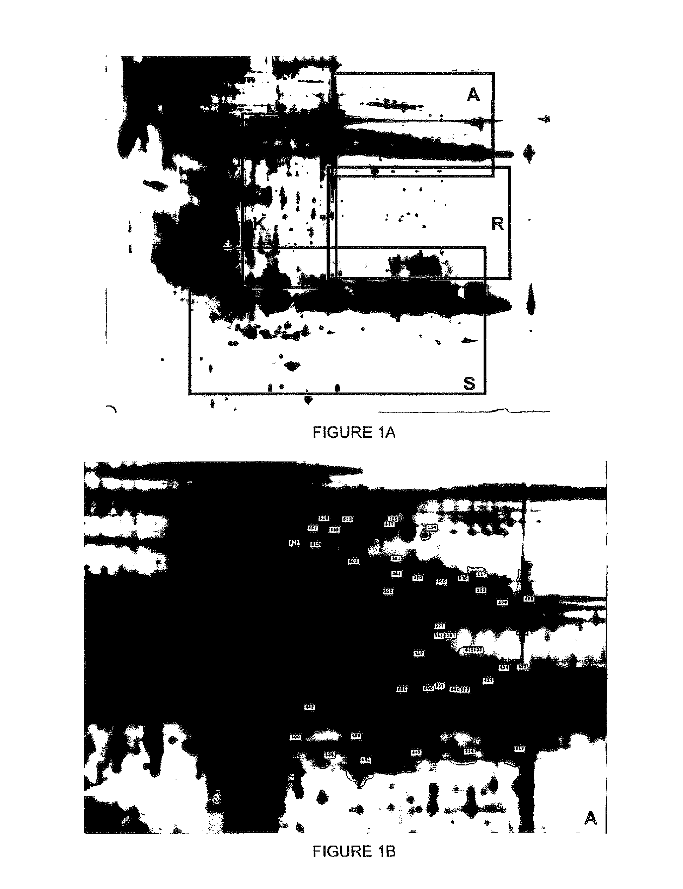

For the second dimension, 26 x 20 cm 12.5% acrylamide were polymerized in the lab using the Ettan DALT twelve Gel Caster from Amersham. Gels were run in the Ettan DALT twelve Large Format Vertical System and following the manufacturer's instructions until the electrophoresis front left the gel. Gels were silver nitrate dyed using the dye kit from Amersham (17-1150-01) following the manufacturer's directions. Gels were then dried and stored for subsequent image analysis of the protein spots (FIGURE 1A). For some urine samples more than one 2D-gel was run.

B. Analyses of the protein spots on the gels

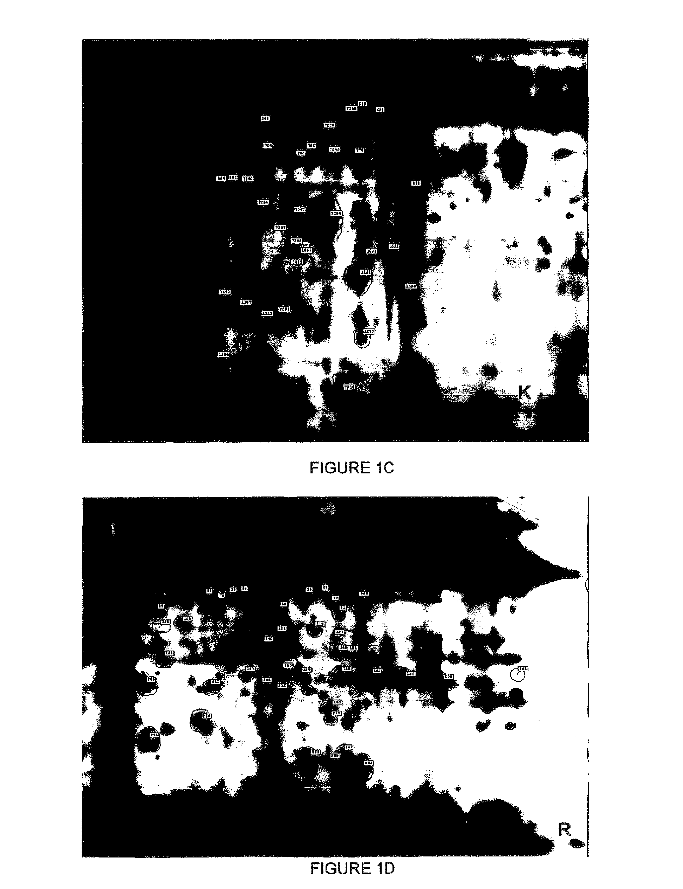

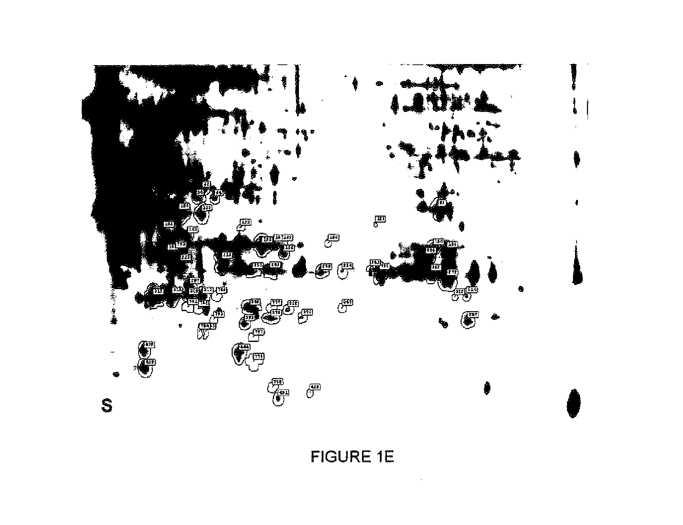

Every gel was scanned to obtain the map of spots for image analysis. Progenesis PG220 software from Nonlinear Dynamics (UK) was used to analyze image files in a 300 dpi (dot per inch) format and 8 bits/channel. To increase the resolution, the analysis was performed in discrete areas of the gels. For each area, named as A (FIGURE 1B), K (FIGURE 1C), R (FIGURE 1 D) and S (FIGURE 1 E), the best gels were selected and scanned for image analysis. The Progenesis PG220 software transformed the information of the flat image into a 3D image, where the intensity of each spot correlated with its volume and with the relative amount of the corresponding protein in the urine of the individual. With the software tables, the intensities for each spot were obtained in each gel. These raw data were the basis for the subsequent statistical analyses.

To perform the statistical analysis of each individual spot and compare its intensity in two different groups (for instance, CV and Ta), a normal distribution had to be proven in these blocks of data. To compare the spots belonging to two groups, a Student's t test was applied and a $p$ value obtained: this was the theoretical error of assigning a spot to one subgroup or to another one. Only the spots with $p$ values $\leq 0.05$ were selected. The fold change or average ratio of these spots was calculated which either referred to 1) the total spot volume of the sample, that is the theoretical total protein content, or to 2) the constantly expressed protein, the amount of which is constant in every sample, or to 3) a second differentially expressed protein of the same sample.

Also the $n$ value or number of samples was taken into account. 40 proteins showed statistical significance and robustness in their fold change when comparing urine samples from control patients with urine samples from patients diagnosed with transitional bladder cancer.

The identification of those spots was carried out by MALDI-TOF (Matrix-Assisted Laser-Desorption/Ionization Time Of Flight) spectroscopy. Those urine samples whose 2D electrophoresis had shown statistically significant spots were submitted again to 2D electrophoresis and the spots excised from the gel. Proteins were digested and analyzed by MALDI-TOF spectrometer. Peptide mass fingerprint enabled the identification of 40 proteins, that were identified as TTHY, ACY1, AKR1A1, ALDOB, ANXA4, BIEA, BLVRB, CATD H, CATD K, CO3, CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC6I, PPAL, PRDX2, PTD012, QPCT, SBP1, STIP1, TALDO, WDR1, AMY, APOA1, GSN40, GSN80 and RETBP (see table 1). Thus, these 40 proteins, that had proven to be differentially expressed in patients diagnosed with BTCC, were identified as biological markers of significant value for the diagnosis, prognosis, monitoring and treatment of the disease.

Table 1

| PROTEIN SYMBOL | SEQ ID No | SPOT No | GI | UniProt | OMIM | PROTEIN NAME |
|---|---|---|---|---|---|---|
| TTHY | 1 | S789 | 55669576 | P02766 | 176300 | Transthyretin |
| ACY1 | 2 | K878 | 4501901 | Q03154 | 104620 | Aminoacylase-1 |

(continued)

| PROTEIN SYMBOL | SEQ ID No | SPOT No | GI | UniProt | OMIM | PROTEIN NAME |
|---|---|---|---|---|---|---|
| AKR1A1 | 3 | R183 | 1633300 | P14550 | 103830 | Aldehyde reductase |
| ALDOB | 4 | R385 | 113611 | P05062 | 229600 | Aldolase B |
| ANXA4 | 5 | K1273 | 1703319 | P09525 | 106491 | Annexin A4 |
| BIEA | 6 | R211 | 47117734 | P53004 | 109750 | Biliverdin reductase A |
| BLVRB | 7 | S314 S322 | 32891807 | P30043 | 600941 | Flavin reductase |
| CATD H | 8 | S165 | 5822091 | P07339 | 116840 | Cathepsin D |
| CATD K | 9 | K754 K760 K780 | 30582659 | P07339 | 116840 | Cathepsin D |
| CO3 | 10 | A594 | 4557385 | P01024 | 120700 | Complement C3 (fragment) |
| CO3 | 11 | A596 | 40786791 | P01024 | 120700 | Complement C3 (fragment) |
| CPGL1 | 12 | K629 | 15620780 | Q96KP4 | 169800 | Cytosolic nonspecific dipeptidase |
| ENOA | 13 | R383 R058 R054 | 4503571 | P06733 | 172430 | Alpha enolase |
| ENOA | 14 | A519 A524 A530 | 31873302 | P06733 | 172430 | Alpha enolase |
| FRIL | 15 | S444 | 182516 | P02792 | 134790 | Ferritin light chain |
| GDIB | 16 | A499 R061 | 6598323 | P50395 | 600767 | Rab GDP dissociation inhibitor beta |
| GPX3 | 17 | S777 | 404108 | P22352 | 138321 | Plasma glutathione peroxidase |
| GSHB | 18 | K7230 | 8248826 | P48637 | 601002 | Glutathione synthetase |
| GSTP1 | 19 | S346 | 20664359 | P09211 | 134660 | Glutathione S-transferase P |
| IDHC | 20 | R119 | 49168486 | 075874 | 147700 | Cytoplasmic Isocitrate dehydrogenase |
| LMAN2 | 21 | K1187 K7280 K7292 | 5803023 | Q12907 | None | Vesicular integral-membrane protein VIP36 precursor |
| LY6G6E | 22 | K7359 | 55961604 | Q5SRS9 | None | Lymphocyte antigen 6 complex |
| MASP2 | 23 | S775 | 50513646 | 000187 | 605102 | Mannan-binding lectin serine protease 2 |

(continued)

| PROTEIN SYMBOL | SEQ ID No | SPOT No | GI | UniProt | OMIM | PROTEIN NAME |
|---|---|---|---|---|---|---|
| NADC | 24 | K1166 | 14603130 | Q15274 | 606248 | Nicotinate-nucleotide pyrophosphorylase |
| NAPSA | 25 | K1213 R276 | 17389633 | O96009 | 605631 | Napsin A |
| PA2G4 | 26 | R056 | 33879698 | Q9UQ80 | 602145 | Proliferation-associated protein 2G4 |
| PARK7 | 27 | S347 | 50513593 | Q99497 | 602533 | Oncogene DJ1; DJ-1 |
| PCBP1 | 28 | R149 | 5453854 | Q15365 | 601209 | Poly(rC)-binding protein 1 |
| PDC6I | 29 | A627 | 46249756 | Q8WUM4 | 608074 | Programmed cell death 6-interacting protein |
| PPAL | 30 | A641 R072 | 32700070 | P11117 | 171650 | Lysosomal acid phosphatase precursor |
| PRDX2 | 31 | S393 | 1617118 | P32119 | 600538 | Peroxiredoxin 2 |
| PTD012 | 32 | R216 | 48257065 | AAD40375 | None | PTD012 protein |
| QPCT | 33 | K7258 K7346 K7347 | 525241 | Q16769 | 607065 | Glutaminyl-peptide cyclotransferase |
| SBP1 | 34 | A482 | 1374792 | Q13228 | 604188 | Selenium-binding protein 1 |
| STIP1 | 35 | A387 | 54696884 | P31948 | 605063 | Stress-induced-phosphoprotein 1 |
| TALDO | 36 | R205 R206 | 16307182 | P37837 | 602063 | Transaldolase |
| WDR1 | 37 | A372 | 3420181 | 075083 | 604734 | WD-repeat protein 1 |
| AMY | 38 | A638 | 10280622 | P19961 | 104660 | Alpha-amylase |
| AMY | 39 | A636 | 4502085 | P04746 | 104650 | Alpha-amylase |
| AMY | 40 | A637 | 1633119 | P04745 | 104700 | Alpha-amylase |
| APOA1 | 41 | S313 S319 S782 | 178775 | P02647 | 107680 | Apolipoprotein A-I |
| GSN40 | 42 | 789 2537 2538 | 17028367 | P06396 | 137350 | Gelsolin |
| GSN80 | 43 | A612 | 4504165 | P06396 | 137350 | Gelsolin |
| RETBP | 44 | S784 S785 | 230284 | P02753 | 180250 | Plasma retinol-binding protein |

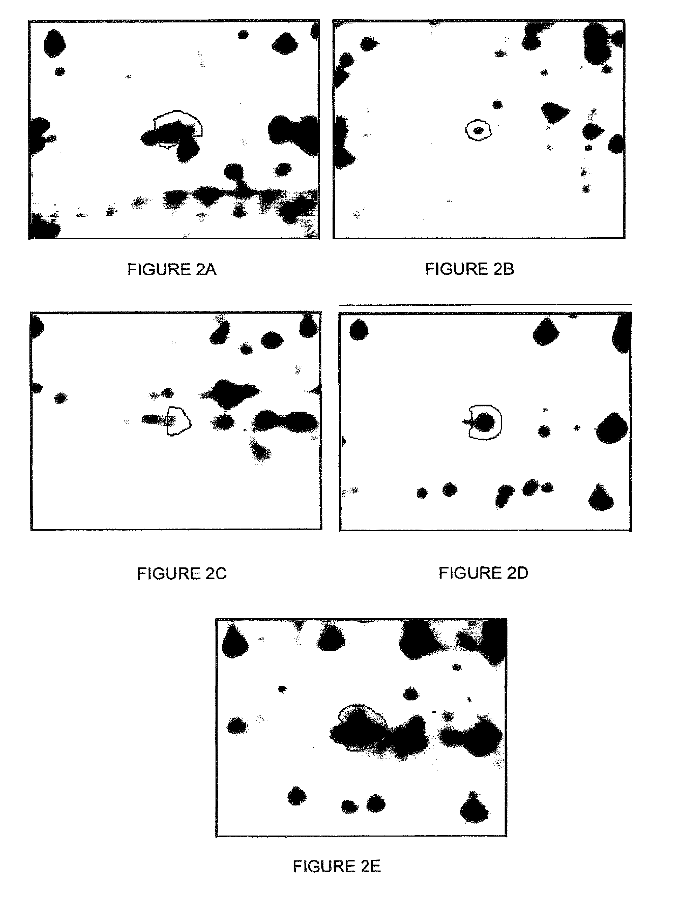

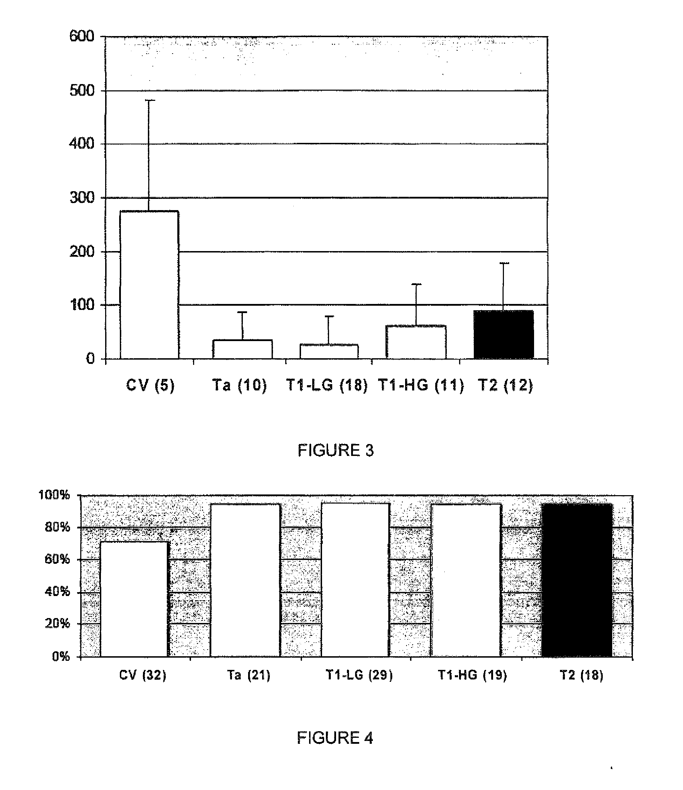

As an example, FIGURE 2 shows spot R211 corresponding to BIEA in the R area of bidimensional electrophoresis (2D) gel obtained from urine samples of a healthy individual (FIGURE 2A), of a patient suffering from cancer at the Ta stage (FIGURE 2B), of a patient suffering from cancer at the T1-low grade stage (FIGURE 2C), of a patient suffering from cancer at the T1-high grade stage (FIGURE 2D) and of a patient suffering from cancer at the T2 stage (FIGURE 2E). It can be seen that the intensity of spot R211 is clearly decreased in the samples of the patients with cancer at different stages, when compared to the healthy urine sample. These differences showed to be significant after statistical analysis with Progenesis PG220 software. FIGURE 3 shows the intensity of spot R211 in samples CV, Ta, T1-low grade, T1-high grade and T2. The number of samples for each group is indicated in parentheses. FIGURE 4 shows the robustness of the intensity measurement for spot R211 in different urine samples. This is expressed as the percentage of the gels that maintain the presence or absence of spot R211 in every gel analyzed. The number of samples for each group is again indicated in parentheses. Table 2 shows the $p$ and fold change values of the statistical analysis for spot R211 in samples of different stages of cancer in comparison to a sample of a non-cancer individual (CV). The fold change of spot R211 was normalized by the total spot volume (TSV) for each individual gel.

Table 2

| Type of cancer | $p$ | Fold change |
|---|---|---|
| Ta | 0.0226 | -3.05 |
| T1-low grade | 0.00565 | -2.82 |
| T1-high grade | 0.0212 | -3.84 |
| T2 | 0.0253 | -2.41 |

II. Antibody development for the proteins found in the urine sample

Available polyclonal and/or monoclonal antibodies (either commercially purchased or generated following immunization protocols) were tested for reactivity and sensitivity, the method generally involving preparation of a standard ("dose response") curve for the protein to be monitored.

A. Immunization Protocols

1. Polyclonal Antibodies

Polyclonal antisera can be raised against a given protein using standard methodologies, such as those disclosed in numerous texts available in the art and known to those generally skilled in the art. In this example, male New Zealand White rabbits are immunized with the protein preparations first in Freund's complete adjuvant (Gibco, Grand Island, N.Y.) and then every month with the protein with incomplete adjuvant for three months. Rabbit sera and sera from mice prior to fusion are used as polyclonal antisera and are shown by standard western blot technique to be reactive with the protein preparations.

2. Recombinant proteins generation

Proteins found in the gels in too low quantities for immunization are cloned and expressed in *E. coli* using the pET28b(+) cloning vector. Crude extracts are obtained as described previously (Boronat A., et al., J. Bacteriol. 1981, 147:181-85) and run on a SDS-PAGE gel. Recombinant proteins are excised from the gel and released from acrylamide. For the generation of antibodies against recombinant proteins, the released proteins are directly used to immunize rabbits as described above.

B. Western blot experiments

Protein samples (20 $\mu$g of total protein) were mixed with SDS-PAGE gel loading buffer supplemented with 5% $\beta$-mercaptoethanol and incubated at 100°C for 5 min, before being loaded on 6% polyacrylamide gel. Following electrophoresis proteins were transferred to nitrocellulose membranes. Duplicate gels were run and blotted. One membrane was proved with antibodies raised against one or more of the selected 40 proteins of the invention (Santa Cruz Biotech. Inc., Santa Cruz, CA, USA.) while the second membrane was proved with an antibody raised against actin (Amersham, Little Chalfont, UK) as a control for protein loading. Finally, membranes were hybridised with a secondary antibody conjugated with peroxidase (Amersham) and the chemoluminescent signal was detected using the ECL system (Amersham) with high performance chemiluminescence film (Hyperfilm ECL, Amersham).

III. Validation of urine test samples using western blot experiments

Several biomarkers were tested on 40 blinded urine samples comprising:

a) No Carcinoma (12 samples)

b) BTCC including: Ta (8 samples), T1-low grade (6 samples), T1-high grade (6 samples) and T2 (8 samples).

The obtained values of sensitivity, specificity and accuracy are shown in table 3. The values of sensitivity, specificity and accuracy were calculated using these formulas:

$$\text{Accuracy} = \frac{(\text{True negatives} + \text{True positives})}{\text{Number of samples}}$$

$$\text{Sensitivity} = \frac{\text{True positives}}{(\text{True positives} + \text{False negatives})}$$

$$\text{Specificity} = \frac{\text{True negatives}}{(\text{True negatives} + \text{False positives})}$$

For example, the biomarker AMY shows sensitivity of 89% and specificity of 75% for an accuracy value of 85%.

Table 3

| Markers | Con trols | | Tum ors | | Accuracy | Specificity | Sensitivity |
|---|---|---|---|---|---|---|---|
| | True negatives | False negatives | False positives | True positives | | | |
| AMY | 9 | 3 | 3 | 25 | 85% | 75% | 89% |
| APOA1 | 9 | 2 | 3 | 26 | 88% | 75% | 93% |
| CATD H | 6 | 5 | 6 | 23 | 73% | 50% | 82% |
| CATD K | 8 | 4 | 4 | 24 | 80% | 67% | 86% |
| ENOA | 12 | 13 | 0 | 15 | 68% | 100% | 54% |
| GSHB | 12 | 18 | 0 | 10 | 55% | 100% | 36% |
| GSN40 | 7 | 0 | 5 | 28 | 88% | 58% | 100% |
| GSN80 | 8 | 6 | 4 | 22 | 75% | 67% | 79% |
| GSTP1 | 6 | 3 | 6 | 25 | 78% | 50% | 89% |
| IDHC | 9 | 7 | 3 | 21 | 75% | 75% | 75% |
| PRDX2 | 11 | 11 | 1 | 17 | 70% | 92% | 61% |
| TTHY | 6 | 3 | 6 | 25 | 78% | 50% | 89% |

By a binomial logistic regression of the data obtained in these experiments it was possible to obtain different combinations of biomarkers which generally increased the sensitivity and the specificity of the diagnostic method, as shown in table 4.

Table 4

| Markers | Controls | | Tumors | | Accuracy | Specificity | Sensitivity |
|---|---|---|---|---|---|---|---|
| | True negatives | False negatives | False positives | True positives | | | |
| APOA1, RETBP | 9 | 2 | 3 | 26 | 88% | 75% | 93% |
| AMY, APOA1, GSN40 | 9 | 3 | 3 | 25 | 85% | 75% | 89% |
| AMY, APOA1, ENOA | 9 | 3 | 3 | 25 | 85% | 75% | 89% |
| APOA1, GSN40, TTHY | 11 | 1 | 1 | 27 | 95% | 92% | 96% |
| CATD K, GSN80, IDHC | 7 | 2 | 5 | 26 | 83% | 58% | 93% |
| APOA1, GSN40, GSN80, TTHY | 10 | 1 | 2 | 27 | 93% | 83% | 96% |
| AMY, APOA1, GSN40, IDHC | 9 | 2 | 3 | 26 | 88% | 75% | 93% |
| CATD H, ENOA, GSTP1, PRDX2 | 9 | 4 | 3 | 24 | 83% | 75% | 86% |
| CATD K, ENOA, PRDX2, TTHY | 8 | 3 | 4 | 25 | 83% | 67% | 89% |
| AMY, CATD K, ENOA, IDHC, PRDX2 | 7 | 4 | 5 | 24 | 78% | 58% | 86% |
| AMY, APOA1, GSN40, GSN80, TTHY | 11 | 1 | 1 | 27 | 95% | 92% | 96% |
| CATD H, ENOA, GSTP1, MASP2, PRDX2, TTHY | 10 | 2 | 2 | 26 | 90% | 83% | 93% |

(continued)

| Markers | Cont rols | | Tum ors | | Accuracy | Specificity | Sensitivity |
|---|---|---|---|---|---|---|---|
| | True negatives | False negatives | False positives | True positives | | | |
| AMY, APOA1, CATD K, ENOA, IDHC, PRDX2, TTHY | 10 | 3 | 2 | 25 | 88% | 83% | 89% |

DESCRIPTION OF THE DRAWINGS

FIGURE 1: Bidimensional electrophoresis (2D) gel obtained from a urine sample and showing the four different studied areas A, K, R and S (FIGURE 1A), area A (FIGURE 1 B), area K (FIGURE 1C), area R (FIGURE 1D) and area S (FIGURE 1E).
FIGURE 2: Spot R211 corresponding to BIEA in the R area of Bidimensional electrophoresis (2D) gel obtained from urine samples of a healthy individual (FIGURE 2A), of a patient suffering from cancer at the Ta stage (FIGURE 2B), of a patient suffering from cancer at the T1-low grade stage (T1-LG, FIGURE 2C), of a patient suffering from cancer at the T1-high grade stage (T1-HG, FIGURE 2D) and of a patient suffering from cancer at the T2 stage (FIGURE 2E).
FIGURE 3: Intensity of spot R211 in samples CV, Ta, T1-low grade (T1-LG), T1-high grade (T1-HG) and T2. The number of samples for each group is indicated in parentheses.
FIGURE 4: Robustness of the intensity measurement for spot R211 in different urine samples. This is expressed as the percentage of the gels that mantain the presence or absence of spot R211 in every gel analyzed. The number of samples for each group is indicated in parentheses.

Preferred embodiments:

1. A non-invasive *in vitro* method that comprises: a) detecting and quantifying one or more biomarkers present in a urine test sample from an individual; and b) comparing the expression measurement obtained in a) in the test urine sample to the corresponding standard value in normal urine, wherein variations in the measurement obtained in a) compared to the corresponding standard value in normal urine are indicative of transitional cell carcinoma of the bladder.

2. Method according to item 1 wherein the step a) comprises detecting and quantifying one or more biomarkers selected from the set comprising TTHY (SEQ ID 1), ACY1 (SEQ ID 2), AKR1A1 (SEQ ID 3), ALDOB (SEQ ID 4), ANXA4 (SEQ ID 5), BIEA (SEQ ID 6), BLVRB (SEQ ID 7), CATD H (SEQ ID 8), CATD K (SEQ ID 9), CO3 (SEQ ID 10, SEQ ID 11), CPGL1 (SEQ ID 12), ENOA (SEQ ID 13, SEQ ID 14), FRIL (SEQ ID 15), GDIB (SEQ ID 16), GPX3 (SEQ ID 17), GSHB (SEQ ID 18), GSTP1 (SEQ ID 19), IDHC (SEQ ID 20), LMAN2 (SEQ ID 21), LY6G6E (SEQ ID 22), MASP2 (SEQ ID 23), NADC (SEQ ID 24), NAPSA (SEQ ID 25), PA2G4 (SEQ ID 26), PARK7 (SEQ ID 27), PCBP1 (SEQ ID 28), PDC6I (SEQ ID 29), PPAL (SEQ ID 30), PRDX2 (SEQ ID 31), PTD012 (SEQ ID 32), QPCT (SEQ ID 33), SBP1 (SEQ ID 34), STIP1 (SEQ ID 35), TALDO (SEQ ID 36), WDR1 (SEQ ID 37), AMY (SEQ ID 38, SEQ ID 39, SEQ ID 40), APOA1 (SEQ ID 41), GSN40 (SEQ ID 42), GSN80 (SEQ ID 43) and RETBP (SEQ ID 44) or a transcriptional or a post-translational variant thereof.

3. Method according to item 2, wherein the step a) comprises detecting and quantifying one or more biomarkers selected from TTHY, ACY1, AKR1A1, ALDOB, ANXA4, BIEA, BLVRB, CATD H, CATD K, C03, CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC6I, PPAL, PRDX2, PTD012, QPCT, SBP1, STIP1, TALDO and WDR1 or a transcriptional or a post-translational variant thereof.

4. Method according to item 2, wherein the step a) comprises detecting and quantifying one or more biomarkers selected from TTHY, AMY, APOA1, GSN40 and GSN80 or a transcriptional or a post-translational variant thereof.

5. Method according to item 2, wherein the step a) comprises detecting and quantifying one or more biomarkers selected from AMY, APOA1, GSN40 and GSN80 or a transcriptional or a post-translafiional variant thereof and one or more biomarkers selected from TTHY, ACY1, AKR1A1, ALDOB, ANXA4, BIEA, BLVRB, CATD H, CATD K, CO3, CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC6I, PPAL, PRDX2, PTD012, QPCT, SBP1, STIP1, TALDO and WDR1 or a transcriptional or a post-translational variant thereof.

6. Method according to item 2, wherein the step a) comprises detecting and quantifying one or more biomarkers selected from CATD H, CATD K, ENOA, GSHB, GSTP1, IDHC, PRDX2 and TTHY or a transcriptional or a post-translational variant thereof and one or more biomarkers selected from ACY1, AKR1A1, ALDOB, ANXA4, BIEA, BLVRB, CO3, CPGL1, FRIL, GDIB, GPX3, LMAN2, LY6G6E, MASP2, NADC, NAPSA, PA2G4, PARK7, PCBP1, PDC6I, PPAL, PTD012, QPCT, SBP1, STIP1, TALDO, WDR1, AMY, APOA1, GSN40, GSN80 and RETBP or a transcriptional or a post-translational variant thereof.

7. Method according to item 2, wherein the step a) comprises detecting and quantifying one or more biomarkers selected from TTHY, CATD H, CATD K, ENOA, GSTP1, IDHC, MASP2, PRDX2, AMY, APOA1, GSN40, GSN80 and RETBP or a transcriptional or a post-translational variant thereof.

8. Method according to any of the items 2 to 7, wherein the step a) comprises detecting and quantifying at least two biomarkers selected from the set as defined in item 2.

9. Method according to any of the items 2 to 7, wherein the step a) comprises detecting and quantifying at least three biomarkers selected from the set as defined in item 2.

10. Method according to any of the items 2 to 7, wherein the step a) comprises detecting and quantifying at least four biomarkers selected from the set as defined in item 2.

11. Method according to any of the items 2 to 7, wherein the step a) comprises detecting and quantifying at least five biomarkers selected from the set as defined in item 2.

12. Method according to item 2, wherein the step a) comprises detecting and quantifying the biomarkers APOA1 , GSN40 and TTHY or the respective transcriptional or post-translational variants thereof.

13. Method according to item 2, wherein the step a) comprises detecting and quantifying the biomarkers APOA1, GSN40, GSN80 and TTHY or the respective transcriptional or post-translational variants thereof.

14. Method according to item 2, wherein the step a) comprises detecting and quantifying the biomarkers AMY, APOA1, GSN40, GSN80 and TTHY or the respective transcriptional or post-translational variants thereof.

15. Method according to item 2, wherein the step a) comprises detecting and quantifying the biomarkers CATD H, ENOA, GSTP1, MASP2, PRDX2 and TTHY or the respective transcriptional or post-translational variants thereof.

16. Method according to item 2, wherein the step a) comprises detecting and quantifying the biomarkers AMY, APOA1, CATD K, ENOA, IDHC, PRDX2 and TTHY or the respective transcriptional or post-translational variants thereof.

17. Method according to any of the items 1 to 16 which is employed to detect the presence of transitional cell carcinoma of the bladder, to determine the stage or severity of this cancer, to assess the lack of disease after surgical resection, to establish the diagnosis and/or prognosis of this cancer and/or to monitor the effect of the treatment administered to an individual suffering from the said cancer.

18. Method according to any of the items 1 to 17 in which the urine sample to be analyzed is obtained from an individual not previously diagnosed with transitional cell carcinoma of the bladder.

19. Method according to any of the items 1 to 17 in which the urine sample to be analyzed is obtained from an individual who has been previously diagnosed with transitional cell carcinoma of the bladder.

20. Method according to any of the items 1 to 17 in which the urine sample to be analyzed is obtained from an individual currently receiving treatment against transitional cell carcinoma of the bladder.

21. Method according to any of the items 1 to 20 characterised in that the detection and quantification of proteins comprises a first step, in which the protein extract of the urine sample is placed in contact with a composition of one or more specific antibodies specific to one or more epitopes of the protein or proteins of item 2, and a second step, in which the resulting antibody-protein complexes are quantified.

22. Method according to item 21 characterised in that the said antibodies are of human origin, humanized or of non-human origin and selected from monoclonal or polyclonal antibodies, intact or recombinant fragments of antibodies, combibodies and Fab or scFv antibody fragments.

23. Method according to item 21 or 22 characterised in that in the detection and quantification of the resulting antibody-protein complexes, the techniques used are selected from the group comprised by: western blot, ELISA (Enzyme-Linked Immunosorbent assay), RIA (Radioimmunoassay), Competitive EIA (Competitive Enzyme Immunoassay), DAS-ELISA (Double Antibody Sandwich-ELISA), immunocytochemical or immunohistochemical techniques, techniques based on the use of biochips or protein microarrays that include specific antibodies, assays based on the precipitation of colloidal gold in formats such as dipsticks; or by affinity chromatography techniques, ligand binding assays or lectin binding assays.

24. Method according to any of the items 1 to 23, wherein the method is used to monitor the efficacy of pharmacological or surgical treatments.

25. Method according to any of the items 1 to 24, wherein the method is used to determine the progression of the disease when the same protein or proteins are compared from different urine samples from the same patient obtained at different times within the evolution of the transitional cell carcinoma of the bladder.

26. *In vitro* use of one or more peptide sequences derived from biomarkers present in urine to detect the presence of transitional cell carcinoma of the bladder via urine analysis, to determine the stage or severity of this cancer, to assess the lack of disease after surgical resection, to establish the diagnosis and/or prognosis of this cancer and/or to monitor the effect of the treatment administered to an individual suffering from said cancer.

27. Use according to item 26 of one or more peptide sequences derived from the biomarkers selected from TTHY (SEQ ID 1 ), ACY1 (SEQ ID 2), AKR1A1 (SEQ ID 3), ALDOB (SEQ ID 4), ANXA4 (SEQ ID 5), BIEA (SEQ ID 6), BLVRB (SEQ ID 7), CATD H (SEQ ID 8), CATD K (SEQ ID 9), CO3 (SEQ ID 10, SEQ ID 11 ), CPGL1 (SEQ ID 12), ENOA (SEQ ID 13, SEQ ID 14), FRIL (SEQ ID 15), GDIB (SEQ ID 16), GPX3 (SEQ ID 17), GSHB (SEQ ID 18), GSTP1 (SEQ ID 19), IDHC (SEQ ID 20), LMAN2 (SEQ ID 21), LY6G6E (SEQ ID 22), MASP2 (SEQ ID 23), NADC (SEQ ID 24), NAPSA (SEQ ID 25), PA2G4 (SEQ ID 26), PARK7 (SEQ ID 27), PCBP1 (SEQ ID 28), PDC6I (SEQ ID 29), PPAL (SEQ ID 30), PRDX2 (SEQ ID 31), PTD012 (SEQ ID 32), QPCT (SEQ ID 33), SBP1 (SEQ ID 34), STIP1 (SEQ ID 35), TALDO (SEQ ID 36), WDR1 (SEQ ID 37), AMY (SEQ ID 38, SEQ ID 39, SEQ ID 40), APOA1 (SEQ ID 41), GSN40 (SEQ ID 42), GSN80 (SEQ ID 43) and RETBP (SEQ ID 44) or a transcriptional or a post-translational variant thereof.

28. Use according to item 27 of one or more peptide sequences derived from the biomarkers selected from TTHY, ACY1, AKR1A1, ALDOB, ANXA4, BIEA, BLVRB, CATD H, CATD K, CO3, CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC6I, PPAL, PRDX2, PTD012, QPCT, SBP1, STIP1, TALDO and WDR1 or a transcriptional or a post-translational variant thereof.

29. Use according to item 27 of one or more peptide sequences derived from the biomarkers selected from TTHY, AMY, APOA1, GSN40 and GSN80 or a transcriptional or a post-translational variant thereof.

30. Use according to item 27 of one or more peptide sequences derived from the biomarkers selected from AMY, APOA1, GSN40 and GSN80 or a transcriptional or a post-translational variant thereof and one or more peptide sequences derived from the biomarkers selected from TTHY, ACY1, AKR1A1, ALDOB, ANXA4, BIEA, BLVRB, CATD H, CATD K, CO3, CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC6I, PPAL, PRDX2, PTD012, QPCT, SBP1, STIP1, TALDO and WDR1 or a transcriptional or a post-translational variant thereof.

31. Use according to item 27 of one or more peptide sequences derived from the biomarkers selected from CATD H, CATD K, ENOA, GSHB, GSTP1, IDHC, PRDX2 and TTHY or a transcriptional or a post-translational variant thereof and one or more peptide sequences derived from the biomarkers selected from ACY1, AKR1A1, ALDOB, ANXA4, BIEA, BLVRB, C03, CPGL1, FRIL, GDIB, GPX3, LMAN2, LY6G6E, MASP2, NADC, NAPSA, PA2G4, PARK7, PCBP1, PDC6I, PPAL, PTD012, QPCT, SBP1, STIP1, TALDO, WDR1, AMY, APOA1, GSN40, GSN80 and RETBP or a transcriptional or a post-translational variant thereof.

32. Use according to item 27 of one or more peptide sequences derived from the biomarkers selected from TTHY, CATD H, CATD K, ENOA, GSTP1, IDHC, MASP2, PRDX2, AMY, APOA1, GSN40, GSN80 and RETBP or a transcriptional or a post-translational variant thereof.

33. Use according to any of items 27 to 32, wherein variations in the measurement of these biomarkers in a urine test sample in comparison to the corresponding standard value in normal urine are indicative of transitional cell carcinoma of the bladder.

34. Use according to any of items 27 to 32 of at least two peptide sequences.

35. Use according to any of items 27 to 32 of at least three peptide sequences.

36. Use according to any of items 27 to 32 of at least four peptide sequences.

37. Use according to any of items 27 to 32 of at least five peptide sequences.

38. Use according to item 27 of the peptide sequences derived from APOA1, GSN40 and TTHY or the respective transcriptional or post-translational variants thereof.

39. Use according to item 27 of the peptide sequences derived from APOA1, GSN40, GSN80 and TTHY or the respective transcriptional or post-translational variants thereof.

40. Use according to item 27 of the peptide sequences derived from AMY, AP0A1, GSN40, GSN80 and TTHY or the respective transcriptional or post- translational variants thereof.

41. Use according to item 27 of the peptide sequences derived from CATD H, ENOA, GSTP1, MASP2, PRDX2 and TTHY or the respective transcriptional or post-translational variants thereof.

42. Use according to item 27 of the peptide sequences derived from AMY, APOA1, CATD K, ENOA, IDHC, PRDX2 and TTHY or the respective transcriptional or post-translational variants thereof.

43. Use of one or more nucleotides or peptide sequences derived from one or more proteins selected from the set as defined in item 2, alone or in any combination, in methods to screen for, identify, develop and/or evaluate the efficacy of therapeutic agents to treat transitional cell carcinoma of the bladder.

44. A kit to perform a method as defined in any of items 1 to 25 comprising 1) at least one antibody that specifically recognises one of the proteins of item 2 and 2) a carrier in a suitable packaging.

45. A kit according to item 44 that is employed to detect the presence of transitional cell carcinoma of the bladder, to determine the stage or severity of this cancer, to assess the lack of disease after surgical resection, to establish the diagnosis and/or prognosis of this cancer and/or to monitor the effect of the treatment administered to an individual suffering from said cancer.

46. A kit according to items 44 or 45 comprising a biochip.

47. A kit according to item 46, wherein the biochip comprises antibodies for the detection of proteins selected from the set as defined in item 2.

## Claims

1. A non-invasive *in vitro* method that comprises: a) detecting and quantifying at least the biomarker gelsolin, which is abbreviated as GSN80 and consists of SEQ ID 43, present in a urine test sample from an individual; and b) comparing the measurement obtained in a) in the test urine sample to the corresponding standard value in normal urine, wherein variations in the measurement obtained in a) compared to the corresponding standard value in normal urine are indicative of transitional cell carcinoma of the bladder.

2. Method of claim 1, wherein the said biomarker is GSN80 and one or more biomarkers selected from the group consisting of transthyretin, which is abbreviated as TTHY and consists of SEQ ID 1; aminoacylase-1, which is abbreviated as ACY1 and consists of SEQ ID 2; aldehyde reductase, which is abbreviated as AKR1A1 and consists of SEQ ID 3; aldolase B, which is abbreviated as ALDOB and consists of SEQ ID 4; annexin A4 which is abbreviated as ANXA4 and consists of SEQ ID 5; biliverdin reductase A, which is abbreviated as BIEA and consists of SEQ ID 6; flavin reductase, which is abbreviated as BLVRB and consists of SEQ ID 7; cathepsin D H, which is abbreviated as CATD H and consists of SEQ ID 8; cathepsin D K, which is abbreviated as CATD K and consists of SEQ ID 9; complement C3, which is abbreviated as CO3 and consists of SEQ ID 10, SEQ ID 11; cytosolic nonspecific dipeptidase, which is abbreviated as CPGL1 and consists of SEQ ID 12; alpha enolase, which is abbreviated as ENOA and consists of SEQ ID 13, SEQ ID 14; ferritin light chain, which is abbreviated as FRIL and consists of SEQ ID 15; rab GDP dissociation inhibitor beta, which is abbreviated as GDIB and consists of SEQ ID 16; plasma glutathione peroxidase, which is abbreviated as GPX3 and consists of SEQ ID 17; glutathione synthetase, which is abbreviated as GSHB and consists of SEQ ID 18; glutathione S-transferase P, which is abbreviated as GSTP1 and consists of SEQ ID 19; cytoplasmic isocitrate dehydrogenase, which is abbreviated as IDHC and consists of SEQ ID 20; vesicular integral-membrane protein VIP36 precursor, which is abbreviated as LMAN2 and consists of SEQ ID 21; lymphocyte antigen 6 complex, which is abbreviated as LY6G6E and consists of SEQ ID 22; mannan-binding lectin serine protease 2, which is abbreviated as MASP2 and consists of SEQ ID 23; nicotinate-nucleotide pyrophosphorylase, which is abbreviated as NADC and consists of SEQ ID 24; napsin A, which is abbreviated as NAPSA and consists of SEQ ID 25; proliferation-associated protein 2G4, which is abbreviated as PA2G4 and consists of SEQ ID 26; oncogene DJ1, which is abbreviated as PARK7 and consists of SEQ ID 27; poly{rC)-binding protein 1, which is abbreviated as PCBP1 and consists of SEQ ID 28; programmed cell death 6-interacting protein, which is abbreviated as PDC6I and consists of SEQ ID 29; lysosomal acid phosphatase precursor, which is abbreviated as PPAL and consists of SEQ ID 30; peroxiredoxin 2, which is abbreviated as PRDX2 and consists of SEQ ID 31; PTD012 protein, which is abbreviated as PTD012 and consists of SEQ ID 32; glutaminyl-peptide cyclotransferase, which is abbreviated as QPCT and consists of SEQ ID 33; selenium-binding protein 1, which is abbreviated as SBP1 and consists of SEQ ID 34; stress-induced-phosphoprotein 1, which is abbreviated as STIP1 and consists of SEQ ID 35; transaldolase, which is abbreviated as TALDO and consists of SEQ ID 36; WD-repeat protein 1, which is abbreviated as WDR1 and consists of SEQ ID 37; alpha-amylase, which is abbreviated as AMY and consists of SEQ ID 38, SEQ ID 39, SEQ ID 40; apolipoprotein A-1, which is abbreviated as APOA1 and consists of SEQ ID 41; gelsolin, which is abbreviated as GSN40 and consists of SEQ ID 42 and plasma retinol-binding protein, which is abbreviated as RETBP and consists of SEQ ID 44 or any sequence with at least 95% identity with the described sequences.

3. Method according to claims 1 or 2, wherein the step a) comprises detecting and quantifying GSN80 and one or more biomarkers selected from the group consisting of either:

   1) TTHY, AMY, GSN40 and APOA1 or a transcriptional or any sequence with at least 95% identity with the described sequences, or
   2) AMY, GSN40, APOA1, TTHY, ACY1, AKR1A1, ALDOB, ANXA4, BIEA, BLVRB, CATD H, CATD K, C03, CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC61, PPAL, PRDX2, PTD012, QPCT, SBP1, STIP1, TALDO and WDR1 or a transcriptional or any sequence with at least 95% identity with the described sequences, or
   3) TTHY, CATD H, CATD K, ENOA, GSTP1, IDHC, MASP2, PRDX2, AMY, APOA1, GSN40 and RETBP or a transcriptional or any sequence with at least 95% identity with the described sequences.

4. Method according to any of the claims 1 to 3, wherein the step a) comprises detecting and quantifying at least three biomarkers, at least four biomarkers or at least five biomarkers selected from the group as defined in claim 2.

5. Method according to claims 1 or 2, wherein the step a) comprises either:

   1) detecting and quantifying the biomarkers GSN80, APOA1, GSN40 and TTHY or any sequence with at least 95% identity with the described sequences, or
   2) detecting and quantifying the biomarkers GSN80, AMY, APOA1, GSN40 and TTHY or any sequence with at least 95% identity with the described sequences.

6. Method according to any of the claims 1 to 5 which is employed to detect the presence of transitional cell carcinoma of the bladder, to determine the stage or severity of this cancer, to determine the progression of the disease when the measurement obtained in a) is compared from different urine samples from the same patient obtained at different times within the evolution of the disease, to assess the lack of disease after surgical resection, to establish the diagnosis or prognosis of this cancer or to monitor the efficacy of pharmacological or surgical treatments administered to an individual suffering from the said cancer, wherein the urine sample to be analyzed is obtained from an individual not previously diagnosed with transitional cell carcinoma of the bladder, from an individual who has been previously diagnosed with transitional cell carcinoma of the bladder or from an individual receiving treatment against transitional cell carcinoma of the bladder.

7. Method according to any of the claims 1 to 6 **characterised in that** the detection and quantification of biomarkers comprises a first step, in which the protein extract of the urine sample is placed in contact with a composition of at least two specific antibodies specific to one or more epitopes of the biomarkers of claim 1, wherein the said antibodies are of human origin, humanized or of non-human origin and selected from monoclonal or polyclonal antibodies, intact or recombinant fragments of antibodies, combibodies and Fab or scFv antibody fragments, and a second step, in which the resulting antibody-protein complexes are quantified.

8. Method according to claim 7 **characterised in that** in the detection and quantification of the resulting antibody-protein complexes, the techniques used are selected from the group consisting of: western blot, ELISA (Enzyme-Linked Immunosorbent assay), RIA (Radioimmunoassay), Competitive EIA (Competitive Enzyme Immunoassay), DAS-ELISA (Double Antibody Sandwich-ELISA), immunocytochemical or immunohistochemical techniques, techniques based on the use of biochips or protein microarrays that include specific antibodies, assays based on the precipitation of colloidal gold in formats such as dipsticks; or by affinity chromatography techniques, ligand binding assays or lectin binding assays.

9. In vitro use of at least a peptide sequence derived from the biomarker GSN80 in urine to detect the presence of transitional cell carcinoma of the bladder via urine analysis, to determine the stage of severity of this cancer, to assess the lack of disease after surgical resection, to establish the diagnosis and/or prognosis of this cancer and/or to monitor the effect of the treatment administered to an individual suffering from said cancer.

10. The use according to claim 8, wherein the peptide sequence is derived from biomarker GSN80 and one or more peptide sequences derived from the biomarkers selected from TTHY, which consists of SEQ ID 1; ACY1, which consists of SEQ ID 2; AKR1A1, which consists of SEQ ID 3; ALDOB, which consists of SEQ ID 4; ANXA4, which consists of SEQ ID 5; BIEA, which consists of SEQ ID 6; BLVRB, which consists of SEQ ID 7; CATD H, which consists of SEQ ID 8; CATD K, which consists of SEQ ID 9; CO3, which consists of SEQ ID 10, SEQ ID 11; CPGL1 which consists of SEQ ID 12; ENOA, which consists of SEQ ID 13, SEQ ID 14; FRIL, which consists of SEQ ID 15; GDIB, which consists of SEQ ID 16; GPX3, which consists of SEQ ID 17; GSHB, which consists of SEQ ID 18; GSTP1, which consists of SEQ ID 19; IDHC, which consists of which SEQ ID 20; LMAN2, which consists of SEQ ID 21; LY6G6E, which consists of SEQ ID 22; MASP2, which consists of SEQ ID 23; NADC, which consists of SEQ ID 24; NAPSA, which consists of SEQ ID 25; PA2G4, which consists of SEQ ID 26; PARK7, which consists of SEQ ID 27; PCBP1, which consists of SEQ ID 28; PDC6I, which consists of SEQ ID 29; PPAL, which consists of SEQ ID 30; PRDX2, which consists of SEQ ID 31; PTD012, which consists of SEQ ID 32; QPCT, which consists of SEQ ID 33; SBP1, which consists of SEQ ID 34; STIP1, which consists of SEQ ID 35; TALDO, which consists of SEQ ID 36; WDR1, which consists of SEQ ID 37; AMY, which consists of SEQ ID 38, SEQ ID 39, SEQ ID 40; APOA1, which consists of SEQ ID 41; GSN40, which consists of SEQ ID 42 and RETBP, which consists of SEQ ID 44 or a transcriptional or a post-translational variant thereof.

11. Use according to claims 9 or 10 of a peptide sequence derived from GSN80 and one or more peptide sequences derived from the biomarkers selected from the group consisting of either:

1) TTHY, AMY, GSN40 and APOA1 or a transcriptional or a post-translational variant thereof, or
2) AMY, GSN40, APOA1, TTHY, ACY1, AKR1A1, ALDOB, ANXA4, BIEA, BLVRB, CATD H, CATD K, C03, CPGL1, ENOA, FRIL, GDIB, GPX3, GSHB, GSTP1, IDHC, LMAN2, LY6G6E, MASP2, NADC, NAPSA, PA2G4, PARK7, PCBP1, PDC61, PPAL, PRDX2, PTD012, QPCT, SBP1, STIP1, TALDO and WDR1 or a transcriptional or a post-translational variant thereof, or
3) TTHY, CATD H, CATD K, ENOA, GSTP1, IDHC, MASP2, APOA1, AMY, GSN40, PRDX2 and RETBP or a transcriptional or a post-translational variant thereof.

12. Use according to any of claims 9 to 11, wherein variations in the measurement of GSN80 and one or more biomarkers in a urine test sample in comparison to the corresponding standard values in normal urine are indicative of transitional cell carcinoma of the bladder.

13. Use according to any of claims 9 to 11 of at least three peptide sequences or of at least four peptide sequences or of at least five peptide sequences.

14. Use according to claims 9 or 10 of the peptide sequences derived from either:

1) APOA1, GSN40, GSN80 and TTHY or the respective transcriptional or post-translational variants thereof, or
2) AMY, APOA1, GSN40, GSN80 and TTHY or the respective transcriptional or post-translational variants thereof.

15. Use of a nucleotide or peptide sequence derived from GSN80, alone or in combination with one or more nucleotide or peptide sequences derived from one or more biomarkers selected from the group as defined in claim 2 in methods to screen for, identify, develop and/or evaluate the efficacy of therapeutic agents to treat transitional cell carcinoma of the bladder.

16. A kit to perform a method as defined in any of claims 1 to 8 comprising 1) an antibody which specifically recognizes GSN80, or alternatively, an antibody which specifically recognises GSN80 and one or more antibodies which independently specifically recognise one or more of the biomarkers of claim 2, and 2) a carrier in a suitable packaging, wherein said kit optionally comprises a biochip which comprises antibodies for the detection of biomarkers selected from the group as defined in claim 2.

FIGURE 1A

FIGURE 1B

FIGURE 1C

FIGURE 1D

FIGURE 1E

FIGURE 2A

FIGURE 2B

FIGURE 2C

FIGURE 2D

FIGURE 2E

FIGURE 3

FIGURE 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BOMAN H. et al.** *J Urol,* 2002, vol. 167, 80-83
- **SOLOWAY M.S. et al.** *J Urol,* 1996, vol. 156, 363-367
- **PHAM H.T. et al.** *Cancer Res,* 1997, vol. 57, 778-783
- **PODE D. et al.** *J Urol,* 1999, vol. 161, 443-446
- **HALIM A.B. et al.** *Int J Biol Markers,* 1992, vol. 7, 234-239
- **WU X.R. et al.** *Cancer Res,* 1998, vol. 58, 1291-1297
- **GOHJI K. et al.** *J Clin Oncol,* 2000, vol. 18, 2963-2971
- **BUCHUMENSKY V. et al.** *J Urol,* 1998, vol. 160, 1971-1974
- **SÁNCHEZ-CARBAYO M. et al.** *Clin Cancer Res,* 2000, vol. 6, 3585-3594
- **MORRISON B.W. et al.** *J Biol Chem,* 1995, vol. 270, 2176-2182
- **LEE D.H. et al.** *Clinical Cancer Research,* 1998, vol. 4, 535-538
- **MEMON A. A. et al.** *Cancer Detect Prev,* 2005, vol. 29, 249-255
- **LANGBEIN, S. et al.** *Technol Cancer Res Treat,* 2006, 67-71
- **RASMUSSEN H. H. et al.** *J Urol,* 1996, vol. 155, 2113-2119
- **CELIS J. E. et al.** *Electrophoresis,* 1999, 300-309
- **MIYAKE H. et al.** *J Urol,* 2002, vol. 167, 1282-1287
- **OZER E. et al.** *Urology,* 1999, vol. 54, 50-5
- **LOACHIM E. et al.** *Anticancer Res,* 2002, vol. 22, 3383-8
- **DUNZENDORFER U. et al.** *Eur Urol,* 1980, vol. 6, 232-6
- **ICZKOWSKI K.A. et al.** *Histopathology,* 1999, vol. 35, 150-6
- **RASMUSSEN, H. H. et al.** *J Urol,* 1996, vol. 155, 2113-2119
- **HAGA K.** *Hokkaido Igaku Zasshi,* 2003, vol. 78, 29-37
- **CELIS A et al.** *Electrophoresis,* 1999, vol. 20, 355-61
- **BORONAT A. et al.** *J. Bacteriol.,* 1981, vol. 147, 181-85

SEQUENCE LISTING

<110>  Laboratorios SALVAT, SA

<120>  Non-invasive in vitro method to detect transitional cell
       carcinoma of the bladder

<130>  DIAG110891

<150>  EP 05384037
<151>  2005-10-11

<160>  44

<170>  PatentIn version 3.3

<210>  1
<211>  117
<212>  PRT
<213>  Homo sapiens

<400>  1

Pro Leu Met Val Lys Val Leu Asp Ala Val Arg Gly Ser Pro Ala Ile
1               5                   10                  15

Asn Val Ala Val His Val Phe Arg Lys Ala Ala Asp Asp Thr Trp Glu
            20                  25                  30

Pro Phe Ala Ser Gly Lys Thr Ser Glu Ser Gly Glu Leu His Gly Leu
        35                  40                  45

Thr Thr Glu Glu Glu Phe Val Glu Gly Ile Tyr Lys Val Glu Ile Asp
    50                  55                  60

Thr Lys Ser Tyr Trp Lys Ala Leu Gly Ile Ser Pro Phe His Glu His
65                  70                  75                  80

Ala Glu Val Val Phe Thr Ala Asn Asp Ser Gly Pro Arg Arg Tyr Thr
                85                  90                  95

Ile Ala Ala Leu Leu Ser Pro Tyr Ser Tyr Ser Thr Thr Ala Val Val
            100                 105                 110

Thr Asn Pro Lys Glu
            115


<210>  2
<211>  408
<212>  PRT
<213>  Homo sapiens

<400>  2

Met Thr Ser Lys Gly Pro Glu Glu Glu His Pro Ser Val Thr Leu Phe
1               5                   10                  15

Arg Gln Tyr Leu Arg Ile Arg Thr Val Gln Pro Lys Pro Asp Tyr Gly
            20                  25                  30

Ala Ala Val Ala Phe Phe Glu Glu Thr Ala Arg Gln Leu Gly Leu Gly
        35              40                  45

Cys Gln Lys Val Glu Val Ala Pro Gly Tyr Val Val Thr Val Leu Thr
        50              55                  60

Trp Pro Gly Thr Asn Pro Thr Leu Ser Ser Ile Leu Leu Asn Ser His
65              70              75                  80

Thr Asp Val Val Pro Val Phe Lys Glu His Trp Ser His Asp Pro Phe
                85              90                  95

Glu Ala Phe Lys Asp Ser Glu Gly Tyr Ile Tyr Ala Arg Gly Ala Gln
            100             105             110

Asp Met Lys Cys Val Ser Ile Gln Tyr Leu Glu Ala Val Arg Arg Leu
        115             120             125

Lys Val Glu Gly His Arg Phe Pro Arg Thr Ile His Met Thr Phe Val
    130             135             140

Pro Asp Glu Glu Val Gly Gly His Gln Gly Met Glu Leu Phe Val Gln
145             150             155             160

Arg Pro Glu Phe His Ala Leu Arg Ala Gly Phe Ala Leu Asp Glu Gly
            165             170             175

Ile Ala Asn Pro Thr Asp Ala Phe Thr Val Phe Tyr Ser Glu Arg Ser
            180             185             190

Pro Trp Trp Val Arg Val Thr Ser Thr Gly Arg Pro Gly His Ala Ser
        195             200             205

Arg Phe Met Glu Asp Thr Ala Ala Glu Lys Leu His Lys Val Val Asn
    210             215             220

Ser Ile Leu Ala Phe Arg Glu Lys Glu Trp Gln Arg Leu Gln Ser Asn
225             230             235             240

Pro His Leu Lys Glu Gly Ser Val Thr Ser Val Asn Leu Thr Lys Leu
            245             250             255

Glu Gly Gly Val Ala Tyr Asn Val Ile Pro Ala Thr Met Ser Ala Ser
            260             265             270

Phe Asp Phe Arg Val Ala Pro Asp Val Asp Phe Lys Ala Phe Glu Glu
        275             280             285

Gln Leu Gln Ser Trp Cys Gln Ala Ala Gly Glu Gly Val Thr Leu Glu
    290             295             300

24

```
Phe Ala Gln Lys Trp Met His Pro Gln Val Thr Pro Thr Asp Asp Ser
305             310             315             320

Asn Pro Trp Trp Ala Ala Phe Ser Arg Val Cys Lys Asp Met Asn Leu
            325             330             335

Thr Leu Glu Pro Glu Ile Met Pro Ala Ala Thr Asp Asn Arg Tyr Ile
            340             345             350

Arg Ala Val Gly Val Pro Ala Leu Gly Phe Ser Pro Met Asn Arg Thr
            355             360             365

Pro Val Leu Leu His Asp His Asp Glu Arg Leu His Glu Ala Val Phe
            370             375             380

Leu Arg Gly Val Asp Ile Tyr Thr Arg Leu Leu Pro Ala Leu Ala Ser
385             390             395             400

Val Pro Ala Leu Pro Ser Asp Ser
                405
```

```
<210>   3
<211>   324
<212>   PRT
<213>   Homo sapiens

<400>   3
```

```
Ala Ala Ser Cys Val Leu Leu His Thr Gly Gln Lys Met Pro Leu Ile
1               5               10              15

Gly Leu Gly Thr Trp Lys Ser Glu Pro Gly Gln Val Lys Ala Ala Val
            20              25              30

Lys Tyr Ala Leu Ser Val Gly Tyr Arg His Ile Asp Cys Ala Ala Ile
            35              40              45

Tyr Gly Asn Glu Pro Glu Ile Gly Glu Ala Leu Lys Glu Asp Val Gly
        50              55              60

Pro Gly Lys Ala Val Pro Arg Glu Glu Leu Phe Val Thr Ser Lys Leu
65              70              75              80

Trp Asn Thr Lys His His Pro Glu Asp Val Glu Pro Ala Leu Arg Lys
            85              90              95

Thr Leu Ala Asp Leu Gln Leu Glu Tyr Leu Asp Leu Tyr Leu Met His
            100             105             110

Trp Pro Tyr Ala Phe Glu Arg Gly Asp Asn Pro Phe Pro Lys Asn Ala
            115             120             125

Asp Gly Thr Ile Cys Tyr Asp Ser Thr His Tyr Lys Glu Thr Trp Lys
            130             135             140
```

```
Ala Leu Glu Ala Leu Val Ala Lys Gly Leu Val Gln Ala Leu Gly Leu
145             150             155             160

Ser Asn Phe Asn Ser Arg Gln Ile Asp Asp Ile Leu Ser Val Ala Ser
                165             170             175

Val Arg Pro Ala Val Leu Gln Val Glu Cys His Pro Tyr Leu Ala Gln
            180             185             190

Asn Glu Leu Ile Ala His Cys Gln Ala Arg Gly Leu Glu Val Thr Ala
        195             200             205

Tyr Ser Pro Leu Gly Ser Ser Asp Arg Ala Trp Arg Asp Pro Asp Glu
    210             215             220

Pro Val Leu Leu Glu Glu Pro Val Val Leu Ala Leu Ala Glu Lys Tyr
225             230             235             240

Gly Arg Ser Pro Ala Gln Ile Leu Leu Arg Trp Gln Val Gln Arg Lys
            245             250             255

Val Ile Cys Ile Pro Lys Ser Ile Thr Pro Ser Arg Ile Leu Gln Asn
            260             265             270

Ile Lys Val Phe Asp Phe Thr Phe Ser Pro Glu Glu Met Lys Gln Leu
        275             280             285

Asn Ala Leu Asn Lys Asn Trp Arg Tyr Ile Val Pro Met Leu Thr Val
    290             295             300

Asp Gly Lys Arg Val Pro Arg Asp Ala Gly His Pro Leu Tyr Pro Phe
305             310             315             320

Asn Asp Pro Tyr


<210>   4
<211>   364
<212>   PRT
<213>   Homo sapiens

<400>   4

Met Ala His Arg Phe Pro Ala Leu Thr Gln Glu Gln Lys Lys Glu Leu
1               5               10              15

Ser Glu Ile Ala Gln Ser Ile Val Ala Asn Gly Lys Gly Ile Leu Ala
            20              25              30

Ala Asp Glu Ser Val Gly Thr Met Gly Asn Arg Leu Gln Arg Ile Lys
        35              40              45
```

Val Glu Asn Thr Glu Glu Asn Arg Arg Gln Phe Arg Glu Ile Leu Phe
50                    55                    60

Ser Val Asp Ser Ser Ile Asn Gln Ser Ile Gly Gly Val Ile Leu Phe
65                    70                    75                    80

His Glu Thr Leu Tyr Gln Lys Asp Ser Gln Gly Lys Leu Phe Arg Asn
85                    90                    95

Ile Leu Lys Glu Lys Gly Ile Val Val Gly Ile Lys Leu Asp Gln Gly
100                    105                    110

Gly Ala Pro Leu Ala Gly Thr Asn Lys Glu Thr Thr Ile Gln Gly Leu
115                    120                    125

Asp Gly Leu Ser Glu Arg Cys Ala Gln Tyr Lys Lys Asp Gly Val Asp
130                    135                    140

Phe Gly Lys Trp Arg Ala Val Leu Arg Ile Ala Asp Gln Cys Pro Ser
145                    150                    155                    160

Ser Leu Ala Ile Gln Glu Asn Ala Asn Ala Leu Ala Arg Tyr Ala Ser
165                    170                    175

Ile Cys Gln Gln Asn Gly Leu Val Pro Ile Val Glu Pro Glu Val Ile
180                    185                    190

Pro Asp Gly Asp His Asp Leu Glu His Cys Gln Tyr Val Thr Glu Lys
195                    200                    205

Val Leu Ala Ala Val Tyr Lys Ala Leu Asn Asp His His Val Tyr Leu
210                    215                    220

Glu Gly Thr Leu Leu Lys Pro Asn Met Val Thr Ala Gly His Ala Cys
225                    230                    235                    240

Thr Lys Lys Tyr Thr Pro Glu Gln Val Ala Met Ala Thr Val Thr Ala
245                    250                    255

Leu His Arg Thr Val Pro Ala Ala Val Pro Gly Ile Cys Phe Leu Ser
260                    265                    270

Gly Gly Met Ser Glu Glu Asp Ala Thr Leu Asn Leu Asn Ala Ile Asn
275                    280                    285

Leu Cys Pro Leu Pro Lys Pro Trp Lys Leu Ser Phe Ser Tyr Gly Arg
290                    295                    300

Ala Leu Gln Ala Ser Ala Leu Ala Ala Trp Gly Gly Lys Ala Ala Asn
305                    310                    315                    320

Lys Glu Ala Thr Gln Glu Ala Phe Met Lys Arg Ala Met Ala Asn Cys

```
                    325                    330                    335

Gln Ala Ala Lys Gly Gln Tyr Val His Thr Gly Ser Ser Gly Ala Ala
            340                    345                350

Ser Thr Gln Ser Leu Phe Thr Ala Cys Tyr Thr Tyr
        355                    360


<210>  5
<211>  319
<212>  PRT
<213>  Homo sapiens

<400>  5

Met Ala Thr Lys Gly Gly Thr Val Lys Ala Ala Ser Gly Phe Asn Ala
1                   5                   10                  15

Met Glu Asp Ala Gln Thr Leu Arg Lys Ala Met Lys Gly Leu Gly Thr
            20                  25                  30

Asp Glu Asp Ala Ile Ile Ser Val Leu Ala Tyr Arg Asn Thr Ala Gln
            35                  40                  45

Arg Gln Glu Ile Arg Thr Ala Tyr Lys Ser Thr Ile Gly Arg Asp Leu
        50                  55                  60

Ile Asp Asp Leu Lys Ser Glu Leu Ser Gly Asn Phe Glu Gln Val Ile
65                  70                  75                  80

Val Gly Met Met Thr Pro Thr Val Leu Tyr Asp Val Gln Glu Leu Arg
                85                  90                  95

Arg Ala Met Lys Gly Ala Gly Thr Asp Glu Gly Cys Leu Ile Glu Ile
            100                 105                 110

Leu Ala Ser Arg Thr Pro Glu Glu Ile Arg Arg Ile Ser Gln Thr Tyr
        115                 120                 125

Gln Gln Gln Tyr Gly Arg Ser Leu Glu Asp Asp Ile Arg Ser Asp Thr
        130                 135                 140

Ser Phe Met Phe Gln Arg Val Leu Val Ser Leu Ser Ala Gly Gly Arg
145                 150                 155                 160

Asp Glu Gly Asn Tyr Leu Asp Asp Ala Leu Val Arg Gln Asp Ala Gln
                165                 170                 175

Asp Leu Tyr Glu Ala Gly Glu Lys Lys Trp Gly Thr Asp Glu Val Lys
            180                 185                 190

Phe Leu Thr Val Leu Cys Ser Arg Asn Arg Asn His Leu Leu His Val
        195                 200                 205
```

28

```
Phe Asp Glu Tyr Lys Arg Ile Ser Gln Lys Asp Ile Glu Gln Ser Ile
    210             215             220

Lys Ser Glu Thr Ser Gly Ser Phe Glu Asp Ala Leu Leu Ala Ile Val
225             230             235             240

Lys Cys Met Arg Asn Lys Ser Ala Tyr Phe Ala Glu Lys Leu Tyr Lys
                245             250             255

Ser Met Lys Gly Leu Gly Thr Asp Asp Asn Thr Leu Ile Arg Val Met
            260             265             270

Val Ser Arg Ala Glu Ile Asp Met Leu Asp Ile Arg Ala His Phe Lys
        275             280             285

Arg Leu Tyr Gly Lys Ser Leu Tyr Ser Phe Ile Lys Gly Asp Thr Ser
    290             295             300

Gly Asp Tyr Arg Lys Val Leu Leu Val Leu Cys Gly Gly Asp Asp
305             310             315
```

```
<210>  6
<211>  296
<212>  PRT
<213>  Homo sapiens

<400>  6
```

```
Met Asn Thr Glu Pro Glu Arg Lys Phe Gly Val Val Val Val Gly Val
1               5               10              15

Gly Arg Ala Gly Ser Val Arg Met Arg Asp Leu Arg Asn Pro His Pro
            20              25              30

Ser Ser Ala Phe Leu Asn Leu Ile Gly Phe Val Ser Arg Arg Glu Leu
        35              40              45

Gly Ser Ile Asp Gly Val Gln Gln Ile Ser Leu Glu Asp Ala Leu Ser
        50              55              60

Ser Gln Glu Val Glu Val Ala Tyr Ile Cys Ser Glu Ser Ser Ser His
65              70              75              80

Glu Asp Tyr Ile Arg Gln Phe Leu Asn Ala Gly Lys His Val Leu Val
                85              90              95

Glu Tyr Pro Met Thr Leu Ser Leu Ala Ala Ala Gln Glu Leu Trp Glu
            100             105             110

Leu Ala Glu Gln Lys Gly Lys Val Leu His Glu Glu His Val Glu Leu
        115             120             125

Leu Met Glu Glu Phe Ala Phe Leu Lys Lys Glu Val Val Gly Lys Asp
```

```
                130                      135                      140

        Leu Leu Lys Gly Ser Leu Leu Phe Thr Ala Gly Pro Leu Glu Glu Glu
        145                 150                 155                 160

        Arg Phe Gly Phe Pro Ala Phe Ser Gly Ile Ser Arg Leu Thr Trp Leu
                        165                 170                 175

        Val Ser Leu Phe Gly Glu Leu Ser Leu Val Ser Ala Thr Leu Glu Glu
                    180                 185                 190

        Arg Lys Glu Asp Gln Tyr Met Lys Met Thr Val Cys Leu Glu Thr Glu
                    195                 200                 205

        Lys Lys Ser Pro Leu Ser Trp Ile Glu Glu Lys Gly Pro Gly Leu Lys
                210                 215                 220

        Arg Asn Arg Tyr Leu Ser Phe His Phe Lys Ser Gly Ser Leu Glu Asn
        225                 230                 235                 240

        Val Pro Asn Val Gly Val Asn Lys Asn Ile Phe Leu Lys Asp Gln Asn
                        245                 250                 255

        Ile Phe Val Gln Lys Leu Leu Gly Gln Phe Ser Glu Lys Glu Leu Ala
                    260                 265                 270

        Ala Glu Lys Lys Arg Ile Leu His Cys Leu Gly Leu Ala Glu Glu Ile
                    275                 280                 285

        Gln Lys Tyr Cys Cys Ser Arg Lys
                290                 295
```

```
<210>   7
<211>   206
<212>   PRT
<213>   Homo sapiens

<400>   7
```

```
        Met Ala Val Lys Lys Ile Ala Ile Phe Gly Ala Thr Gly Gln Thr Gly
        1               5                   10                  15

        Leu Thr Thr Leu Ala Gln Ala Val Gln Ala Gly Tyr Glu Val Thr Val
                    20                  25                  30

        Leu Val Arg Asp Ser Ser Arg Leu Pro Ser Glu Gly Pro Arg Pro Ala
                35                  40                  45

        His Val Val Val Gly Asp Val Leu Gln Ala Ala Asp Val Asp Lys Thr
                50                  55                  60

        Val Ala Gly Gln Asp Ala Val Ile Val Leu Leu Gly Thr Arg Asn Asp
        65                  70                  75                  80
```

```
Leu Ser Pro Thr Thr Val Met Ser Glu Gly Ala Arg Asn Ile Val Ala
                85              90              95

Ala Met Lys Ala His Gly Val Asp Lys Val Val Ala Cys Thr Ser Ala
            100             105             110

Phe Leu Leu Trp Asp Pro Thr Lys Val Pro Pro Arg Leu Gln Ala Val
            115             120             125

Thr Asp Asp His Ile Arg Met His Lys Val Leu Arg Glu Ser Gly Leu
    130             135             140

Lys Tyr Val Ala Val Met Pro Pro His Ile Gly Asp Gln Pro Leu Thr
145             150             155             160

Gly Ala Tyr Thr Val Thr Leu Asp Gly Arg Gly Pro Ser Arg Val Ile
                165             170             175

Ser Lys His Asp Leu Gly His Phe Met Leu Arg Cys Leu Thr Thr Asp
            180             185             190

Glu Tyr Asp Gly His Ser Thr Tyr Pro Ser His Gln Tyr Gln
        195             200             205
```

```
<210>   8
<211>   241
<212>   PRT
<213>   Homo sapiens

<400>   8
```

```
Gly Gly Val Lys Val Glu Arg Gln Val Phe Gly Glu Ala Thr Lys Gln
1               5               10              15

Pro Gly Ile Thr Phe Ile Ala Ala Lys Phe Asp Gly Ile Leu Gly Met
            20              25              30

Ala Tyr Pro Arg Ile Ser Val Asn Asn Val Leu Pro Val Phe Asp Asn
            35              40              45

Leu Met Gln Gln Lys Leu Val Asp Gln Asn Ile Phe Ser Phe Tyr Leu
        50              55              60

Ser Arg Asp Pro Asp Ala Gln Pro Gly Gly Glu Leu Met Leu Gly Gly
65              70              75              80

Thr Asp Ser Lys Tyr Tyr Lys Gly Ser Leu Ser Tyr Leu Asn Val Thr
                85              90              95

Arg Lys Ala Tyr Trp Gln Val His Leu Asp Gln Val Glu Val Ala Ser
            100             105             110

Gly Leu Thr Leu Cys Lys Glu Gly Cys Glu Ala Ile Val Asp Thr Gly
```

```
              115                    120                    125

Thr Ser Leu Met Val Gly Pro Val Asp Glu Val Arg Glu Leu Gln Lys
    130                 135                 140

Ala Ile Gly Ala Val Pro Leu Ile Gln Gly Glu Tyr Met Ile Pro Cys
145                 150                 155                 160

Glu Lys Val Ser Thr Leu Pro Ala Ile Thr Leu Lys Leu Gly Gly Lys
                165                 170                 175

Gly Tyr Lys Leu Ser Pro Glu Asp Tyr Thr Leu Lys Val Ser Gln Ala
            180                 185                 190

Gly Lys Thr Leu Cys Leu Ser Gly Phe Met Gly Met Asp Ile Pro Pro
        195                 200                 205

Pro Ser Gly Pro Leu Trp Ile Leu Gly Asp Val Phe Ile Gly Arg Tyr
    210                 215                 220

Tyr Thr Val Phe Asp Arg Asp Asn Asn Arg Val Gly Phe Ala Glu Ala
225                 230                 235                 240

Ala
```

```
<210>    9
<211>    412
<212>    PRT
<213>    Homo sapiens

<400>    9

Met Gln Pro Ser Ser Leu Leu Pro Leu Ala Leu Cys Leu Leu Ala Ala
1               5                   10                  15

Pro Ala Ser Ala Leu Val Arg Ile Pro Leu His Lys Phe Thr Ser Ile
                20                  25                  30

Arg Arg Thr Met Ser Glu Val Gly Gly Ser Val Glu Asp Leu Ile Ala
            35                  40                  45

Lys Gly Pro Val Ser Lys Tyr Ser Gln Ala Val Pro Ala Val Thr Glu
        50                  55                  60

Gly Pro Ile Pro Glu Val Leu Lys Asn Tyr Met Asp Ala Gln Tyr Tyr
65                  70                  75                  80

Gly Glu Ile Gly Ile Gly Thr Pro Pro Gln Cys Phe Thr Val Val Phe
                85                  90                  95

Asp Thr Gly Ser Ser Asn Leu Trp Val Pro Ser Ile His Cys Lys Leu
            100                 105                 110
```

Leu Asp Ile Ala Cys Trp Ile His His Lys Tyr Asn Ser Asp Lys Ser
115 120 125

Ser Thr Tyr Val Lys Asn Gly Thr Ser Phe Asp Ile His Tyr Gly Ser
130 135 140

Gly Ser Leu Ser Gly Tyr Leu Ser Gln Asp Thr Val Ser Val Pro Cys
145 150 155 160

Gln Ser Ala Ser Ser Ala Ser Ala Leu Gly Gly Val Lys Val Glu Arg
165 170 175

Gln Val Phe Gly Glu Ala Thr Lys Gln Pro Gly Ile Thr Phe Ile Ala
180 185 190

Ala Lys Phe Asp Gly Ile Leu Gly Met Ala Tyr Pro Arg Ile Ser Val
195 200 205

Asn Asn Val Leu Pro Val Phe Asp Asn Leu Met Gln Gln Lys Leu Val
210 215 220

Asp Gln Asn Ile Phe Ser Phe Tyr Leu Ser Arg Asp Pro Asp Ala Gln
225 230 235 240

Pro Gly Gly Glu Leu Met Leu Gly Gly Thr Asp Ser Lys Tyr Tyr Lys
245 250 255

Gly Ser Leu Ser Tyr Leu Asn Val Thr Arg Lys Ala Tyr Trp Gln Val
260 265 270

His Leu Asp Gln Val Glu Val Ala Ser Gly Leu Thr Leu Cys Lys Glu
275 280 285

Gly Cys Glu Ala Ile Val Asp Thr Gly Thr Ser Leu Met Val Gly Pro
290 295 300

Val Asp Glu Val Arg Glu Leu Gln Lys Ala Ile Gly Ala Val Pro Leu
305 310 315 320

Ile Gln Gly Glu Tyr Met Ile Pro Cys Glu Lys Val Ser Thr Leu Pro
325 330 335

Ala Ile Thr Leu Lys Leu Gly Gly Lys Gly Tyr Lys Leu Ser Pro Glu
340 345 350

Asp Tyr Thr Leu Lys Val Ser Gln Ala Gly Lys Thr Leu Cys Leu Ser
355 360 365

Gly Phe Met Gly Met Asp Ile Pro Pro Pro Ser Gly Pro Leu Trp Ile
370 375 380

33

Leu Gly Asp Val Phe Ile Gly Arg Tyr Tyr Thr Val Phe Asp Arg Asp
385                 390                 395                 400

Asn Asn Arg Val Gly Phe Ala Glu Ala Ala Arg Leu
                405                 410

<210>   10
<211>   1663
<212>   PRT
<213>   Homo sapiens

<400>   10

Met Gly Pro Thr Ser Gly Pro Ser Leu Leu Leu Leu Leu Thr His His
1               5                   10                  15

Leu Pro Leu Ala Leu Gly Ser Pro Met Tyr Ser Ile Ile Thr Pro Asn
            20                  25                  30

Ile Leu Arg Leu Glu Ser Glu Glu Thr Met Val Leu Glu Ala His Asp
        35                  40                  45

Ala Gln Gly Asp Val Pro Val Thr Val Thr Val His Asp Phe Pro Gly
        50                  55                  60

Lys Lys Leu Val Leu Ser Ser Glu Lys Thr Val Leu Thr Pro Ala Thr
65                  70                  75                  80

Asn His Met Gly Asn Val Thr Phe Thr Ile Pro Ala Asn Arg Glu Phe
                85                  90                  95

Lys Ser Glu Lys Gly Arg Asn Lys Phe Val Thr Val Gln Ala Thr Phe
            100                 105                 110

Gly Thr Gln Val Val Glu Lys Val Val Leu Val Ser Leu Gln Ser Gly
            115                 120                 125

Tyr Leu Phe Ile Gln Thr Asp Lys Thr Ile Tyr Thr Pro Gly Ser Thr
        130                 135                 140

Val Leu Tyr Arg Ile Phe Thr Val Asn His Lys Leu Leu Pro Val Gly
145                 150                 155                 160

Arg Thr Val Met Val Asn Ile Glu Asn Pro Glu Gly Ile Pro Val Lys
                165                 170                 175

Gln Asp Ser Leu Ser Ser Gln Asn Gln Leu Gly Val Leu Pro Leu Ser
            180                 185                 190

Trp Asp Ile Pro Glu Leu Val Asn Met Gly Gln Trp Lys Ile Arg Ala
            195                 200                 205

Tyr Tyr Glu Asn Ser Pro Gln Gln Val Phe Ser Thr Glu Phe Glu Val
        210                 215                 220

Lys Glu Tyr Val Leu Pro Ser Phe Glu Val Ile Val Glu Pro Thr Glu
225                 230             235             240

Lys Phe Tyr Tyr Ile Tyr Asn Glu Lys Gly Leu Glu Val Thr Ile Thr
            245             250             255

Ala Arg Phe Leu Tyr Gly Lys Lys Val Glu Gly Thr Ala Phe Val Ile
            260             265             270

Phe Gly Ile Gln Asp Gly Glu Gln Arg Ile Ser Leu Pro Glu Ser Leu
            275             280             285

Lys Arg Ile Pro Ile Glu Asp Gly Ser Gly Glu Val Val Leu Ser Arg
        290             295             300

Lys Val Leu Leu Asp Gly Val Gln Asn Leu Arg Ala Glu Asp Leu Val
305             310             315             320

Gly Lys Ser Leu Tyr Val Ser Ala Thr Val Ile Leu His Ser Gly Ser
            325             330             335

Asp Met Val Gln Ala Glu Arg Ser Gly Ile Pro Ile Val Thr Ser Pro
            340             345             350

Tyr Gln Ile His Phe Thr Lys Thr Pro Lys Tyr Phe Lys Pro Gly Met
            355             360             365

Pro Phe Asp Leu Met Val Phe Val Thr Asn Pro Asp Gly Ser Pro Ala
        370             375             380

Tyr Arg Val Pro Val Ala Val Gln Gly Glu Asp Thr Val Gln Ser Leu
385             390             395             400

Thr Gln Gly Asp Gly Val Ala Lys Leu Ser Ile Asn Thr His Pro Ser
            405             410             415

Gln Lys Pro Leu Ser Ile Thr Val Arg Thr Lys Lys Gln Glu Leu Ser
            420             425             430

Glu Ala Glu Gln Ala Thr Arg Thr Met Gln Ala Leu Pro Tyr Ser Thr
            435             440             445

Val Gly Asn Ser Asn Asn Tyr Leu His Leu Ser Val Leu Arg Thr Glu
        450             455             460

Leu Arg Pro Gly Glu Thr Leu Asn Val Asn Phe Leu Leu Arg Met Asp
465             470             475             480

Arg Ala His Glu Ala Lys Ile Arg Tyr Tyr Thr Tyr Leu Ile Met Asn
            485             490             495

35

Lys Gly Arg Leu Leu Lys Ala Gly Arg Gln Val Arg Glu Pro Gly Gln
        500                   505                   510

Asp Leu Val Val Leu Pro Leu Ser Ile Thr Thr Asp Phe Ile Pro Ser
        515                   520                   525

Phe Arg Leu Val Ala Tyr Tyr Thr Leu Ile Gly Ala Ser Gly Gln Arg
    530                   535                   540

Glu Val Val Ala Asp Ser Val Trp Val Asp Val Lys Asp Ser Cys Val
545                   550                   555                   560

Gly Ser Leu Val Val Lys Ser Gly Gln Ser Glu Asp Arg Gln Pro Val
                565                   570                   575

Pro Gly Gln Gln Met Thr Leu Lys Ile Glu Gly Asp His Gly Ala Arg
            580                   585                   590

Val Val Leu Val Ala Val Asp Lys Gly Val Phe Val Leu Asn Lys Lys
        595                   600                   605

Asn Lys Leu Thr Gln Ser Lys Ile Trp Asp Val Val Glu Lys Ala Asp
    610                   615                   620

Ile Gly Cys Thr Pro Gly Ser Gly Lys Asp Tyr Ala Gly Val Phe Ser
625                   630                   635                   640

Asp Ala Gly Leu Thr Phe Thr Ser Ser Gly Gln Gln Thr Ala Gln
                645                   650                   655

Arg Ala Glu Leu Gln Cys Pro Gln Pro Ala Ala Arg Arg Arg Arg Ser
            660                   665                   670

Val Gln Leu Thr Glu Lys Arg Met Asp Lys Val Gly Lys Tyr Pro Lys
        675                   680                   685

Glu Leu Arg Lys Cys Cys Glu Asp Gly Met Arg Glu Asn Pro Met Arg
    690                   695                   700

Phe Ser Cys Gln Arg Arg Thr Arg Phe Ile Ser Leu Gly Glu Ala Cys
705                   710                   715                   720

Lys Lys Val Phe Leu Asp Cys Cys Asn Tyr Ile Thr Glu Leu Arg Arg
                725                   730                   735

Gln His Ala Arg Ala Ser His Leu Gly Leu Ala Arg Ser Asn Leu Asp
            740                   745                   750

Glu Asp Ile Ile Ala Glu Glu Asn Ile Val Ser Arg Ser Glu Phe Pro
        755                   760                   765

```
Glu Ser Trp Leu Trp Asn Val Glu Asp Leu Lys Glu Pro Pro Lys Asn
    770             775             780

Gly Ile Ser Thr Lys Leu Met Asn Ile Phe Leu Lys Asp Ser Ile Thr
785             790             795             800

Thr Trp Glu Ile Leu Ala Val Ser Met Ser Asp Lys Lys Gly Ile Cys
            805             810             815

Val Ala Asp Pro Phe Glu Val Thr Val Met Gln Asp Phe Phe Ile Asp
            820             825             830

Leu Arg Leu Pro Tyr Ser Val Val Arg Asn Glu Gln Val Glu Ile Arg
        835             840             845

Ala Val Leu Tyr Asn Tyr Arg Gln Asn Gln Glu Leu Lys Val Arg Val
    850             855             860

Glu Leu Leu His Asn Pro Ala Phe Cys Ser Leu Ala Thr Thr Lys Arg
865             870             875             880

Arg His Gln Gln Thr Val Thr Ile Pro Pro Lys Ser Ser Leu Ser Val
            885             890             895

Pro Tyr Val Ile Val Pro Leu Lys Thr Gly Leu Gln Glu Val Glu Val
            900             905             910

Lys Ala Ala Val Tyr His His Phe Ile Ser Asp Gly Val Arg Lys Ser
        915             920             925

Leu Lys Val Val Pro Glu Gly Ile Arg Met Asn Lys Thr Val Ala Val
    930             935             940

Arg Thr Leu Asp Pro Glu Arg Leu Gly Arg Glu Gly Val Gln Lys Glu
945             950             955             960

Asp Ile Pro Pro Ala Asp Leu Ser Asp Gln Val Pro Asp Thr Glu Ser
            965             970             975

Glu Thr Arg Ile Leu Leu Gln Gly Thr Pro Val Ala Gln Met Thr Glu
            980             985             990

Asp Ala Val Asp Ala Glu Arg Leu Lys His Leu Ile Val Thr Pro Ser
        995             1000            1005

Gly Cys Gly Glu Gln Asn Met Ile Gly Met Thr Pro Thr Val Ile
    1010            1015            1020

Ala Val His Tyr Leu Asp Glu Thr Glu Gln Trp Glu Lys Phe Gly
    1025            1030            1035

Leu Glu Lys Arg Gln Gly Ala Leu Glu Leu Ile Lys Lys Gly Tyr
```

1040 1045 1050

Thr Gln Gln Leu Ala Phe Arg Gln Pro Ser Ser Ala Phe Ala Ala
1055 1060 1065

Phe Val Lys Arg Ala Pro Ser Thr Trp Leu Thr Ala Tyr Val Val
1070 1075 1080

Lys Val Phe Ser Leu Ala Val Asn Leu Ile Ala Ile Asp Ser Gln
1085 1090 1095

Val Leu Cys Gly Ala Val Lys Trp Leu Ile Leu Glu Lys Gln Lys
1100 1105 1110

Pro Asp Gly Val Phe Gln Glu Asp Ala Pro Val Ile His Gln Glu
1115 1120 1125

Met Ile Gly Gly Leu Arg Asn Asn Asn Glu Lys Asp Met Ala Leu
1130 1135 1140

Thr Ala Phe Val Leu Ile Ser Leu Gln Glu Ala Lys Asp Ile Cys
1145 1150 1155

Glu Glu Gln Val Asn Ser Leu Pro Gly Ser Ile Thr Lys Ala Gly
1160 1165 1170

Asp Phe Leu Glu Ala Asn Tyr Met Asn Leu Gln Arg Ser Tyr Thr
1175 1180 1185

Val Ala Ile Ala Gly Tyr Ala Leu Ala Gln Met Gly Arg Leu Lys
1190 1195 1200

Gly Pro Leu Leu Asn Lys Phe Leu Thr Thr Ala Lys Asp Lys Asn
1205 1210 1215

Arg Trp Glu Asp Pro Gly Lys Gln Leu Tyr Asn Val Glu Ala Thr
1220 1225 1230

Ser Tyr Ala Leu Leu Ala Leu Leu Gln Leu Lys Asp Phe Asp Phe
1235 1240 1245

Val Pro Pro Val Val Arg Trp Leu Asn Glu Gln Arg Tyr Tyr Gly
1250 1255 1260

Gly Gly Tyr Gly Ser Thr Gln Ala Thr Phe Met Val Phe Gln Ala
1265 1270 1275

Leu Ala Gln Tyr Gln Lys Asp Ala Pro Asp His Gln Glu Leu Asn
1280 1285 1290

Leu Asp Val Ser Leu Gln Leu Pro Ser Arg Ser Ser Lys Ile Thr
1295 1300 1305

His Arg Ile His Trp Glu Ser Ala Ser Leu Leu Arg Ser Glu Glu
1310 1315 1320

Thr Lys Glu Asn Glu Gly Phe Thr Val Thr Ala Glu Gly Lys Gly
1325 1330 1335

Gln Gly Thr Leu Ser Val Val Thr Met Tyr His Ala Lys Ala Lys
1340 1345 1350

Asp Gln Leu Thr Cys Asn Lys Phe Asp Leu Lys Val Thr Ile Lys
1355 1360 1365

Pro Ala Pro Glu Thr Glu Lys Arg Pro Gln Asp Ala Lys Asn Thr
1370 1375 1380

Met Ile Leu Glu Ile Cys Thr Arg Tyr Arg Gly Asp Gln Asp Ala
1385 1390 1395

Thr Met Ser Ile Leu Asp Ile Ser Met Met Thr Gly Phe Ala Pro
1400 1405 1410

Asp Thr Asp Asp Leu Lys Gln Leu Ala Asn Gly Val Asp Arg Tyr
1415 1420 1425

Ile Ser Lys Tyr Glu Leu Asp Lys Ala Phe Ser Asp Arg Asn Thr
1430 1435 1440

Leu Ile Ile Tyr Leu Asp Lys Val Ser His Ser Glu Asp Asp Cys
1445 1450 1455

Leu Ala Phe Lys Val His Gln Tyr Phe Asn Val Glu Leu Ile Gln
1460 1465 1470

Pro Gly Ala Val Lys Val Tyr Ala Tyr Tyr Asn Leu Glu Glu Ser
1475 1480 1485

Cys Thr Arg Phe Tyr His Pro Glu Lys Glu Asp Gly Lys Leu Asn
1490 1495 1500

Lys Leu Cys Arg Asp Glu Leu Cys Arg Cys Ala Glu Glu Asn Cys
1505 1510 1515

Phe Ile Gln Lys Ser Asp Asp Lys Val Thr Leu Glu Glu Arg Leu
1520 1525 1530

Asp Lys Ala Cys Glu Pro Gly Val Asp Tyr Val Tyr Lys Thr Arg
1535 1540 1545

Leu Val Lys Val Gln Leu Ser Asn Asp Phe Asp Glu Tyr Ile Met
1550 1555 1560

39

```
Ala Ile  Glu Gln Thr Ile Lys  Ser Gly Ser Asp Glu  Val Gln Val
    1565             1570             1575

Gly Gln  Gln Arg Thr Phe Ile  Ser Pro Ile Lys Cys  Arg Glu Ala
    1580             1585             1590

Leu Lys  Leu Glu Glu Lys Lys  His Tyr Leu Met Trp  Gly Leu Ser
    1595             1600             1605

Ser Asp  Phe Trp Gly Glu Lys  Pro Asn Leu Ser Tyr  Ile Ile Gly
    1610             1615             1620

Lys Asp  Thr Trp Val Glu His  Trp Pro Glu Glu Asp  Glu Cys Gln
    1625             1630             1635

Asp Glu  Glu Asn Gln Lys Gln  Cys Gln Asp Leu Gly  Ala Phe Thr
    1640             1645             1650

Glu Ser  Met Val Val Phe Gly  Cys Pro Asn
    1655             1660
```

```
<210>  11
<211>  1663
<212>  PRT
<213>  Homo sapiens

<400>  11
```

```
Met Gly Pro Thr Ser Gly Pro Ser Leu Leu Leu Leu Leu Thr His
1               5             10                  15

Leu Pro Leu Ala Leu Gly Ser Pro Met Tyr Ser Ile Ile Thr Pro Asn
            20              25              30

Ile Leu Arg Leu Glu Ser Glu Glu Thr Met Val Leu Glu Ala His Asp
        35              40              45

Ala Gln Gly Asp Val Pro Val Thr Val Thr Val His Asp Phe Pro Gly
    50              55              60

Lys Lys Leu Val Leu Ser Ser Glu Lys Thr Val Leu Thr Pro Ala Thr
65              70              75              80

Asn His Met Gly Asn Val Thr Phe Thr Ile Pro Ala Asn Arg Glu Phe
            85              90              95

Lys Ser Glu Lys Gly Arg Asn Lys Phe Val Thr Val Gln Ala Thr Phe
            100             105             110

Gly Thr Gln Val Val Glu Lys Val Val Leu Val Ser Leu Gln Ser Gly
        115             120             125

Tyr Leu Phe Ile Gln Thr Asp Lys Thr Ile Tyr Thr Pro Gly Ser Thr
```

|     |     |     | 130 |     |     |     | 135 |     |     |     | 140 |     |     |
|-----|-----|-----|-----|-----|-----|-----|-----|-----|-----|-----|-----|-----|-----|

Val Leu Tyr Arg Ile Phe Thr Val Asn His Lys Leu Leu Pro Val Gly
145                 150                 155                 160

Arg Thr Val Met Val Asn Ile Glu Asn Pro Glu Gly Ile Pro Val Lys
                165                 170                 175

Gln Asp Ser Leu Ser Ser Gln Asn Gln Leu Gly Val Leu Pro Leu Ser
                180                 185                 190

Trp Asp Ile Pro Glu Leu Val Asn Met Gly Gln Trp Lys Ile Arg Ala
            195                 200                 205

Tyr Tyr Glu Asn Ser Pro Gln Gln Val Phe Ser Thr Glu Phe Glu Val
    210                 215                 220

Lys Glu Tyr Val Leu Pro Ser Phe Glu Val Ile Val Glu Pro Thr Glu
225                 230                 235                 240

Lys Phe Tyr Tyr Ile Tyr Asn Glu Lys Gly Leu Glu Val Thr Ile Thr
            245                 250                 255

Ala Arg Phe Leu Tyr Gly Lys Lys Val Glu Gly Thr Ala Phe Val Ile
            260                 265                 270

Phe Gly Ile Gln Asp Gly Glu Gln Arg Ile Ser Leu Pro Glu Ser Leu
        275                 280                 285

Lys Arg Ile Pro Ile Glu Asp Gly Ser Gly Glu Val Val Leu Ser Arg
    290                 295                 300

Lys Val Leu Leu Asp Gly Val Gln Asn Pro Arg Ala Glu Asp Leu Val
305                 310                 315                 320

Gly Lys Ser Leu Tyr Val Ser Ala Thr Val Ile Leu His Ser Gly Ser
                325                 330                 335

Asp Met Val Gln Ala Glu Arg Ser Gly Ile Pro Ile Val Thr Ser Pro
            340                 345                 350

Tyr Gln Ile His Phe Thr Lys Thr Pro Lys Tyr Phe Lys Pro Gly Met
        355                 360                 365

Pro Phe Asp Leu Met Val Phe Val Thr Asn Pro Asp Gly Ser Pro Ala
    370                 375                 380

Tyr Arg Val Pro Val Ala Val Gln Gly Glu Asp Thr Val Gln Ser Leu
385                 390                 395                 400

Thr Gln Gly Asp Gly Val Ala Lys Leu Ser Ile Asn Thr His Pro Ser
                405                 410                 415

41

Gln Lys Pro Leu Ser Ile Thr Val Arg Thr Lys Lys Gln Glu Leu Ser
            420                 425                 430

Glu Ala Glu Gln Ala Thr Arg Thr Met Gln Ala Leu Pro Tyr Ser Thr
            435                 440                 445

Val Gly Asn Ser Asn Asn Tyr Leu His Leu Ser Val Leu Arg Thr Glu
            450                 455                 460

Leu Arg Pro Gly Glu Thr Leu Asn Val Asn Phe Leu Leu Arg Met Asp
465                 470                 475                 480

Arg Ala His Glu Ala Lys Ile Arg Tyr Tyr Thr Tyr Leu Ile Met Asn
                485                 490                 495

Lys Gly Arg Leu Leu Lys Ala Gly Arg Gln Val Arg Glu Pro Gly Gln
            500                 505                 510

Asp Leu Val Val Leu Pro Leu Ser Ile Thr Thr Asp Phe Ile Pro Ser
            515                 520                 525

Phe Arg Leu Val Ala Tyr Tyr Thr Leu Ile Gly Ala Ser Gly Gln Arg
    530                 535                 540

Glu Val Val Ala Asp Ser Val Trp Val Asp Val Lys Asp Ser Cys Val
545                 550                 555                 560

Gly Ser Leu Val Val Lys Ser Gly Gln Ser Glu Asp Arg Gln Pro Val
                565                 570                 575

Pro Gly Gln Gln Met Thr Leu Lys Ile Glu Gly Asp His Gly Ala Arg
            580                 585                 590

Val Val Leu Val Ala Val Asp Lys Gly Val Phe Val Leu Asn Lys Lys
            595                 600                 605

Asn Lys Leu Thr Gln Ser Lys Ile Trp Asp Val Val Glu Lys Ala Asp
    610                 615                 620

Ile Gly Cys Thr Pro Gly Ser Gly Lys Asp Tyr Ala Gly Val Phe Ser
625                 630                 635                 640

Asp Ala Gly Leu Thr Phe Thr Ser Ser Gly Gln Gln Thr Ala Gln
                645                 650                 655

Arg Ala Glu Leu Gln Cys Pro Gln Pro Ala Ala Arg Arg Arg Arg Ser
            660                 665                 670

Val Gln Leu Thr Glu Lys Arg Met Asp Lys Val Gly Lys Tyr Pro Lys
            675                 680                 685

Glu Leu Arg Lys Cys Cys Glu Asp Gly Met Arg Glu Asn Pro Met Arg
690                 695             700

Phe Ser Cys Gln Arg Arg Thr Arg Phe Ile Ser Leu Gly Glu Ala Cys
705             710             715                 720

Lys Lys Val Phe Leu Asp Cys Cys Asn Tyr Ile Thr Glu Leu Arg Arg
725             730                 735

Gln His Ala Arg Ala Ser His Leu Gly Leu Ala Arg Ser Asn Leu Asp
740             745             750

Glu Asp Ile Ile Ala Glu Glu Asn Ile Val Ser Arg Ser Glu Phe Pro
755             760             765

Glu Ser Trp Leu Trp Asn Val Glu Asp Leu Lys Glu Pro Pro Lys Asn
770             775             780

Gly Ile Ser Thr Lys Leu Met Asn Ile Phe Leu Lys Asp Ser Ile Thr
785             790             795             800

Thr Trp Glu Ile Leu Ala Val Ser Met Ser Asp Lys Lys Gly Ile Cys
805             810             815

Val Ala Asp Pro Phe Glu Val Thr Val Met Gln Asp Phe Phe Ile Asp
820             825             830

Leu Arg Leu Pro Tyr Ser Val Val Arg Asn Glu Gln Val Glu Ile Arg
835             840             845

Ala Val Leu Tyr Asn Tyr Arg Gln Asn Gln Glu Leu Lys Val Arg Val
850             855             860

Glu Leu Leu His Asn Pro Ala Phe Cys Ser Leu Ala Thr Thr Lys Arg
865             870             875             880

Arg His Gln Gln Thr Val Thr Ile Pro Pro Lys Ser Ser Leu Ser Val
885             890             895

Pro Tyr Val Ile Val Pro Leu Lys Thr Gly Leu Gln Glu Val Glu Val
900             905             910

Lys Ala Ala Val Tyr His His Phe Ile Ser Asp Gly Val Arg Lys Ser
915             920             925

Leu Lys Val Val Pro Glu Gly Ile Arg Met Asn Lys Thr Val Ala Val
930             935             940

Arg Thr Leu Asp Pro Glu Arg Leu Gly Arg Glu Gly Val Gln Lys Glu
945             950             955             960

43

```
Asp Ile Pro Pro Ala Asp Leu Ser Asp Gln Val Pro Asp Thr Glu Ser
            965                 970                    975

Glu Thr Arg Ile Leu Leu Gln Gly Thr Pro Val Ala Gln Met Thr Glu
            980                 985                    990

Asp Ala Val Asp Ala Glu Arg Leu  Lys His Leu Ile Val  Thr Pro Ser
            995             1000                1005

Gly Cys  Gly Glu Gln Asn Met  Ile Gly Met Thr Pro  Thr Val Ile
    1010                1015                1020

Ala Val  His Tyr Leu Asp Glu  Thr Glu Gln Trp Glu  Lys Phe Gly
    1025                1030                1035

Leu Glu  Lys Arg Gln Gly Ala  Leu Glu Leu Ile Lys  Lys Gly Tyr
    1040                1045                1050

Thr Gln  Gln Leu Ala Phe Arg  Gln Pro Ser Ser Ala  Phe Ala Ala
    1055                1060                1065

Phe Val  Lys Arg Ala Pro Ser  Thr Trp Leu Thr Ala  Tyr Val Val
    1070                1075                1080

Lys Val  Phe Ser Leu Ala Val  Asn Leu Ile Ala Ile  Asp Ser Gln
    1085                1090                1095

Val Leu  Cys Gly Ala Val Lys  Trp Leu Ile Leu Glu  Lys Gln Lys
    1100                1105                1110

Pro Asp  Gly Val Phe Gln Glu  Asp Ala Pro Val Ile  His Gln Glu
    1115                1120                1125

Met Ile  Gly Gly Leu Arg Asn  Asn Asn Glu Lys Asp  Met Ala Leu
    1130                1135                1140

Thr Ala  Phe Val Leu Ile Ser  Leu Gln Glu Ala Lys  Asp Ile Cys
    1145                1150                1155

Glu Glu  Gln Val Asn Ser Leu  Pro Gly Ser Ile Thr  Lys Ala Gly
    1160                1165                1170

Asp Phe  Leu Glu Ala Asn Tyr  Met Asn Leu Gln Arg  Ser Tyr Thr
    1175                1180                1185

Val Ala  Ile Ala Gly Tyr Ala  Leu Ala Gln Met Gly  Arg Leu Lys
    1190                1195                1200

Gly Pro  Leu Leu Asn Lys Phe  Leu Thr Thr Ala Lys  Asp Lys Asn
    1205                1210                1215

Arg Trp  Glu Asp Pro Gly Lys  Gln Leu Tyr Asn Val  Glu Ala Thr
```

|     |     |     |     |
| --- | --- | --- | --- |
| 1220 | 1225 | 1230 |

Ser Tyr Ala Leu Leu Ala Leu Leu Gln Leu Lys Asp Phe Asp Phe
    1235            1240            1245

Val Pro Pro Val Val Arg Trp Leu Asn Glu Gln Arg Tyr Tyr Gly
    1250            1255            1260

Gly Gly Tyr Gly Ser Thr Gln Ala Thr Phe Met Val Phe Gln Ala
    1265            1270            1275

Leu Ala Gln Tyr Gln Lys Asp Ala Pro Asp His Gln Glu Leu Asn
    1280            1285            1290

Leu Asp Val Ser Leu Gln Leu Pro Ser Arg Ser Ser Lys Ile Thr
    1295            1300            1305

His Arg Ile His Trp Glu Ser Ala Ser Leu Leu Arg Ser Glu Glu
    1310            1315            1320

Thr Lys Glu Asn Glu Gly Phe Thr Val Thr Ala Glu Gly Lys Gly
    1325            1330            1335

Gln Gly Thr Leu Ser Val Val Thr Met Tyr His Ala Lys Ala Lys
    1340            1345            1350

Asp Gln Leu Thr Cys Asn Lys Phe Asp Leu Lys Val Thr Ile Lys
    1355            1360            1365

Pro Ala Pro Glu Thr Glu Lys Arg Pro Gln Asp Ala Lys Asn Thr
    1370            1375            1380

Met Ile Leu Glu Ile Cys Thr Arg Tyr Arg Gly Asp Gln Asp Ala
    1385            1390            1395

Thr Met Ser Ile Leu Asp Ile Ser Met Met Thr Gly Phe Ala Pro
    1400            1405            1410

Asp Thr Asp Asp Leu Lys Gln Leu Ala Asn Gly Val Asp Arg Tyr
    1415            1420            1425

Ile Ser Lys Tyr Glu Leu Asp Lys Ala Phe Ser Asp Arg Asn Thr
    1430            1435            1440

Leu Ile Ile Tyr Leu Asp Lys Val Ser His Ser Glu Asp Asp Cys
    1445            1450            1455

Leu Ala Phe Lys Val His Gln Tyr Phe Asn Val Glu Leu Ile Gln
    1460            1465            1470

Pro Gly Ala Val Lys Val Tyr Ala Tyr Tyr Asn Leu Glu Glu Ser
    1475            1480            1485

```
Cys Thr  Arg Phe Tyr His Pro  Glu Lys Glu Asp Gly  Lys Leu Asn
    1490              1495             1500

Lys Leu  Cys Arg Asp Glu Leu  Cys Arg Cys Ala Glu  Glu Asn Cys
    1505              1510             1515

Phe Ile  Gln Lys Ser Asp Asp  Lys Val Thr Leu Glu  Glu Arg Leu
    1520              1525             1530

Asp Lys  Ala Cys Glu Pro Gly  Val Asp Tyr Val Tyr  Lys Thr Arg
    1535              1540             1545

Leu Val  Lys Val Gln Leu Ser  Asn Asp Phe Asp Glu  Tyr Ile Met
    1550              1555             1560

Ala Ile  Glu Gln Thr Ile Lys  Ser Gly Ser Asp Glu  Val Gln Val
    1565              1570             1575

Gly Gln  Gln Arg Thr Phe Ile  Ser Pro Ile Lys Cys  Arg Glu Ala
    1580              1585             1590

Leu Lys  Leu Glu Glu Lys Lys  His Tyr Leu Met Trp  Gly Leu Ser
    1595              1600             1605

Ser Asp  Phe Trp Gly Glu Lys  Pro Asn Leu Ser Tyr  Ile Ile Gly
    1610              1615             1620

Lys Asp  Thr Trp Val Glu His  Trp Pro Glu Glu Asp  Glu Cys Gln
    1625              1630             1635

Asp Glu  Glu Asn Gln Lys Gln  Cys Gln Asp Leu Gly  Ala Phe Thr
    1640              1645             1650

Glu Ser  Met Val Val Phe Gly  Cys Pro Asn
    1655              1660


<210>  12
<211>  475
<212>  PRT
<213>  Homo sapiens

<400>  12

Met Ala Ala Leu Thr Thr Leu Phe Lys Tyr Ile Asp Glu Asn Gln Asp
1             5                 10                15

Arg Tyr Ile Lys Lys Leu Ala Lys Trp Val Ala Ile Gln Ser Val Ser
            20                25                30

Ala Trp Pro Glu Lys Arg Gly Glu Ile Arg Arg Met Met Glu Val Ala
            35                40                45
```

Ala Ala Asp Val Lys Gln Leu Gly Gly Ser Val Glu Leu Val Asp Ile
        50              55                 60

Gly Lys Gln Lys Leu Pro Asp Gly Ser Glu Ile Pro Leu Pro Pro Ile
65              70                 75                 80

Leu Leu Gly Arg Leu Gly Ser Asp Pro Gln Lys Lys Thr Val Cys Ile
            85              90                 95

Tyr Gly His Leu Asp Val Gln Pro Ala Ala Leu Glu Asp Gly Trp Asp
            100             105             110

Ser Glu Pro Phe Thr Leu Val Glu Arg Asp Gly Lys Leu His Gly Arg
        115             120             125

Gly Ser Thr Asp Asp Lys Gly Pro Val Ala Gly Trp Ile Asn Ala Leu
    130             135             140

Glu Ala Tyr Gln Lys Thr Gly Gln Glu Ile Pro Val Asn Val Arg Phe
145             150             155             160

Cys Leu Glu Gly Met Glu Glu Ser Gly Ser Glu Gly Leu Asp Glu Leu
            165             170             175

Ile Phe Ala Arg Lys Asp Thr Phe Phe Lys Asp Val Asp Tyr Val Cys
            180             185             190

Ile Ser Asp Asn Tyr Trp Leu Gly Lys Lys Lys Pro Cys Ile Thr Tyr
        195             200             205

Gly Leu Arg Gly Ile Cys Tyr Phe Phe Ile Glu Val Glu Cys Ser Asn
    210             215             220

Lys Asp Leu His Ser Gly Val Tyr Gly Gly Ser Val His Glu Ala Met
225             230             235             240

Thr Asp Leu Ile Leu Leu Met Gly Ser Leu Val Asp Lys Arg Gly Asn
            245             250             255

Ile Leu Ile Pro Gly Ile Asn Glu Ala Val Ala Ala Val Thr Glu Glu
            260             265             270

Glu His Lys Leu Tyr Asp Asp Ile Asp Phe Asp Ile Glu Glu Phe Ala
        275             280             285

Lys Asp Val Gly Ala Gln Ile Leu Leu His Ser His Lys Lys Asp Ile
        290             295             300

Leu Met His Arg Trp Arg Tyr Pro Ser Leu Ser Leu His Gly Ile Glu
305             310             315             320

Gly Ala Phe Ser Gly Ser Gly Ala Lys Thr Val Ile Pro Arg Lys Val

47

```
                325                    330                    335
Val Gly Lys Phe Ser Ile Arg Leu Val Pro Asn Met Thr Pro Glu Val
        340                 345                 350

Val Gly Glu Gln Val Thr Ser Tyr Leu Thr Lys Lys Phe Ala Glu Leu
        355                 360                 365

Arg Ser Pro Asn Glu Phe Lys Val Tyr Met Gly His Gly Gly Lys Pro
        370                 375                 380

Trp Val Ser Asp Phe Ser His Pro His Tyr Leu Ala Gly Arg Arg Ala
385                 390                 395                 400

Met Lys Thr Val Phe Gly Val Glu Pro Asp Leu Thr Arg Glu Gly Gly
                405                 410                 415

Ser Ile Pro Val Thr Leu Thr Phe Gln Glu Ala Thr Gly Lys Asn Val
            420                 425                 430

Met Leu Leu Pro Val Gly Ser Ala Asp Asp Gly Ala His Ser Gln Asn
        435                 440                 445

Glu Lys Leu Asn Arg Tyr Asn Tyr Ile Glu Gly Thr Lys Met Leu Ala
    450                 455                 460

Ala Tyr Leu Tyr Glu Val Ser Gln Leu Lys Asp
465                 470                 475

<210>   13
<211>   434
<212>   PRT
<213>   Homo sapiens

<400>   13

Met Ser Ile Leu Lys Ile His Ala Arg Glu Ile Phe Asp Ser Arg Gly
1               5                   10                  15

Asn Pro Thr Val Glu Val Asp Leu Phe Thr Ser Lys Gly Leu Phe Arg
            20                  25                  30

Ala Ala Val Pro Ser Gly Ala Ser Thr Gly Ile Tyr Glu Ala Leu Glu
        35                  40                  45

Leu Arg Asp Asn Asp Lys Thr Arg Tyr Met Gly Lys Gly Val Ser Lys
    50                  55                  60

Ala Val Glu His Ile Asn Lys Thr Ile Ala Pro Ala Leu Val Ser Lys
65                  70                  75                  80

Lys Leu Asn Val Thr Glu Gln Glu Lys Ile Asp Lys Leu Met Ile Glu
                85                  90                  95
```

Met Asp Gly Thr Glu Asn Lys Ser Lys Phe Gly Ala Asn Ala Ile Leu
100 105 110

Gly Val Ser Leu Ala Val Cys Lys Ala Gly Ala Val Glu Lys Gly Val
115 120 125

Pro Leu Tyr Arg His Ile Ala Asp Leu Ala Gly Asn Ser Glu Val Ile
130 135 140

Leu Pro Val Pro Ala Phe Asn Val Ile Asn Gly Gly Ser His Ala Gly
145 150 155 160

Asn Lys Leu Ala Met Gln Glu Phe Met Ile Leu Pro Val Gly Ala Ala
165 170 175

Asn Phe Arg Glu Ala Met Arg Ile Gly Ala Glu Val Tyr His Asn Leu
180 185 190

Lys Asn Val Ile Lys Glu Lys Tyr Gly Lys Asp Ala Thr Asn Val Gly
195 200 205

Asp Glu Gly Gly Phe Ala Pro Asn Ile Leu Glu Asn Lys Glu Gly Leu
210 215 220

Glu Leu Leu Lys Thr Ala Ile Gly Lys Ala Gly Tyr Thr Asp Lys Val
225 230 235 240

Val Ile Gly Met Asp Val Ala Ala Ser Glu Phe Phe Arg Ser Gly Lys
245 250 255

Tyr Asp Leu Asp Phe Lys Ser Pro Asp Asp Pro Ser Arg Tyr Ile Ser
260 265 270

Pro Asp Gln Leu Ala Asp Leu Tyr Lys Ser Phe Ile Lys Asp Tyr Pro
275 280 285

Val Val Ser Ile Glu Asp Pro Phe Asp Gln Asp Asp Trp Gly Ala Trp
290 295 300

Gln Lys Phe Thr Ala Ser Ala Gly Ile Gln Val Val Gly Asp Asp Leu
305 310 315 320

Thr Val Thr Asn Pro Lys Arg Ile Ala Lys Ala Val Asn Glu Lys Ser
325 330 335

Cys Asn Cys Leu Leu Leu Lys Val Asn Gln Ile Gly Ser Val Thr Glu
340 345 350

Ser Leu Gln Ala Cys Lys Leu Ala Gln Ala Asn Gly Trp Gly Val Met
355 360 365

Val Ser His Arg Ser Gly Glu Thr Glu Asp Thr Phe Ile Ala Asp Leu
370 375 380

Val Val Gly Leu Cys Thr Gly Gln Ile Lys Thr Gly Ala Pro Cys Arg
385 390 395 400

Ser Glu Arg Leu Ala Lys Tyr Asn Gln Leu Leu Arg Ile Glu Glu Glu
405 410 415

Leu Gly Ser Lys Ala Lys Phe Ala Gly Arg Asn Phe Arg Asn Pro Leu
420 425 430

Ala Lys


<210> 14
<211> 434
<212> PRT
<213> Homo sapiens

<400> 14

Met Ser Ile Leu Lys Ile His Ala Arg Glu Ile Phe Asp Ser Arg Gly
1 5 10 15

Asn Pro Thr Val Glu Val Asp Leu Phe Thr Ser Lys Gly Leu Phe Arg
20 25 30

Ala Ala Val Pro Ser Gly Ala Ser Thr Gly Ile Tyr Glu Ala Leu Glu
35 40 45

Leu Arg Asp Asn Asp Lys Ala Arg Tyr Met Gly Lys Gly Val Ser Lys
50 55 60

Ala Val Glu His Ile Asn Lys Thr Ile Ala Pro Ala Leu Val Ser Lys
65 70 75 80

Lys Leu Asn Val Thr Glu Gln Glu Lys Ile Asp Lys Leu Met Ile Glu
85 90 95

Met Asp Gly Thr Glu Asn Lys Ser Lys Phe Gly Ala Asn Ala Ile Leu
100 105 110

Gly Val Ser Leu Ala Val Cys Lys Ala Gly Ala Val Glu Lys Gly Val
115 120 125

Pro Leu Tyr Arg His Ile Ala Asp Leu Ala Gly Asn Ser Glu Val Ile
130 135 140

Leu Pro Val Pro Ala Phe Asn Val Ile Asn Gly Gly Ser His Ala Gly
145 150 155 160

Asn Lys Leu Ala Met Gln Glu Phe Met Ile Leu Pro Val Gly Ala Ala
165 170 175

Asn Phe Arg Glu Ala Met Arg Ile Gly Ala Gly Val Tyr His Asn Leu
180 185 190

Lys Asn Val Ile Lys Glu Lys Tyr Gly Lys Asp Ala Thr Asn Val Gly
195 200 205

Asp Glu Gly Gly Phe Ala Pro Asn Ile Leu Glu Asn Lys Glu Gly Leu
210 215 220

Glu Leu Leu Lys Thr Ala Ile Gly Lys Ala Gly Tyr Thr Asp Lys Val
225 230 235 240

Val Ile Gly Met Asp Val Ala Ala Ser Glu Phe Phe Arg Ser Gly Lys
245 250 255

Tyr Asp Leu Asp Phe Lys Ser Pro Asp Asp Pro Ser Arg Tyr Ile Ser
260 265 270

Pro Asp Gln Leu Ala Asp Leu Tyr Lys Ser Phe Ile Lys Asp Tyr Pro
275 280 285

Val Val Ser Ile Glu Asp Pro Phe Asp Gln Asp Asp Trp Gly Ala Trp
290 295 300

Gln Lys Phe Thr Ala Ile Ala Gly Ile Gln Val Val Gly Asp Asp Leu
305 310 315 320

Thr Val Thr Asn Pro Lys Arg Ile Ala Lys Ala Val Asn Glu Lys Ser
325 330 335

Cys Asn Cys Leu Leu Leu Lys Val Asn Gln Ile Gly Ser Val Thr Glu
340 345 350

Ser Leu Gln Ala Cys Lys Leu Ala Gln Ala Asn Gly Trp Gly Val Met
355 360 365

Val Ser His Arg Ser Gly Glu Thr Glu Asp Thr Phe Ile Ala Asp Leu
370 375 380

Val Val Gly Leu Cys Thr Gly Gln Ile Lys Thr Gly Ala Pro Cys Arg
385 390 395 400

Ser Glu Arg Leu Ala Lys Tyr Asn Gln Leu Leu Arg Ile Glu Glu Glu
405 410 415

Leu Gly Ser Lys Ala Lys Phe Ala Gly Arg Asn Phe Arg Asn Pro Leu
420 425 430

Ala Lys

```
<210>   15
<211>   143
<212>   PRT
<213>   Homo sapiens

<400>   15

Ser Leu Gly Phe Tyr Phe Asp Arg Asp Asp Val Ala Leu Glu Gly Val
1               5                   10                  15

Ser His Phe Phe Arg Glu Leu Ala Glu Glu Lys Arg Glu Gly Tyr Glu
            20                  25                  30

Arg Leu Leu Lys Met Gln Asn Gln Arg Gly Gly Arg Ala Leu Phe Gln
            35                  40                  45

Asp Ile Lys Lys Pro Ala Glu Asp Glu Trp Gly Lys Thr Pro Asp Ala
        50                  55                  60

Met Lys Ala Ala Met Ala Leu Glu Lys Lys Leu Asn Gln Ala Leu Leu
65                  70                  75                  80

Asp Leu His Ala Leu Gly Ser Ala Arg Thr Asp Pro His Leu Cys Asp
                85                  90                  95

Phe Leu Glu Thr His Phe Leu Asp Glu Glu Val Lys Leu Ile Lys Lys
                100                 105                 110

Met Gly Asp His Leu Thr Asn Leu His Arg Leu Gly Gly Pro Glu Ala
            115                 120                 125

Gly Leu Gly Glu Tyr Leu Phe Glu Arg Leu Thr Leu Lys His Asp
            130                 135                 140


<210>   16
<211>   445
<212>   PRT
<213>   Homo sapiens

<400>   16

Met Asn Glu Glu Tyr Asp Val Ile Val Leu Gly Thr Gly Leu Thr Glu
1               5                   10                  15

Cys Ile Leu Ser Gly Ile Met Ser Val Asn Gly Lys Lys Val Leu His
            20                  25                  30

Met Asp Arg Asn Pro Tyr Tyr Gly Gly Glu Ser Ala Ser Ile Thr Pro
            35                  40                  45

Leu Glu Asp Leu Tyr Lys Arg Phe Lys Ile Pro Gly Ser Pro Pro Glu
        50                  55                  60

Ser Met Gly Arg Gly Arg Asp Trp Asn Val Asp Leu Ile Pro Lys Phe
65                  70                  75                  80
```

52

```
Leu Met Ala Asn Gly Gln Leu Val Lys Met Leu Leu Tyr Thr Glu Val
                 85                  90                  95

Thr Arg Tyr Leu Asp Phe Lys Val Thr Glu Gly Ser Phe Val Tyr Lys
            100                 105                 110

Gly Gly Lys Ile Tyr Lys Val Pro Ser Thr Glu Ala Glu Ala Leu Ala
            115                 120                 125

Ser Ser Leu Met Gly Leu Phe Glu Lys Arg Arg Phe Arg Lys Phe Leu
    130                 135                 140

Val Tyr Val Ala Asn Phe Asp Glu Lys Asp Pro Arg Thr Phe Glu Gly
145                 150                 155                 160

Ile Asp Pro Lys Lys Thr Thr Met Arg Asp Val Tyr Lys Lys Phe Asp
                165                 170                 175

Leu Gly Gln Asp Val Ile Asp Phe Thr Gly His Ala Leu Ala Leu Tyr
                180                 185                 190

Arg Thr Asp Asp Tyr Leu Asp Gln Pro Cys Tyr Glu Thr Ile Asn Arg
            195                 200                 205

Ile Lys Leu Tyr Ser Glu Ser Leu Ala Arg Tyr Gly Lys Ser Pro Tyr
        210                 215                 220

Leu Tyr Pro Leu Tyr Gly Leu Gly Glu Leu Pro Gln Gly Phe Ala Arg
225                 230                 235                 240

Leu Ser Ala Ile Tyr Gly Gly Thr Tyr Met Leu Asn Lys Pro Ile Glu
                245                 250                 255

Glu Ile Ile Val Gln Asn Gly Lys Val Ile Gly Val Lys Ser Glu Gly
                260                 265                 270

Glu Ile Ala Arg Cys Lys Gln Leu Ile Cys Asp Pro Ser Tyr Val Lys
            275                 280                 285

Asp Arg Val Glu Lys Val Gly Gln Val Ile Arg Val Ile Cys Ile Leu
        290                 295                 300

Ser His Pro Ile Lys Asn Thr Asn Asp Ala Asn Ser Cys Gln Ile Ile
305                 310                 315                 320

Ile Pro Gln Asn Gln Val Asn Arg Lys Ser Asp Ile Tyr Val Cys Met
                325                 330                 335

Ile Ser Phe Ala His Asn Val Ala Ala Gln Gly Lys Tyr Ile Ala Ile
                340                 345                 350
```

Val Ser Thr Thr Val Glu Thr Lys Glu Pro Glu Lys Glu Ile Arg Pro
        355             360             365

Ala Leu Glu Leu Leu Glu Pro Ile Glu Gln Lys Phe Val Ser Ile Ser
    370             375             380

Asp Leu Leu Val Pro Lys Asp Leu Gly Thr Glu Ser Gln Ile Phe Ile
385             390             395             400

Ser Arg Thr Tyr Asp Ala Thr Thr His Phe Glu Thr Thr Cys Asp Asp
            405             410             415

Ile Lys Asn Ile Tyr Lys Arg Met Thr Gly Ser Glu Phe Asp Phe Glu
            420             425             430

Glu Met Lys Arg Lys Lys Asn Asp Ile Tyr Gly Glu Asp
        435             440             445

<210>  17
<211>  145
<212>  PRT
<213>  Homo sapiens

<400>  17

Leu Asn Ala Leu Gln Glu Glu Leu Ala Pro Phe Gly Leu Val Ile Leu
1           5               10              15

Gly Phe Pro Cys Asn Gln Phe Gly Lys Gln Glu Pro Gly Glu Asn Ser
            20              25              30

Glu Ile Leu Pro Thr Leu Lys Tyr Val Arg Pro Gly Gly Gly Phe Val
        35              40              45

Pro Asn Phe Gln Leu Phe Glu Lys Gly Asp Val Asn Gly Glu Lys Glu
    50              55              60

Gln Lys Phe Tyr Thr Phe Leu Lys Asn Ser Cys Pro Pro Thr Ser Glu
65              70              75              80

Leu Leu Gly Thr Ser Asp Arg Leu Phe Trp Glu Pro Met Lys Val His
            85              90              95

Asp Ile Arg Trp Asn Phe Glu Lys Phe Leu Val Gly Pro Asp Gly Ile
        100             105             110

Pro Ile Met Arg Trp His His Arg Thr Thr Val Ser Asn Val Lys Met
        115             120             125

Asp Ile Leu Ser Tyr Met Arg Arg Gln Ala Ala Leu Gly Val Lys Arg
        130             135             140

Lys

145

<210> 18
<211> 474
<212> PRT
<213> Homo sapiens

<400> 18

```
Met Ala Thr Asn Trp Gly Ser Leu Leu Gln Asp Lys Gln Gln Leu Glu
1               5                   10                  15

Glu Leu Ala Arg Gln Ala Val Asp Arg Ala Leu Ala Glu Gly Val Leu
                20                  25                  30

Leu Arg Thr Ser Gln Glu Pro Thr Ser Ser Glu Val Val Ser Tyr Ala
            35                  40                  45

Pro Phe Thr Leu Phe Pro Ser Leu Val Pro Ser Ala Leu Leu Glu Gln
        50                  55                  60

Ala Tyr Ala Val Gln Met Asp Phe Asn Leu Leu Val Asp Ala Val Ser
65                  70                  75                  80

Gln Asn Ala Ala Phe Leu Glu Gln Thr Leu Ser Ser Thr Ile Lys Gln
                85                  90                  95

Asp Asp Phe Thr Ala Arg Leu Phe Asp Ile His Lys Gln Val Leu Lys
            100                 105                 110

Glu Gly Ile Ala Gln Thr Val Phe Leu Gly Leu Asn Arg Ser Asp Tyr
        115                 120                 125

Met Phe Gln Arg Ser Ala Asp Gly Ser Pro Ala Leu Lys Gln Ile Glu
    130                 135                 140

Ile Asn Thr Ile Ser Ala Ser Phe Gly Gly Leu Ala Ser Arg Thr Pro
145                 150                 155                 160

Ala Val His Arg His Val Leu Ser Val Leu Ser Lys Thr Lys Glu Ala
                165                 170                 175

Gly Lys Ile Leu Ser Asn Asn Pro Ser Lys Gly Leu Ala Leu Gly Ile
            180                 185                 190

Ala Lys Ala Trp Glu Leu Tyr Gly Ser Pro Asn Ala Leu Val Leu Leu
        195                 200                 205

Ile Ala Gln Glu Lys Glu Arg Asn Ile Phe Asp Gln Arg Ala Ile Glu
    210                 215                 220

Asn Glu Leu Leu Ala Arg Asn Ile His Val Ile Arg Arg Thr Phe Glu
225                 230                 235                 240
```

```
Asp Ile Ser Glu Lys Gly Ser Leu Asp Gln Asp Arg Arg Leu Phe Val
              245             250             255

Asp Gly Gln Glu Ile Ala Val Val Tyr Phe Arg Asp Gly Tyr Met Pro
          260             265             270

Arg Gln Tyr Ser Leu Gln Asn Trp Glu Ala Arg Leu Leu Leu Glu Arg
          275             280             285

Ser His Ala Ala Lys Cys Pro Asp Ile Ala Thr Gln Leu Ala Gly Thr
      290             295             300

Lys Lys Val Gln Gln Glu Leu Ser Arg Pro Gly Met Leu Glu Met Leu
305             310             315             320

Leu Pro Gly Gln Pro Glu Ala Val Ala Arg Leu Arg Ala Thr Phe Ala
              325             330             335

Gly Leu Tyr Ser Leu Asp Val Gly Glu Glu Gly Asp Gln Ala Ile Ala
          340             345             350

Glu Ala Leu Ala Ala Pro Ser Arg Phe Val Leu Lys Pro Gln Arg Glu
          355             360             365

Gly Gly Gly Asn Asn Leu Tyr Gly Glu Glu Met Val Gln Ala Leu Lys
      370             375             380

Gln Leu Lys Asp Ser Glu Glu Arg Ala Ser Tyr Ile Leu Met Glu Lys
385             390             395             400

Ile Glu Pro Glu Pro Phe Glu Asn Cys Leu Leu Arg Pro Gly Ser Pro
              405             410             415

Ala Arg Val Val Gln Cys Ile Ser Glu Leu Gly Ile Phe Gly Val Tyr
              420             425             430

Val Arg Gln Glu Lys Thr Leu Val Met Asn Lys His Val Gly His Leu
          435             440             445

Leu Arg Thr Lys Ala Ile Glu His Ala Asp Gly Gly Val Ala Ala Gly
      450             455             460

Val Ala Val Leu Asp Asn Pro Tyr Pro Val
465             470
```

```
<210>   19
<211>   208
<212>   PRT
<213>   Homo sapiens

<400>   19
```

```
Pro Pro Tyr Thr Val Val Tyr Phe Pro Val Arg Gly Arg Cys Ala Ala
```

56

```
        1                   5                       10                      15

        Leu Arg Met Leu Leu Ala Asp Gln Gly Gln Ser Trp Lys Glu Glu Val
                20                  25                  30

        Val Thr Val Glu Thr Trp Gln Glu Gly Ser Leu Lys Ala Ser Cys Leu
                35                  40                  45

        Tyr Gly Gln Leu Pro Lys Phe Gln Asp Gly Asp Leu Thr Leu Tyr Gln
            50                  55                  60

        Ser Asn Thr Ile Leu Arg His Leu Gly Arg Thr Leu Gly Leu Tyr Gly
        65                  70                  75                      80

        Lys Asp Gln Gln Glu Ala Ala Leu Val Asp Met Val Asn Asp Gly Val
                        85                  90                  95

        Glu Asp Leu Arg Cys Lys Tyr Ile Ser Leu Ile Tyr Thr Asn Tyr Glu
                    100                 105                 110

        Ala Gly Lys Asp Asp Tyr Val Lys Ala Leu Pro Gly Gln Leu Lys Pro
                115                 120                 125

        Phe Glu Thr Leu Leu Ser Gln Asn Gln Gly Gly Lys Thr Phe Ile Val
            130                 135                 140

        Gly Asp Gln Ile Ser Phe Ala Asp Tyr Asn Leu Leu Asp Leu Leu Leu
        145                 150                 155                 160

        Ile His Glu Val Leu Ala Pro Gly Cys Leu Asp Ala Phe Pro Leu Leu
                    165                 170                 175

        Ser Ala Tyr Val Gly Arg Leu Ser Ala Arg Pro Lys Leu Lys Ala Phe
                180                 185                 190

        Leu Ala Ser Pro Glu Tyr Val Asn Leu Pro Met Asp Glu Lys Ser Leu
                195                 200                 205

        <210>   20
        <211>   414
        <212>   PRT
        <213>   Homo sapiens

        <400>   20

        Met Ser Lys Lys Ile Ser Gly Gly Ser Val Val Glu Met Gln Gly Asp
        1               5                   10                  15

        Glu Met Thr Arg Ile Ile Trp Glu Leu Ile Lys Glu Lys Leu Ile Phe
                20                  25                  30

        Pro Tyr Val Glu Leu Asp Leu His Ser Tyr Asp Leu Gly Ile Glu Asn
                35                  40                  45
```

```
Arg Asp Ala Thr Asn Asp Gln Val Thr Lys Asp Ala Ala Glu Ala Ile
    50                55                60

Lys Lys His Asn Val Gly Val Lys Cys Ala Thr Ile Thr Pro Asp Glu
65                70                75                80

Lys Arg Val Glu Glu Phe Lys Leu Lys Gln Met Trp Lys Ser Pro Asn
            85                90                95

Gly Thr Ile Arg Asn Ile Leu Gly Gly Thr Val Phe Arg Glu Ala Ile
            100               105               110

Ile Cys Lys Asn Ile Pro Arg Leu Val Ser Gly Trp Val Lys Pro Ile
        115               120               125

Ile Ile Gly Arg His Ala Tyr Gly Asp Gln Tyr Arg Ala Thr Asp Phe
    130               135               140

Val Val Pro Gly Pro Gly Lys Val Glu Met Thr Tyr Thr Pro Ser Asp
145               150               155               160

Gly Thr Gln Lys Val Thr Tyr Leu Val His Asn Phe Glu Glu Gly Gly
            165               170               175

Gly Val Ala Met Gly Met Tyr Asn Gln Asp Lys Ser Ile Glu Asp Phe
            180               185               190

Ala His Ser Ser Phe Gln Met Ala Leu Ser Lys Gly Trp Pro Leu Tyr
        195               200               205

Leu Ser Thr Lys Asn Thr Ile Leu Lys Lys Tyr Asp Gly Arg Phe Lys
    210               215               220

Asp Ile Phe Gln Glu Ile Tyr Asp Lys Gln Tyr Lys Ser Gln Phe Glu
225               230               235               240

Ala Gln Lys Ile Trp Tyr Glu His Arg Leu Ile Asp Asp Met Val Ala
            245               250               255

Gln Ala Met Lys Ser Glu Gly Gly Phe Ile Trp Ala Cys Lys Asn Tyr
            260               265               270

Asp Gly Asp Val Gln Ser Asp Ser Val Ala Gln Gly Tyr Gly Ser Leu
        275               280               285

Gly Met Met Thr Ser Val Leu Val Cys Pro Asp Gly Lys Thr Val Glu
    290               295               300

Ala Glu Ala Ala His Gly Thr Val Thr Arg His Tyr Arg Met Tyr Gln
305               310               315               320
```

58

Lys Gly Gln Glu Thr Ser Thr Asn Pro Ile Ala Ser Ile Phe Ala Trp
              325         330                 335

Thr Arg Gly Leu Ala His Arg Ala Lys Leu Asp Asn Asn Lys Glu Leu
            340             345             350

Ala Phe Phe Ala Asn Ala Leu Glu Glu Val Ser Ile Glu Thr Ile Glu
        355             360             365

Ala Gly Phe Met Thr Lys Asp Leu Ala Ala Cys Ile Lys Gly Leu Pro
    370             375             380

Asn Val Gln Arg Ser Asp Tyr Leu Asn Thr Phe Glu Phe Met Asp Lys
385             390             395             400

Leu Gly Glu Asn Leu Lys Ile Lys Leu Ala Gln Ala Lys Leu
            405             410

<210> 21
<211> 356
<212> PRT
<213> Homo sapiens

<400> 21

Met Ala Ala Glu Gly Trp Ile Trp Arg Trp Gly Trp Gly Arg Arg Cys
1               5               10              15

Leu Gly Arg Pro Gly Leu Leu Gly Pro Gly Pro Gly Pro Thr Thr Pro
            20              25              30

Leu Phe Leu Leu Leu Leu Leu Gly Ser Val Thr Ala Asp Ile Thr Asp
        35              40              45

Gly Asn Ser Glu His Leu Lys Arg Glu His Ser Leu Ile Lys Pro Tyr
    50              55              60

Gln Gly Val Gly Ser Ser Ser Met Pro Leu Trp Asp Phe Gln Gly Ser
65              70              75              80

Thr Met Leu Thr Ser Gln Tyr Val Arg Leu Thr Pro Asp Glu Arg Ser
            85              90              95

Lys Glu Gly Ser Ile Trp Asn His Gln Pro Cys Phe Leu Lys Asp Trp
            100             105             110

Glu Met His Val His Phe Lys Val His Gly Thr Gly Lys Lys Asn Leu
        115             120             125

His Gly Asp Gly Ile Ala Leu Trp Tyr Thr Arg Asp Arg Leu Val Pro
    130             135             140

Gly Pro Val Phe Gly Ser Lys Asp Asn Phe His Gly Leu Ala Ile Phe
145             150             155             160

```
Leu Asp Thr Tyr Pro Asn Asp Glu Thr Thr Glu Arg Val Phe Pro Tyr
                165                 170                 175

Ile Ser Val Met Val Asn Asn Gly Ser Leu Ser Tyr Asp His Ser Lys
                180                 185                 190

Asp Gly Arg Trp Thr Glu Leu Ala Gly Cys Thr Ala Asp Phe Arg Asn
            195                 200                 205

Arg Asp His Asp Thr Phe Leu Ala Val Arg Tyr Ser Arg Gly Arg Leu
        210                 215                 220

Thr Val Met Thr Asp Leu Glu Asp Lys Asn Glu Trp Lys Asn Cys Ile
225                 230                 235                 240

Asp Ile Thr Gly Val Arg Leu Pro Thr Gly Tyr Tyr Phe Gly Ala Ser
                245                 250                 255

Ala Gly Thr Gly Asp Leu Ser Asp Asn His Asp Ile Ile Ser Met Lys
            260                 265                 270

Leu Phe Gln Leu Met Val Glu His Thr Pro Asp Glu Glu Ser Ile Asp
        275                 280                 285

Trp Thr Lys Ile Glu Pro Ser Val Asn Phe Leu Lys Ser Pro Lys Asp
    290                 295                 300

Asn Val Asp Asp Pro Thr Gly Asn Phe Arg Ser Gly Pro Leu Thr Gly
305                 310                 315                 320

Trp Arg Val Phe Leu Leu Leu Leu Cys Ala Leu Leu Gly Ile Val Val
                325                 330                 335

Cys Ala Val Val Gly Ala Val Val Phe Gln Lys Arg Gln Glu Arg Asn
            340                 345                 350

Lys Arg Phe Tyr
            355


<210>  22
<211>  128
<212>  PRT
<213>  Homo sapiens

<400>  22

Met Gly Thr Ser Ser Ile Phe Leu Cys Val Leu Phe Leu Cys Gly Ala
1               5               10              15

Leu Gly Leu Thr Met Ser Pro Ala Arg Gly Arg Leu Arg Cys Tyr Ile
            20              25              30
```

```
Cys Gly Phe Thr Lys Pro Cys His Pro Val Pro Thr Glu Cys Arg Asp
        35              40              45

Asp Glu Ala Cys Gly Ile Ser Ile Gly Thr Ser Val Lys Lys Leu Pro
        50              55              60

Leu Lys Gly Pro Val Pro Ser Ala Arg Leu Cys His Leu Leu Ala Ala
65              70              75              80

Leu Leu His Ser Val Ala Pro Leu Leu Arg Ala Gly Pro Val Gln His
                85              90              95

Ser Arg Phe Pro Thr Ala Ala His Gln Pro Pro Arg Leu Pro Ala Pro
            100             105             110

Leu Cys Gly Gln Leu Arg Trp Glu Arg Thr Pro Pro Pro Leu Ala Ser
            115             120             125

<210>   23
<211>   170
<212>   PRT
<213>   Homo sapiens

<400>   23

Thr Pro Leu Gly Pro Lys Trp Pro Glu Pro Val Phe Gly Arg Leu Ala
1               5               10              15

Ser Pro Gly Phe Pro Gly Glu Tyr Ala Asn Asp Gln Glu Arg Arg Trp
            20              25              30

Thr Leu Thr Ala Pro Pro Gly Tyr Arg Leu Arg Leu Tyr Phe Thr His
        35              40              45

Phe Asp Leu Glu Leu Ser His Leu Cys Glu Tyr Asp Phe Val Lys Leu
        50              55              60

Ser Ser Gly Ala Lys Val Leu Ala Thr Leu Cys Gly Gln Glu Ser Thr
65              70              75              80

Asp Thr Glu Arg Ala Pro Gly Lys Asp Thr Phe Tyr Ser Leu Gly Ser
                85              90              95

Ser Leu Asp Ile Thr Phe Arg Ser Asp Tyr Ser Asn Glu Lys Pro Phe
            100             105             110

Thr Gly Phe Glu Ala Phe Tyr Ala Ala Glu Asp Ile Asp Glu Cys Gln
            115             120             125

Val Ala Pro Gly Glu Ala Pro Thr Cys Asp His His Cys His Asn His
        130             135             140

Leu Gly Gly Phe Tyr Cys Ser Cys Arg Ala Gly Tyr Val Leu His Arg
145             150             155             160
```

EP 2 369 349 A2

Asn Lys Arg Thr Cys Ser Glu Gln Ser Leu
                    165                 170


<210> 24
<211> 297
<212> PRT
<213> Homo sapiens

<400> 24

Met Asp Ala Glu Gly Leu Ala Leu Leu Pro Pro Val Thr Leu Ala
1               5                   10                  15


Ala Leu Val Asp Ser Trp Leu Arg Glu Asp Cys Pro Gly Leu Asn Tyr
            20              25                  30


Ala Ala Leu Val Ser Gly Ala Gly Pro Ser Gln Ala Ala Leu Trp Ala
        35                  40                  45


Lys Ser Pro Gly Val Leu Ala Gly Gln Pro Phe Phe Asp Ala Ile Phe
    50                  55                  60


Thr Gln Leu Asn Cys Gln Val Ser Trp Phe Leu Pro Glu Gly Ser Lys
65                  70                  75                  80


Leu Val Pro Val Ala Arg Val Ala Glu Val Arg Gly Pro Ala His Cys
                85                  90                  95


Leu Leu Leu Gly Glu Arg Val Ala Leu Asn Thr Leu Ala Arg Cys Ser
            100                 105                 110


Gly Ile Ala Ser Ala Ala Ala Ala Ala Val Glu Ala Ala Arg Gly Ala
        115                 120                 125


Gly Trp Thr Gly His Val Ala Gly Thr Arg Lys Thr Thr Pro Gly Phe
    130                 135                 140


Arg Leu Val Glu Lys Tyr Gly Leu Leu Val Gly Gly Ala Ala Ser His
145                 150                 155                 160


Arg Tyr Asp Leu Gly Gly Leu Val Met Val Lys Asp Asn His Val Val
            165                 170                 175


Ala Ala Gly Gly Val Glu Lys Ala Val Arg Ala Ala Arg Gln Ala Ala
            180                 185                 190


Asp Phe Ala Leu Lys Val Glu Val Glu Cys Ser Ser Leu Gln Glu Ala
        195                 200                 205


Val Gln Ala Ala Glu Ala Gly Ala Asp Leu Val Leu Leu Asp Asn Phe
    210                 215                 220


62

```
Lys Pro Glu Glu Leu His Pro Thr Ala Thr Val Leu Lys Ala Gln Phe
225                 230             235                 240

Pro Ser Val Ala Val Glu Ala Ser Gly Gly Ile Thr Leu Asp Asn Leu
                245             250                 255

Pro Gln Phe Cys Gly Pro His Ile Asp Val Ile Ser Met Gly Met Leu
            260             265                 270

Thr Gln Ala Ala Pro Ala Leu Asp Phe Ser Leu Lys Leu Phe Ala Lys
            275             280                 285

Glu Val Ala Pro Val Pro Lys Ile His
        290             295
```

```
<210>  25
<211>  420
<212>  PRT
<213>  Homo sapiens

<400>  25
```

```
Met Ser Pro Pro Pro Leu Leu Gln Pro Leu Leu Leu Leu Leu Pro Leu
1               5               10              15

Leu Asn Val Glu Pro Ser Gly Ala Thr Leu Ile Arg Ile Pro Leu His
            20              25              30

Arg Val Gln Pro Gly Arg Arg Thr Leu Asn Leu Leu Arg Gly Trp Arg
            35              40              45

Glu Pro Ala Glu Leu Pro Lys Leu Gly Ala Pro Ser Pro Gly Asp Lys
        50              55              60

Pro Ile Phe Val Pro Leu Ser Asn Tyr Arg Asp Val Gln Tyr Phe Gly
65              70              75              80

Glu Ile Gly Leu Gly Thr Pro Pro Gln Asn Phe Thr Val Ala Phe Asp
            85              90              95

Thr Gly Ser Ser Asn Leu Trp Val Pro Ser Arg Arg Cys His Phe Phe
            100             105             110

Ser Val Pro Cys Trp Leu His His Arg Phe Asp Pro Lys Ala Ser Ser
        115             120             125

Ser Phe Gln Ala Asn Gly Thr Lys Phe Ala Ile Gln Tyr Gly Thr Gly
        130             135             140

Arg Val Asp Gly Ile Leu Ser Glu Asp Lys Leu Thr Ile Gly Gly Ile
145             150             155             160

Lys Gly Ala Ser Val Ile Phe Gly Glu Ala Leu Trp Glu Pro Ser Leu
            165             170             175
```

Val Phe Ala Phe Ala His Phe Asp Gly Ile Leu Gly Leu Gly Phe Pro
        180             185             190

Ile Leu Ser Val Glu Gly Val Arg Pro Pro Met Asp Val Leu Val Glu
        195         200             205

Gln Gly Leu Leu Asp Lys Pro Val Phe Ser Phe Tyr Leu Asn Arg Asp
    210         215             220

Pro Glu Glu Pro Asp Gly Gly Glu Leu Val Leu Gly Gly Ser Asp Pro
225             230             235             240

Ala His Tyr Ile Pro Pro Leu Thr Phe Val Pro Val Thr Val Pro Ala
            245             250             255

Tyr Trp Gln Ile His Met Glu Arg Val Lys Val Gly Pro Gly Leu Thr
            260             265             270

Leu Cys Ala Lys Gly Cys Ala Ala Ile Leu Asp Thr Gly Thr Ser Leu
        275             280             285

Ile Thr Gly Pro Thr Glu Glu Ile Arg Ala Leu His Ala Ala Ile Gly
    290             295             300

Gly Ile Pro Leu Leu Ala Gly Glu Tyr Ile Ile Leu Cys Ser Glu Ile
305             310             315             320

Pro Lys Leu Pro Ala Val Ser Phe Leu Leu Gly Gly Val Trp Phe Asn
            325             330             335

Leu Thr Ala His Asp Tyr Val Ile Gln Thr Thr Arg Asn Gly Val Arg
        340             345             350

Leu Cys Leu Ser Gly Phe Gln Ala Leu Asp Val Pro Pro Pro Ala Gly
        355             360             365

Pro Phe Trp Ile Leu Gly Asp Val Phe Leu Gly Thr Tyr Val Ala Val
    370             375             380

Phe Asp Arg Gly Asp Met Lys Ser Ser Ala Arg Val Gly Leu Ala Arg
385             390             395             400

Ala Arg Thr Arg Gly Ala Asp Leu Gly Trp Gly Glu Thr Ala Gln Ala
            405             410             415

Gln Phe Pro Gly
        420

<210>    26
<211>    373
<212>    PRT

<213> Homo sapiens

<400> 26

Met Ser Gly Glu Asp Glu Gln Gln Glu Gln Thr Ile Ala Glu Asp Leu
1               5                   10                  15

Val Val Thr Lys Tyr Lys Met Gly Gly Asp Ile Ala Asn Arg Val Leu
                20                  25                  30

Arg Ser Leu Val Glu Ala Ser Ser Ser Gly Val Ser Val Leu Ser Leu
            35                  40                  45

Cys Glu Lys Gly Asp Ala Met Ile Met Glu Glu Thr Gly Lys Ile Phe
        50                  55                  60

Lys Lys Glu Lys Glu Met Lys Lys Gly Ile Ala Phe Pro Thr Ser Ile
65                  70                  75                  80

Ser Val Asn Asn Cys Val Cys His Phe Ser Pro Leu Lys Ser Asp Gln
                85                  90                  95

Asp Tyr Ile Leu Lys Glu Gly Asp Leu Val Lys Ile Asp Leu Gly Val
            100                 105                 110

His Val Asp Gly Phe Ile Ala Asn Val Ala His Thr Phe Val Val Asp
        115                 120                 125

Val Ala Gln Gly Thr Gln Val Thr Gly Arg Lys Ala Asp Val Ile Lys
    130                 135                 140

Ala Ala His Leu Cys Ala Glu Ala Ala Leu Arg Leu Val Lys Pro Gly
145                 150                 155                 160

Asn Gln Asn Thr Gln Val Thr Glu Ala Trp Asn Lys Val Ala His Ser
                165                 170                 175

Phe Asn Cys Thr Pro Ile Glu Gly Met Leu Ser His Gln Leu Lys Gln
                180                 185                 190

His Val Ile Asp Gly Glu Lys Thr Ile Ile Gln Asn Pro Thr Asp Gln
        195                 200                 205

Gln Lys Lys Asp His Glu Lys Ala Glu Phe Glu Val His Glu Val Tyr
    210                 215                 220

Ala Val Asp Val Leu Val Ser Ser Gly Glu Gly Lys Ala Lys Asp Ala
225                 230                 235                 240

Gly Gln Arg Thr Thr Ile Tyr Lys Arg Asp Pro Ser Lys Gln Tyr Gly
                245                 250                 255

Leu Lys Met Lys Thr Ser Arg Ala Phe Phe Ser Glu Val Glu Arg Arg

260                    265                    270

Phe Asp Ala Met Pro Phe Thr Leu Arg Ala Phe Glu Asp Glu Lys Lys
        275                 280                 285

Ala Arg Met Gly Val Val Glu Cys Ala Lys His Glu Leu Leu Gln Pro
        290                 295                 300

Phe Asn Val Leu Tyr Glu Lys Glu Gly Glu Phe Val Ala Gln Phe Lys
305                 310                 315                 320

Phe Thr Val Leu Leu Met Pro Asn Gly Pro Met Arg Ile Thr Ser Gly
                325                 330                 335

Pro Phe Glu Pro Asp Leu Tyr Lys Ser Glu Met Glu Val Gln Asp Ala
                340                 345                 350

Glu Leu Lys Ala Leu Leu Gln Ser Ser Ala Ser Arg Lys Thr Gln Lys
        355                 360                 365

Lys Lys Lys Lys Lys
        370

<210>  27
<211>  189
<212>  PRT
<213>  Homo sapiens


<220>
<221>  MISC_FEATURE
<222>  (106)..(106)
<223>  X can be any naturally occurring amino acid

<400>  27

Met Ala Ser Lys Arg Ala Leu Val Ile Leu Ala Lys Gly Ala Glu Glu
1               5                   10                  15

Met Glu Thr Val Ile Pro Val Asp Val Met Arg Arg Ala Gly Ile Lys
            20                  25                  30

Val Thr Val Ala Gly Leu Ala Gly Lys Asp Pro Val Gln Cys Ser Arg
            35                  40                  45

Asp Val Val Ile Cys Pro Asp Ala Ser Leu Glu Asp Ala Lys Lys Glu
        50                  55                  60

Gly Pro Tyr Asp Val Val Val Leu Pro Gly Gly Asn Leu Gly Ala Gln
65                  70                  75                  80

Asn Leu Ser Glu Ser Ala Ala Val Lys Glu Ile Leu Lys Glu Gln Glu
                85                  90                  95

Asn Arg Lys Gly Leu Ile Ala Ala Ile Xaa Ala Gly Pro Thr Ala Leu

<pre>
                   100                     105                     110

      Leu Ala His Glu Ile Gly Phe Gly Ser Lys Val Thr Thr His Pro Leu
              115                 120                 125

      Ala Lys Asp Lys Met Met Asn Gly Gly His Tyr Thr Tyr Ser Glu Asn
              130                 135                 140

      Arg Val Glu Lys Asp Gly Leu Ile Leu Thr Ser Arg Gly Pro Gly Thr
      145                 150                 155                 160

      Ser Phe Glu Phe Ala Leu Ala Ile Val Glu Ala Leu Asn Gly Lys Glu
                      165                 170                 175

      Val Ala Ala Gln Val Lys Ala Pro Leu Val Leu Lys Asp
                  180                 185
</pre>

<210> 28
<211> 356
<212> PRT
<213> Homo sapiens

<400> 28

<pre>
      Met Asp Ala Gly Val Thr Glu Ser Gly Leu Asn Val Thr Leu Thr Ile
      1               5                   10                  15

      Arg Leu Leu Met His Gly Lys Glu Val Gly Ser Ile Ile Gly Lys Lys
                  20                  25                  30

      Gly Glu Ser Val Lys Arg Ile Arg Glu Glu Ser Gly Ala Arg Ile Asn
                  35                  40                  45

      Ile Ser Glu Gly Asn Cys Pro Glu Arg Ile Ile Thr Leu Thr Gly Pro
              50                  55                  60

      Thr Asn Ala Ile Phe Lys Ala Phe Ala Met Ile Ile Asp Lys Leu Glu
      65                  70                  75                  80

      Glu Asp Ile Asn Ser Ser Met Thr Asn Ser Thr Ala Ala Ser Arg Pro
                      85                  90                  95

      Pro Val Thr Leu Arg Leu Val Val Pro Ala Thr Gln Cys Gly Ser Leu
                  100                 105                 110

      Ile Gly Lys Gly Gly Cys Lys Ile Lys Glu Ile Arg Glu Ser Thr Gly
                  115                 120                 125

      Ala Gln Val Gln Val Ala Gly Asp Met Leu Pro Asn Ser Thr Glu Arg
              130                 135                 140

      Ala Ile Thr Ile Ala Gly Val Pro Gln Ser Val Thr Glu Cys Val Lys
      145                 150                 155                 160
</pre>

```
Gln Ile Cys Leu Val Met Leu Glu Thr Leu Ser Gln Ser Pro Gln Gly
            165             170             175

Arg Val Met Thr Ile Pro Tyr Gln Pro Met Pro Ala Ser Ser Pro Val
            180             185             190

Ile Cys Ala Gly Gly Gln Asp Arg Cys Ser Asp Ala Val Gly Tyr Pro
        195             200             205

His Ala Thr His Asp Leu Glu Gly Pro Pro Leu Asp Ala Tyr Ser Ile
    210             215             220

Gln Gly Gln His Thr Ile Ser Pro Leu Asp Leu Ala Lys Leu Asn Gln
225             230             235             240

Val Ala Arg Gln Gln Ser His Phe Ala Met Met His Gly Gly Thr Gly
            245             250             255

Phe Ala Gly Ile Asp Ser Ser Ser Pro Glu Val Lys Gly Tyr Trp Ala
            260             265             270

Ser Leu Asp Ala Ser Thr Gln Thr Thr His Glu Leu Thr Ile Pro Asn
        275             280             285

Asn Leu Ile Gly Cys Ile Ile Gly Arg Gln Gly Ala Asn Ile Asn Glu
    290             295             300

Ile Arg Gln Met Ser Gly Ala Gln Ile Lys Ile Ala Asn Pro Val Glu
305             310             315             320

Gly Ser Ser Gly Arg Gln Val Thr Ile Thr Gly Ser Ala Ala Ser Ile
            325             330             335

Ser Leu Ala Gln Tyr Leu Ile Asn Ala Arg Leu Ser Ser Glu Lys Gly
            340             345             350

Met Gly Cys Ser
            355


<210>   29
<211>   873
<212>   PRT
<213>   Homo sapiens

<400>   29

Met Ala Thr Phe Ile Ser Met Gln Leu Lys Lys Thr Ser Glu Val Asp
1               5               10              15

Leu Ala Lys Pro Leu Val Lys Phe Ile Gln Gln Thr Tyr Pro Ser Gly
            20              25              30

Gly Glu Glu Gln Ala Gln Tyr Cys Arg Ala Ala Glu Glu Leu Ser Lys
```

                    35                              40                              45

Leu Arg Arg Ala Ala Val Gly Arg Pro Leu Asp Lys His Glu Gly Ala
    50              55                  60

Leu Glu Thr Leu Leu Arg Tyr Tyr Asp Gln Ile Cys Ser Ile Glu Pro
65              70                  75                  80

Lys Phe Pro Phe Ser Glu Asn Gln Ile Cys Leu Thr Phe Thr Trp Lys
                85                  90                  95

Asp Ala Phe Asp Lys Gly Ser Leu Phe Gly Gly Ser Val Lys Leu Ala
            100             105                 110

Leu Ala Ser Leu Gly Tyr Glu Lys Ser Cys Val Leu Phe Asn Cys Ala
        115             120                 125

Ala Leu Ala Ser Gln Ile Ala Ala Glu Gln Asn Leu Asp Asn Asp Glu
    130             135                 140

Gly Leu Lys Ile Ala Ala Lys His Tyr Gln Phe Ala Ser Gly Ala Phe
145             150                 155                 160

Leu His Ile Lys Glu Thr Val Leu Ser Ala Leu Ser Arg Glu Pro Thr
            165                 170                 175

Val Asp Ile Ser Pro Asp Thr Val Gly Thr Leu Ser Leu Ile Met Leu
            180                 185                 190

Ala Gln Ala Gln Glu Val Phe Phe Leu Lys Ala Thr Arg Asp Lys Met
        195                 200                 205

Lys Asp Ala Ile Ile Ala Lys Leu Ala Asn Gln Ala Ala Asp Tyr Phe
    210                 215                 220

Gly Asp Ala Phe Lys Gln Cys Gln Tyr Lys Asp Thr Leu Pro Lys Tyr
225                 230                 235                 240

Phe Tyr Phe Gln Glu Val Phe Pro Val Leu Ala Ala Lys His Cys Ile
            245                 250                 255

Met Gln Ala Asn Ala Glu Tyr His Gln Ser Ile Leu Ala Lys Gln Gln
        260                 265                 270

Lys Lys Phe Gly Glu Glu Ile Ala Arg Leu Gln His Ala Ala Glu Leu
        275                 280                 285

Ile Lys Thr Val Ala Ser Arg Tyr Asp Glu Tyr Val Asn Val Lys Asp
    290                 295                 300

Phe Ser Asp Lys Ile Asn Arg Ala Leu Ala Ala Ala Lys Lys Asp Asn
305                 310                 315                 320

```
Asp Phe Ile Tyr His Asp Arg Val Pro Asp Leu Lys Asp Leu Asp Pro
                325             330             335

Ile Gly Lys Ala Thr Leu Val Lys Ser Thr Pro Val Asn Val Pro Ile
            340             345             350

Ser Gln Lys Phe Thr Asp Leu Phe Glu Lys Met Val Pro Val Ser Val
        355             360             365

Gln Gln Ser Leu Ala Ala Tyr Asn Gln Arg Lys Ala Asp Leu Ile Asn
    370             375             380

Arg Ser Ile Ala Gln Met Arg Glu Ala Thr Thr Leu Ala Asn Gly Val
385             390             395             400

Leu Ala Ser Leu Asn Leu Pro Ala Ala Ile Glu Asp Val Ser Gly Asp
            405             410             415

Thr Val Pro Gln Ser Ile Leu Thr Lys Ser Arg Ser Val Ile Glu Gln
            420             425             430

Gly Gly Ile Gln Thr Val Asp Gln Leu Ile Lys Glu Leu Pro Glu Leu
            435             440             445

Leu Gln Arg Asn Arg Glu Ile Leu Asp Glu Ser Leu Arg Leu Leu Asp
    450             455             460

Glu Glu Glu Ala Thr Asp Asn Asp Leu Arg Ala Lys Phe Lys Glu Arg
465             470             475             480

Trp Gln Arg Thr Pro Ser Asn Glu Leu Tyr Lys Pro Leu Arg Ala Glu
            485             490             495

Gly Thr Asn Phe Arg Thr Val Leu Asp Lys Ala Val Gln Ala Asp Gly
            500             505             510

Gln Val Lys Glu Cys Tyr Gln Ser His Arg Asp Thr Ile Val Leu Leu
        515             520             525

Cys Lys Pro Glu Pro Glu Leu Asn Ala Ala Ile Pro Ser Ala Asn Pro
    530             535             540

Ala Lys Thr Met Gln Gly Ser Glu Val Val Asn Val Leu Lys Ser Leu
545             550             555             560

Leu Ser Asn Leu Asp Glu Val Lys Lys Glu Arg Glu Gly Leu Glu Asn
            565             570             575

Asp Leu Lys Ser Val Asn Phe Asp Met Thr Ser Lys Phe Leu Thr Ala
            580             585             590
```

Leu Ala Gln Asp Gly Val Ile Asn Glu Glu Ala Leu Ser Val Thr Glu
        595                 600                 605

Leu Asp Arg Val Tyr Gly Gly Leu Thr Thr Lys Val Gln Glu Ser Leu
        610             615                 620

Lys Lys Gln Glu Gly Leu Leu Lys Asn Ile Gln Val Ser His Gln Glu
625                 630                 635                 640

Phe Ser Lys Met Lys Gln Ser Asn Asn Glu Ala Asn Leu Arg Glu Glu
                645                 650                 655

Val Leu Lys Asn Leu Ala Thr Ala Tyr Asp Asn Phe Val Glu Leu Val
            660                 665                 670

Ala Asn Leu Lys Glu Gly Thr Lys Phe Tyr Asn Glu Leu Thr Glu Ile
            675                 680                 685

Leu Val Arg Phe Gln Asn Lys Cys Ser Asp Ile Val Phe Ala Arg Lys
        690                 695                 700

Thr Glu Arg Asp Glu Leu Leu Lys Asp Leu Gln Gln Ser Ile Ala Arg
705                 710                 715                 720

Glu Pro Ser Ala Pro Ser Ile Pro Thr Pro Ala Tyr Gln Ser Ser Pro
                725                 730                 735

Ala Gly Gly His Ala Pro Thr Pro Pro Thr Pro Ala Pro Arg Thr Met
            740                 745                 750

Pro Pro Thr Lys Pro Gln Pro Pro Ala Arg Pro Pro Pro Pro Val Leu
            755                 760                 765

Pro Ala Asn Arg Ala Pro Ser Ala Thr Ala Pro Ser Pro Val Gly Ala
        770                 775                 780

Gly Thr Ala Ala Pro Ala Pro Ser Gln Thr Pro Gly Ser Ala Pro Pro
785                 790                 795                 800

Pro Gln Ala Gln Gly Pro Pro Tyr Pro Thr Tyr Pro Gly Tyr Pro Gly
                805                 810                 815

Tyr Cys Gln Met Pro Met Pro Met Gly Tyr Asn Pro Tyr Ala Tyr Gly
            820                 825                 830

Gln Tyr Asn Met Pro Tyr Pro Pro Val Tyr His Gln Ser Pro Gly Gln
        835                 840                 845

Ala Pro Tyr Pro Gly Pro Gln Gln Pro Ser Tyr Pro Phe Pro Gln Pro
    850                 855                 860

Pro Gln Gln Ser Tyr Tyr Pro Gln Gln
865                 870

<210>   30
<211>   423
<212>   PRT
<213>   Homo sapiens

<400>   30

Met Ala Gly Lys Arg Ser Gly Trp Ser Arg Ala Ala Leu Leu Gln Leu
1               5                   10                  15

Leu Leu Gly Val Asn Leu Val Val Met Pro Pro Thr Gln Ala Arg Ser
                20                  25                  30

Leu Arg Phe Val Thr Leu Leu Tyr Arg His Gly Asp Arg Ser Pro Val
        35                  40                  45

Lys Thr Tyr Pro Lys Asp Pro Tyr Gln Glu Glu Glu Trp Pro Gln Gly
    50                  55                  60

Phe Gly Gln Leu Thr Lys Glu Gly Met Leu Gln His Trp Glu Leu Gly
65                  70                  75                  80

Gln Ala Leu Arg Gln Arg Tyr His Gly Phe Leu Asn Thr Ser Tyr His
                85                  90                  95

Arg Gln Glu Val Tyr Val Arg Ser Thr Asp Phe Asp Arg Thr Leu Met
                100                 105                 110

Ser Ala Glu Ala Asn Leu Ala Gly Leu Phe Pro Pro Asn Gly Met Gln
            115                 120                 125

Arg Phe Asn Pro Asn Ile Ser Trp Gln Pro Ile Pro Val His Thr Val
        130                 135                 140

Pro Ile Thr Glu Asp Arg Leu Leu Lys Phe Pro Leu Gly Pro Cys Pro
145                 150                 155                 160

Arg Tyr Glu Gln Leu Gln Asn Glu Thr Arg Gln Thr Pro Glu Tyr Gln
                165                 170                 175

Asn Glu Ser Ser Arg Asn Ala Gln Phe Leu Asp Met Val Ala Asn Glu
                180                 185                 190

Thr Gly Leu Thr Asp Leu Thr Leu Glu Thr Val Trp Asn Val Tyr Asp
            195                 200                 205

Thr Leu Phe Cys Glu Gln Thr His Gly Leu Arg Leu Pro Pro Trp Ala
        210                 215                 220

Ser Pro Gln Thr Met Gln Arg Leu Ser Arg Leu Lys Asp Phe Ser Phe
225                 230                 235                 240

Arg Phe Leu Phe Gly Ile Tyr Gln Gln Ala Glu Lys Ala Arg Leu Gln
245              250              255

Gly Gly Val Leu Leu Ala Gln Ile Arg Lys Asn Leu Thr Leu Met Ala
260              265              270

Thr Thr Ser Gln Leu Pro Lys Leu Leu Val Tyr Ser Ala His Asp Thr
275              280              285

Thr Leu Val Ala Leu Gln Met Ala Leu Asp Val Tyr Asn Gly Glu Gln
290              295              300

Ala Pro Tyr Ala Ser Cys His Ile Phe Glu Leu Tyr Gln Glu Asp Ser
305              310              315              320

Gly Asn Phe Ser Val Glu Met Tyr Phe Arg Asn Glu Ser Asp Lys Ala
325              330              335

Pro Trp Pro Leu Ser Leu Pro Gly Cys Pro His Arg Cys Pro Leu Gln
340              345              350

Asp Phe Leu Arg Leu Thr Glu Pro Val Val Pro Lys Asp Trp Gln Gln
355              360              365

Glu Cys Gln Leu Ala Ser Gly Pro Ala Asp Thr Glu Val Ile Val Ala
370              375              380

Leu Ala Val Cys Gly Ser Ile Leu Phe Leu Leu Ile Val Leu Leu Leu
385              390              395              400

Thr Val Leu Phe Arg Met Gln Ala Gln Pro Pro Gly Tyr Arg His Val
405              410              415

Ala Asp Gly Glu Asp His Ala
420

<210> 31
<211> 164
<212> PRT
<213> Homo sapiens

<400> 31

Gly Lys Tyr Val Val Leu Phe Phe Tyr Pro Leu Asp Phe Thr Phe Val
1              5              10              15

Cys Pro Thr Glu Ile Ile Ala Phe Ser Asn Arg Ala Glu Asp Phe Arg
20              25              30

Lys Leu Gly Cys Glu Val Leu Gly Val Ser Val Asp Ser Gln Phe Thr
35              40              45

His Leu Ala Trp Ile Asn Thr Pro Arg Lys Glu Gly Gly Leu Gly Pro
    50                  55                  60

Leu Asn Ile Pro Leu Leu Ala Asp Val Thr Arg Arg Leu Ser Glu Asp
65                  70                  75                  80

Tyr Gly Val Leu Lys Thr Asp Glu Gly Ile Ala Tyr Arg Gly Leu Phe
              85                  90                  95

Ile Ile Asp Gly Lys Gly Val Leu Arg Gln Ile Thr Val Asn Asp Leu
             100                 105                 110

Pro Val Gly Arg Ser Val Asp Glu Ala Leu Arg Leu Val Gln Ala Phe
             115                 120                 125

Gln Tyr Thr Asp Glu His Gly Glu Val Cys Pro Ala Gly Trp Lys Pro
        130                 135                 140

Gly Ser Asp Thr Ile Lys Pro Asn Val Asp Asp Ser Lys Glu Tyr Phe
145                 150                 155                 160

Ser Lys His Asn


<210>  32
<211>  310
<212>  PRT
<213>  Homo sapiens

<400>  32

Arg Ser Gly Leu Trp Val Phe Cys Tyr Arg Asp Arg Leu Val Met Gln
1               5                   10                  15

Lys Gly Leu Lys Asp Asn Phe Ala Asp Val Gln Val Ser Val Val Asp
             20                  25                  30

Cys Pro Asp Leu Thr Lys Glu Pro Phe Thr Phe Pro Val Lys Gly Ile
        35                  40                  45

Cys Gly Lys Thr Arg Ile Ala Glu Val Gly Gly Val Pro Tyr Leu Leu
        50                  55                  60

Pro Leu Val Asn Gln Lys Lys Val Tyr Asp Leu Asn Lys Ile Ala Lys
65                  70                  75                  80

Glu Ile Lys Leu Pro Gly Ala Phe Ile Leu Gly Ala Gly Ala Gly Pro
              85                  90                  95

Phe Gln Thr Leu Gly Phe Asn Ser Glu Phe Met Pro Val Ile Gln Thr
             100                 105                 110

Glu Ser Glu His Lys Pro Pro Val Asn Gly Ser Tyr Phe Ala His Val
        115                 120                 125

Asn Pro Ala Asp Gly Gly Cys Leu Leu Glu Lys Tyr Ser Glu Lys Cys
        130                 135                 140

His Asp Phe Gln Cys Ala Leu Leu Ala Asn Leu Phe Ala Ser Glu Gly
145                 150                 155                 160

Gln Pro Gly Lys Val Ile Glu Val Lys Ala Lys Arg Arg Thr Gly Pro
                165                 170                 175

Leu Asn Phe Val Thr Cys Met Arg Glu Thr Leu Glu Lys His Tyr Gly
            180                 185                 190

Asn Lys Pro Ile Gly Met Gly Gly Thr Phe Ile Ile Gln Lys Gly Lys
        195                 200                 205

Val Lys Ser His Ile Met Pro Ala Glu Phe Ser Ser Cys Pro Leu Asn
    210                 215                 220

Ser Asp Glu Glu Val Asn Lys Trp Leu His Phe Tyr Glu Met Lys Ala
225                 230                 235                 240

Pro Leu Val Cys Leu Pro Val Phe Val Ser Arg Asp Pro Gly Phe Asp
                245                 250                 255

Leu Arg Leu Glu His Thr His Phe Phe Ser Arg His Gly Glu Gly Gly
            260                 265                 270

His Tyr His Tyr Asp Thr Thr Pro Asp Ile Val Glu Tyr Leu Gly Tyr
        275                 280                 285

Phe Leu Pro Ala Glu Phe Leu Tyr Arg Ile Asp Gln Pro Lys Glu Thr
        290                 295                 300

His Ser Ile Gly Arg Asp
305                 310

<210>   33
<211>   251
<212>   PRT
<213>   Homo sapiens

<400>   33

Gln Asn Asp Leu Arg Pro Leu Leu Ile Glu Arg Tyr Pro Gly Ser Pro
1               5                   10                  15

Gly Ser Tyr Ala Ala Arg Gln His Ile Met Gln Arg Ile Gln Arg Leu
            20                  25                  30

Gln Ala Asp Trp Val Leu Glu Ile Asp Thr Phe Leu Ser Gln Thr Pro
        35                  40                  45

```
Tyr Gly Tyr Arg Ser Phe Ser Asn Ile Ile Ser Thr Leu Asn Pro Thr
    50              55              60

Ala Lys Arg His Leu Val Leu Ala Cys His Tyr Asp Ser Lys Tyr Phe
65              70              75              80

Ser His Trp Asn Asn Arg Val Phe Val Gly Ala Thr Asp Ser Ala Val
            85              90              95

Pro Cys Ala Met Met Leu Glu Leu Ala Arg Ala Leu Asp Lys Lys Leu
            100             105             110

Leu Ser Leu Lys Thr Val Ser Asp Ser Lys Pro Asp Leu Ser Leu Gln
        115             120             125

Leu Ile Phe Phe Asp Gly Glu Glu Ala Phe Leu His Trp Ser Pro Gln
    130             135             140

Asp Ser Leu Tyr Gly Ser Arg His Leu Ala Ala Lys Met Ala Ser Thr
145             150             155             160

Pro His Pro Pro Gly Ala Arg Gly Thr Ser Gln Leu His Gly Met Asp
            165             170             175

Leu Leu Val Leu Leu Asp Leu Ile Gly Ala Pro Asn Pro Thr Phe Pro
        180             185             190

Asn Phe Phe Pro Asn Ser Ala Arg Trp Phe Glu Arg Leu Gln Ala Ile
        195             200             205

Glu His Glu Leu His Glu Leu Gly Leu Leu Lys Asp His Ser Leu Glu
    210             215             220

Gly Arg Tyr Phe Gln Asn Tyr Ser Tyr Gly Gly Val Ile Gln Asp Asp
225             230             235             240

His Ile Pro Phe Leu Arg Arg Gly Val Pro Val
            245             250
```

```
<210>   34
<211>   472
<212>   PRT
<213>   Homo sapiens

<400>   34
```

```
Met Ala Thr Lys Cys Gly Asn Cys Gly Pro Gly Tyr Ser Thr Pro Leu
1               5               10              15

Glu Ala Met Lys Gly Pro Arg Glu Glu Ile Val Tyr Leu Pro Cys Ile
            20              25              30

Tyr Arg Asn Thr Gly Thr Glu Ala Pro Asp Tyr Leu Ala Thr Val Asp
        35              40              45
```

Val Asp Pro Lys Ser Pro Gln Tyr Cys Gln Val Ile His Arg Leu Pro
    50              55              60

Met Pro Asn Leu Lys Asp Glu Leu His His Ser Gly Trp Asn Thr Tyr
65              70              75              80

Ser Ser Cys Phe Gly Asp Ser Thr Lys Ser Arg Asn Lys Leu Val Leu
                85              90              95

Pro Ser Leu Ile Ser Ser Arg Ile Tyr Val Val Asp Val Gly Ser Glu
            100             105             110

Pro Gly Pro Gln Lys Leu His Lys Val Ile Glu Pro Lys Asp Ile His
        115             120             125

Ala Lys Cys Glu Leu Ala Cys Leu His Thr Ser His Cys Leu Ala Ser
    130             135             140

Gly Glu Val Met Ile Ser Ser Leu Gly Asp Val Lys Gly Asn Gly Lys
145             150             155             160

Gly Gly Phe Val Leu Leu Asp Gly Glu Thr Phe Glu Val Lys Gly Thr
            165             170             175

Trp Glu Arg Pro Gly Gly Ala Ala Pro Leu Gly Tyr Asp Phe Trp Tyr
        180             185             190

Gln Pro Arg His Asn Val Met Ile Ser Thr Glu Trp Ala Ala Pro Asn
        195             200             205

Val Leu Arg Asp Gly Phe Asn Pro Ala Asp Val Glu Ala Gly Leu Tyr
    210             215             220

Gly Ser His Leu Tyr Val Trp Asp Trp Gln Arg His Glu Ile Val Gln
225             230             235             240

Thr Leu Ser Leu Lys Asp Gly Leu Ile Pro Leu Glu Ile Arg Phe Leu
            245             250             255

His Asn Pro Ser Ala Thr Gln Gly Phe Val Gly Cys Ala Ser Ala Pro
        260             265             270

Asn Ile Gln Arg Phe Tyr Lys Thr Arg Glu Gly Thr Trp Ser Val Glu
        275             280             285

Lys Val Ile Gln Val Pro Pro Lys Lys Val Lys Gly Trp Leu Leu Pro
    290             295             300

Gly Val Pro Gly Leu Ile Thr Asp Ile Leu Leu Ser Leu Asp Asp Arg
305             310             315             320

```
Phe Leu Tyr Phe Ser Asn Trp Leu His Gly Asp Leu Arg Gln Tyr Asp
                325                 330                 335

Ile Ser Asp Pro Gln Arg Pro Arg Leu Thr Gly Gln Leu Phe Leu Gly
            340                 345                 350

Gly Ser Ile Val Lys Gly Gly Pro Val Gln Val Leu Glu Asp Glu Glu
        355                 360                 365

Leu Lys Ser Gln Pro Glu Pro Leu Val Val Lys Gly Lys Arg Val Ala
    370                 375                 380

Gly Gly Pro Gln Met Ile Gln Leu Ser Leu Asp Gly Lys Arg Leu Tyr
385                 390                 395                 400

Ile Thr Thr Ser Leu Tyr Ser Ala Trp Glu Lys Gln Phe Tyr Pro Asp
                405                 410                 415

Leu Ile Arg Glu Gly Ser Val Met Leu Gln Val Asp Val Asp Thr Val
            420                 425                 430

Lys Gly Gly Leu Lys Leu Asn Pro Asn Cys Leu Val Asp Phe Gly Lys
        435                 440                 445

Glu Pro Leu Gly Pro Ala Leu Ala His Glu Leu Arg Tyr Pro Gly Gly
    450                 455                 460

Asp Cys Ser Ser Asp Ile Trp Ile
465                 470
```

```
<210>  35
<211>  543
<212>  PRT
<213>  Homo sapiens

<400>  35
```

```
Met Glu Gln Val Asn Glu Leu Lys Glu Lys Gly Asn Lys Ala Leu Ser
1               5                   10                  15

Val Gly Asn Ile Asp Asp Ala Leu Gln Cys Tyr Ser Glu Ala Ile Lys
            20                  25                  30

Leu Asp Pro His Asn His Val Leu Tyr Ser Asn Arg Ser Ala Ala Tyr
        35                  40                  45

Ala Lys Lys Gly Asp Tyr Gln Lys Ala Tyr Glu Asp Gly Cys Lys Thr
    50                  55                  60

Val Asp Leu Lys Pro Asp Trp Gly Lys Gly Tyr Ser Arg Lys Ala Ala
65                  70                  75                  80

Ala Leu Glu Phe Leu Asn Arg Phe Glu Glu Ala Lys Arg Thr Tyr Glu
```

|  | 85 |  |  |  |  | 90 |  |  |  |  | 95 |  |  |
|---|---|---|---|---|---|---|---|---|---|---|---|---|---|

Glu Gly Leu Lys His Glu Ala Asn Asn Pro Gln Leu Lys Glu Gly Leu
100 105 110

Gln Asn Met Glu Ala Arg Leu Ala Glu Arg Lys Phe Met Asn Pro Phe
115 120 125

Asn Met Pro Asn Leu Tyr Gln Lys Leu Glu Ser Asp Pro Arg Thr Arg
130 135 140

Thr Leu Leu Ser Asp Pro Thr Tyr Arg Glu Leu Ile Glu Gln Leu Arg
145 150 155 160

Asn Lys Pro Ser Asp Leu Gly Thr Lys Leu Gln Asp Pro Arg Ile Met
165 170 175

Thr Thr Leu Ser Val Leu Leu Gly Val Asp Leu Gly Ser Met Asp Glu
180 185 190

Glu Glu Glu Ile Ala Thr Pro Pro Pro Pro Pro Pro Lys Lys Glu
195 200 205

Thr Lys Pro Glu Pro Met Glu Glu Asp Leu Pro Glu Asn Lys Lys Gln
210 215 220

Ala Leu Lys Glu Lys Glu Leu Gly Asn Asp Ala Tyr Lys Lys Lys Asp
225 230 235 240

Phe Asp Thr Ala Leu Lys His Tyr Asp Lys Ala Lys Glu Leu Asp Pro
245 250 255

Thr Asn Met Thr Tyr Ile Thr Asn Gln Ala Ala Val Tyr Phe Glu Lys
260 265 270

Gly Asp Tyr Asn Lys Cys Arg Glu Leu Cys Glu Lys Ala Ile Glu Val
275 280 285

Gly Arg Glu Asn Arg Glu Asp Tyr Arg Gln Ile Ala Lys Ala Tyr Ala
290 295 300

Arg Ile Gly Asn Ser Tyr Phe Lys Glu Glu Lys Tyr Lys Asp Ala Ile
305 310 315 320

His Phe Tyr Asn Lys Ser Leu Ala Glu His Arg Thr Pro Asp Val Leu
325 330 335

Lys Lys Cys Gln Gln Ala Glu Lys Ile Leu Lys Glu Gln Glu Arg Leu
340 345 350

Ala Tyr Ile Asn Pro Asp Leu Ala Leu Glu Glu Lys Asn Lys Gly Asn
355 360 365

```
Glu Cys Phe Gln Lys Gly Asp Tyr Pro Gln Ala Met Lys His Tyr Thr
    370             375             380

Glu Ala Ile Lys Arg Asn Pro Lys Asp Ala Lys Leu Tyr Ser Asn Arg
385             390             395             400

Ala Ala Cys Tyr Thr Lys Leu Leu Glu Phe Gln Leu Ala Leu Lys Asp
            405             410             415

Cys Glu Glu Cys Ile Gln Leu Glu Pro Thr Phe Ile Lys Gly Tyr Thr
            420             425             430

Arg Lys Ala Ala Ala Leu Glu Ala Met Lys Asp Tyr Thr Lys Ala Met
        435             440             445

Asp Val Tyr Gln Lys Ala Leu Asp Leu Asp Ser Ser Cys Lys Glu Ala
    450             455             460

Ala Asp Gly Tyr Gln Arg Cys Met Met Ala Gln Tyr Asn Arg His Asp
465             470             475             480

Ser Pro Glu Asp Val Lys Arg Arg Ala Met Ala Asp Pro Glu Val Gln
            485             490             495

Gln Ile Met Ser Asp Pro Ala Met Arg Leu Ile Leu Glu Gln Met Gln
            500             505             510

Lys Asp Pro Gln Ala Leu Ser Glu His Leu Lys Asn Pro Val Ile Ala
        515             520             525

Gln Lys Ile Gln Lys Leu Met Asp Val Gly Leu Ile Ala Ile Arg
    530             535             540
```

```
<210>    36
<211>    318
<212>    PRT
<213>    Homo sapiens

<400>    36
```

```
Met Ser Ser Ser Pro Val Lys Arg Gln Arg Met Glu Ser Ala Leu Asp
1               5               10              15

Gln Leu Lys Gln Phe Thr Thr Val Val Ala Asp Thr Gly Asp Phe His
            20              25              30

Ala Ile Asp Glu Tyr Lys Pro Gln Asp Ala Thr Thr Asn Pro Ser Leu
        35              40              45

Ile Leu Ala Ala Ala Gln Met Pro Ala Tyr Gln Glu Leu Val Glu Glu
    50              55              60
```

Ala Ile Ala Tyr Gly Arg Lys Leu Gly Gly Ser Gln Glu Asp Gln Ile
65              70              75              80

Lys Asn Ala Ile Asp Lys Leu Phe Val Leu Phe Gly Ala Glu Ile Leu
                85              90              95

Lys Lys Ile Pro Gly Arg Val Ser Thr Glu Val Asp Ala Arg Leu Ser
            100             105             110

Phe Asp Lys Asp Ala Met Val Ala Arg Ala Arg Arg Leu Ile Glu Leu
        115             120             125

Tyr Lys Glu Ala Gly Ile Ser Lys Asp Arg Ile Leu Ile Lys Leu Ser
    130             135             140

Ser Thr Trp Glu Gly Ile Gln Ala Gly Lys Glu Leu Glu Glu Gln His
145             150             155             160

Gly Ile His Cys Asn Met Thr Leu Leu Phe Ser Phe Ala Gln Ala Val
            165             170             175

Ala Cys Ala Glu Ala Gly Val Thr Leu Ile Ser Pro Phe Val Gly Arg
            180             185             190

Ile Leu Asp Trp His Val Ala Asn Thr Asp Lys Lys Ser Tyr Glu Pro
        195             200             205

Leu Glu Asp Pro Gly Val Lys Ser Val Thr Lys Ile Tyr Asn Tyr Tyr
    210             215             220

Lys Lys Phe Ser Tyr Lys Thr Ile Val Met Gly Ala Ser Phe Arg Asn
225             230             235             240

Thr Gly Glu Ile Lys Ala Leu Ala Gly Cys Asp Phe Leu Thr Ile Ser
            245             250             255

Pro Lys Leu Leu Gly Glu Leu Leu Gln Asp Asn Ala Lys Leu Val Pro
        260             265             270

Val Leu Ser Ala Lys Pro Lys Pro Val Thr Trp Lys Lys Ser Thr Trp
        275             280             285

Met Arg Ser Leu Ser Val Gly Cys Thr Thr Arg Thr Arg Trp Leu Trp
    290             295             300

Arg Ser Ser Leu Thr Gly Ser Ala Ser Leu Pro Leu Met Gln
305             310             315

<210>   37
<211>   534
<212>   PRT
<213>   Homo sapiens

81

<400> 37

Met Pro Tyr Glu Ile Lys Lys Val Phe Ala Ser Leu Pro Gln Val Glu
1               5                   10                  15

Arg Gly Val Ser Lys Ile Ile Gly Gly Asp Pro Lys Gly Asn Asn Phe
                20              25                  30

Leu Tyr Thr Asn Gly Lys Cys Val Ile Leu Arg Asn Ile Asp Asn Pro
        35              40                  45

Ala Leu Ala Asp Ile Tyr Thr Glu His Ala His Gln Val Val Val Ala
    50                  55                  60

Lys Tyr Ala Pro Ser Gly Phe Tyr Ile Ala Ser Gly Asp Val Ser Gly
65              70                  75                  80

Lys Leu Arg Ile Trp Asp Thr Thr Gln Lys Glu His Leu Leu Lys Tyr
                85                  90                  95

Glu Tyr Gln Pro Phe Ala Gly Lys Ile Lys Asp Ile Ala Trp Thr Glu
            100                 105                 110

Asp Ser Lys Arg Ile Ala Val Val Gly Glu Gly Arg Glu Lys Phe Gly
        115                 120                 125

Ala Val Phe Leu Trp Asp Ser Gly Ser Ser Val Gly Glu Ile Thr Gly
    130                 135                 140

His Asn Lys Val Ile Asn Ser Val Asp Ile Lys Gln Ser Arg Pro Tyr
145                 150                 155                 160

Arg Leu Ala Thr Gly Ser Asp Asp Asn Cys Ala Ala Phe Phe Glu Gly
                165                 170                 175

Pro Pro Phe Lys Phe Lys Phe Thr Ile Gly Asp His Ser Arg Phe Val
            180                 185                 190

Asn Cys Val Arg Phe Ser Pro Asp Gly Asn Arg Phe Ala Thr Ala Ser
        195                 200                 205

Ala Asp Gly Gln Ile Tyr Ile Tyr Asp Gly Lys Thr Gly Glu Lys Val
    210                 215                 220

Cys Ala Leu Gly Gly Ser Lys Ala His Asp Gly Gly Ile Tyr Ala Ile
225                 230                 235                 240

Ser Trp Ser Pro Asp Ser Thr His Leu Leu Ser Ala Ser Gly Asp Lys
                245                 250                 255

Thr Ser Lys Ile Trp Asp Val Ser Val Asn Ser Val Val Ser Thr Phe
                260                 265                 270

```
Pro Met Gly Ser Thr Val Leu Asp Gln Gln Leu Gly Cys Leu Trp Gln
        275             280             285

Lys Asp His Leu Leu Ser Val Ser Leu Ser Gly Tyr Ile Asn Tyr Leu
    290             295             300

Asp Arg Asn Asn Pro Ser Lys Pro Leu His Val Ile Lys Gly His Ser
305             310             315             320

Lys Ser Ile Gln Cys Leu Thr Val His Lys Asn Gly Gly Lys Ser Tyr
            325             330             335

Ile Tyr Ser Gly Ser His Asp Gly His Ile Asn Tyr Trp Asp Ser Glu
            340             345             350

Thr Gly Glu Asn Asp Ser Phe Ala Gly Lys Gly His Thr Asn Gln Val
        355             360             365

Ser Arg Met Thr Val Asp Glu Ser Gly Gln Leu Ile Ser Cys Ser Met
    370             375             380

Asp Asp Thr Val Arg Tyr Thr Ser Leu Met Leu Arg Asp Tyr Ser Gly
385             390             395             400

Gln Gly Val Val Lys Leu Asp Val Gln Pro Lys Cys Val Ala Val Gly
            405             410             415

Pro Gly Gly Tyr Ala Val Val Val Cys Ile Gly Gln Ile Val Leu Leu
            420             425             430

Lys Asp Gln Arg Lys Cys Phe Ser Ile Asp Asn Pro Gly Tyr Glu Pro
        435             440             445

Glu Val Val Ala Val His Pro Gly Gly Asp Thr Val Ala Ile Gly Gly
    450             455             460

Val Asp Gly Asn Val Arg Leu Tyr Ser Ile Leu Gly Thr Thr Leu Lys
465             470             475             480

Asp Glu Gly Lys Leu Leu Glu Ala Lys Gly Pro Val Thr Asp Val Ala
            485             490             495

Tyr Ser His Asp Gly Ala Phe Leu Ala Val Cys Asp Ala Ser Lys Val
        500             505             510

Val Thr Val Phe Ser Val Ala Asp Gly Tyr Ser Glu Asn Asn Val Phe
        515             520             525

Tyr Gly His His Glu Lys
        530
```

```
<210>   38
<211>   511
<212>   PRT
<213>   Homo sapiens

<400>   38
```

Met Lys Phe Phe Leu Leu Leu Phe Thr Ile Gly Phe Cys Trp Ala Gln
1                   5                   10                  15

Tyr Ser Pro Asn Thr Gln Gln Gly Arg Thr Ser Ile Val His Leu Phe
                20                  25                  30

Glu Trp Arg Trp Val Asp Ile Ala Leu Glu Cys Glu Arg Tyr Leu Ala
            35                  40                  45

Pro Lys Gly Phe Gly Gly Val Gln Val Ser Pro Pro Asn Glu Asn Val
        50                  55                  60

Ala Ile His Asn Pro Phe Arg Pro Trp Trp Glu Arg Tyr Gln Pro Val
65                  70                  75                  80

Ser Tyr Lys Leu Cys Thr Arg Ser Gly Asn Glu Asp Glu Phe Arg Asn
                85                  90                  95

Met Val Thr Arg Cys Asn Asn Val Gly Val Arg Ile Tyr Val Asp Ala
            100                 105                 110

Val Ile Asn His Met Ser Gly Asn Ala Val Ser Ala Gly Thr Ser Ser
            115                 120                 125

Thr Cys Gly Ser Tyr Phe Asn Pro Gly Ser Arg Asp Phe Pro Ala Val
    130                 135                 140

Pro Tyr Ser Gly Trp Asp Phe Asn Asp Gly Lys Cys Lys Thr Gly Ser
145                 150                 155                 160

Gly Asp Ile Glu Asn Tyr Asn Asp Ala Thr Gln Val Arg Asp Cys Arg
                165                 170                 175

Leu Val Gly Leu Leu Asp Leu Ala Leu Glu Lys Asp Tyr Val Arg Ser
            180                 185                 190

Lys Ile Ala Glu Tyr Met Asn His Leu Ile Asp Ile Gly Val Ala Gly
            195                 200                 205

Phe Arg Leu Asp Ala Ser Lys His Met Trp Pro Gly Asp Ile Lys Ala
    210                 215                 220

Ile Leu Asp Lys Leu His Asn Leu Asn Ser Asn Trp Phe Pro Ala Gly
225                 230                 235                 240

Ser Lys Pro Phe Ile Tyr Gln Glu Val Ile Asp Leu Gly Gly Glu Pro
                245                 250                 255

```
Ile Lys Ser Ser Asp Tyr Phe Gly Asn Gly Arg Val Thr Glu Phe Lys
            260             265                 270

Tyr Gly Ala Lys Leu Gly Thr Val Ile Arg Lys Trp Asn Gly Glu Lys
        275             280                 285

Met Ser Tyr Leu Lys Asn Trp Gly Glu Gly Trp Gly Phe Met Pro Ser
    290             295                 300

Asp Arg Ala Leu Val Phe Val Asp Asn His Asp Asn Gln Arg Gly His
305             310                 315                 320

Gly Ala Gly Gly Ala Ser Ile Leu Thr Phe Trp Asp Ala Arg Leu Tyr
                325             330                 335

Lys Met Ala Val Gly Phe Met Leu Ala His Pro Tyr Gly Phe Thr Arg
        340             345                 350

Val Met Ser Ser Tyr Arg Trp Pro Arg Gln Phe Gln Asn Gly Asn Asp
        355             360                 365

Val Asn Asp Trp Val Gly Pro Pro Asn Asn Asn Gly Val Ile Lys Glu
    370             375                 380

Val Thr Ile Asn Pro Asp Thr Thr Cys Gly Asn Asp Trp Val Cys Glu
385             390                 395                 400

His Arg Trp Arg Gln Ile Arg Asn Met Val Asn Phe Arg Asn Val Val
            405             410                 415

Asp Gly Gln Pro Phe Thr Asn Trp Tyr Asp Asn Gly Ser Asn Gln Val
            420             425                 430

Ala Phe Gly Arg Gly Asn Arg Gly Phe Ile Val Phe Asn Asn Asp Asp
        435             440                 445

Trp Thr Phe Ser Leu Thr Leu Gln Thr Gly Leu Pro Ala Gly Thr Tyr
    450             455                 460

Cys Asp Val Ile Ser Gly Asp Lys Ile Asn Gly Asn Cys Thr Gly Ile
465             470                 475                 480

Lys Ile Tyr Val Ser Asp Asp Gly Lys Ala His Phe Ser Ile Ser Asn
            485             490                 495

Ser Ala Glu Asp Pro Phe Ile Ala Ile His Ala Glu Ser Lys Leu
            500             505                 510

<210>   39
<211>   511
<212>   PRT
```

<213>  Homo sapiens

<400>  39

Met Lys Phe Phe Leu Leu Leu Phe Thr Ile Gly Phe Cys Trp Ala Gln
1               5                   10                  15

Tyr Ser Pro Asn Thr Gln Gln Gly Arg Thr Ser Ile Val His Leu Phe
            20              25                  30

Glu Trp Arg Trp Val Asp Ile Ala Leu Glu Cys Glu Arg Tyr Leu Ala
        35              40                  45

Pro Lys Gly Phe Gly Gly Val Gln Val Ser Pro Pro Asn Glu Asn Val
    50              55                  60

Ala Ile Tyr Asn Pro Phe Arg Pro Trp Trp Glu Arg Tyr Gln Pro Val
65              70              75                  80

Ser Tyr Lys Leu Cys Thr Arg Ser Gly Asn Glu Asp Glu Phe Arg Asn
            85              90                  95

Met Val Thr Arg Cys Asn Asn Val Gly Val Arg Ile Tyr Val Asp Ala
            100             105                 110

Val Ile Asn His Met Cys Gly Asn Ala Val Ser Ala Gly Thr Ser Ser
            115             120             125

Thr Cys Gly Ser Tyr Phe Asn Pro Gly Ser Arg Asp Phe Pro Ala Val
    130             135             140

Pro Tyr Ser Gly Trp Asp Phe Asn Asp Gly Lys Cys Lys Thr Gly Ser
145             150             155                 160

Gly Asp Ile Glu Asn Tyr Asn Asp Ala Thr Gln Val Arg Asp Cys Arg
            165             170             175

Leu Thr Gly Leu Leu Asp Leu Ala Leu Glu Lys Asp Tyr Val Arg Ser
            180             185             190

Lys Ile Ala Glu Tyr Met Asn His Leu Ile Asp Ile Gly Val Ala Gly
            195             200             205

Phe Arg Leu Asp Ala Ser Lys His Met Trp Pro Gly Asp Ile Lys Ala
    210             215             220

Ile Leu Asp Lys Leu His Asn Leu Asn Ser Asn Trp Phe Pro Ala Gly
225             230             235             240

Ser Lys Pro Phe Ile Tyr Gln Glu Val Ile Asp Leu Gly Gly Glu Pro
            245             250             255

Ile Lys Ser Ser Asp Tyr Phe Gly Asn Gly Arg Val Thr Glu Phe Lys

260 265 270

Tyr Gly Ala Lys Leu Gly Thr Val Ile Arg Lys Trp Asn Gly Glu Lys
275 280 285

Met Ser Tyr Leu Lys Asn Trp Gly Glu Gly Trp Gly Phe Val Pro Ser
290 295 300

Asp Arg Ala Leu Val Phe Val Asp Asn His Asp Asn Gln Arg Gly His
305 310 315 320

Gly Ala Gly Gly Ala Ser Ile Leu Thr Phe Trp Asp Ala Arg Leu Tyr
325 330 335

Lys Met Ala Val Gly Phe Met Leu Ala His Pro Tyr Gly Phe Thr Arg
340 345 350

Val Met Ser Ser Tyr Arg Trp Pro Arg Gln Phe Gln Asn Gly Asn Asp
355 360 365

Val Asn Asp Trp Val Gly Pro Pro Asn Asn Asn Gly Val Ile Lys Glu
370 375 380

Val Thr Ile Asn Pro Asp Thr Thr Cys Gly Asn Asp Trp Val Cys Glu
385 390 395 400

His Arg Trp Arg Gln Ile Arg Asn Met Val Ile Phe Arg Asn Val Val
405 410 415

Asp Gly Gln Pro Phe Thr Asn Trp Tyr Asp Asn Gly Ser Asn Gln Val
420 425 430

Ala Phe Gly Arg Gly Asn Arg Gly Phe Ile Val Phe Asn Asn Asp Asp
435 440 445

Trp Ser Phe Ser Leu Thr Leu Gln Thr Gly Leu Pro Ala Gly Thr Tyr
450 455 460

Cys Asp Val Ile Ser Gly Asp Lys Ile Asn Gly Asn Cys Thr Gly Ile
465 470 475 480

Lys Ile Tyr Val Ser Asp Asp Gly Lys Ala His Phe Ser Ile Ser Asn
485 490 495

Ser Ala Glu Asp Pro Phe Ile Ala Ile His Ala Glu Ser Lys Leu
500 505 510

<210> 40
<211> 496
<212> PRT
<213> Homo sapiens

```
<220>
<221>  MISC_FEATURE
<222>  (1)..(1)
<223>  X can be any naturally occurring amino acid

<400>  40

Xaa Tyr Ser Ser Asn Thr Gln Gln Gly Arg Thr Ser Ile Val His Leu
1               5                   10                  15

Phe Glu Trp Arg Trp Val Asp Ile Ala Leu Glu Cys Glu Arg Tyr Leu
            20                  25                  30

Ala Pro Lys Gly Phe Gly Gly Val Gln Val Ser Pro Pro Asn Glu Asn
            35                  40                  45

Val Ala Ile His Asn Pro Phe Arg Pro Trp Trp Glu Arg Tyr Gln Pro
        50                  55                  60

Val Ser Tyr Lys Leu Cys Thr Arg Ser Gly Asn Glu Asp Glu Phe Arg
65                  70                  75                  80

Asn Met Val Thr Arg Cys Asn Asn Val Gly Val Arg Ile Tyr Val Asp
                85                  90                  95

Ala Val Ile Asn His Met Cys Gly Asn Ala Val Ser Ala Gly Thr Ser
                100                 105                 110

Ser Thr Cys Gly Ser Tyr Phe Asn Pro Gly Ser Arg Asp Phe Pro Ala
            115                 120                 125

Val Pro Tyr Ser Gly Trp Asp Phe Asn Asp Gly Lys Cys Lys Thr Gly
        130                 135                 140

Ser Gly Asp Ile Glu Asn Tyr Asn Asp Ala Thr Gln Val Arg Asp Cys
145                 150                 155                 160

Arg Leu Ser Gly Leu Leu Asp Leu Ala Leu Gly Lys Asp Tyr Val Arg
                165                 170                 175

Ser Lys Ile Ala Glu Tyr Met Asn His Leu Ile Asp Ile Gly Val Ala
            180                 185                 190

Gly Phe Arg Ile Asp Ala Ser Lys His Met Trp Pro Gly Asp Ile Lys
            195                 200                 205

Ala Ile Leu Asp Lys Leu His Asn Leu Asn Ser Asn Trp Phe Pro Glu
            210                 215                 220

Gly Ser Lys Pro Phe Ile Tyr Gln Glu Val Ile Asp Leu Gly Gly Glu
225                 230                 235                 240

Pro Ile Lys Ser Ser Asp Tyr Phe Gly Asn Gly Arg Val Thr Glu Phe
                245                 250                 255
```

Lys Tyr Gly Ala Lys Leu Gly Thr Val Ile Arg Lys Trp Asn Gly Glu
                260                 265                 270

Lys Met Ser Tyr Leu Lys Asn Trp Gly Glu Gly Trp Gly Phe Met Pro
        275                 280                 285

Ser Asp Arg Ala Leu Val Phe Val Asp Asn His Asp Asn Gln Arg Gly
    290                 295                 300

His Gly Ala Gly Gly Ala Ser Ile Leu Thr Phe Trp Asp Ala Arg Leu
305                 310                 315                 320

Tyr Lys Met Ala Val Gly Phe Met Leu Ala His Pro Tyr Gly Phe Thr
                325                 330                 335

Arg Val Met Ser Ser Tyr Arg Trp Pro Arg Tyr Phe Glu Asn Gly Lys
            340                 345                 350

Asp Val Asn Asp Trp Val Gly Pro Pro Asn Asp Asn Gly Val Thr Lys
            355                 360                 365

Glu Val Thr Ile Asn Pro Asp Thr Thr Cys Gly Asn Asp Trp Val Cys
    370                 375                 380

Glu His Arg Trp Arg Gln Ile Arg Asn Met Val Asn Phe Arg Asn Val
385                 390                 395                 400

Val Asp Gly Gln Pro Phe Thr Asn Trp Tyr Asp Asn Gly Ser Asn Gln
                405                 410                 415

Val Ala Phe Gly Arg Gly Asn Arg Gly Phe Ile Val Phe Asn Asn Asp
            420                 425                 430

Asp Trp Thr Phe Ser Leu Thr Leu Gln Thr Gly Leu Pro Ala Gly Thr
        435                 440                 445

Tyr Cys Asp Val Ile Ser Gly Asp Lys Ile Asn Gly Asn Cys Thr Gly
    450                 455                 460

Ile Lys Ile Tyr Val Ser Asp Asp Gly Lys Ala His Phe Ser Ile Ser
465                 470                 475                 480

Asn Ser Ala Glu Asp Pro Phe Ile Ala Ile His Ala Glu Ser Lys Leu
            485                 490                 495

<210> 41
<211> 249
<212> PRT
<213> Homo sapiens

<400> 41

```
Arg His Phe Trp Gln Gln Asp Glu Pro Pro Gln Ser Pro Trp Asp Arg
1               5                   10              15

Val Lys Asp Leu Ala Thr Val Tyr Val Asp Val Leu Lys Asp Ser Gly
            20              25              30

Arg Asp Tyr Val Ser Gln Phe Glu Gly Ser Ala Leu Gly Lys Gln Leu
        35              40              45

Asn Leu Lys Leu Leu Asp Asn Trp Asp Ser Val Thr Ser Thr Phe Ser
    50              55              60

Lys Leu Arg Glu Gln Leu Gly Pro Val Thr Gln Glu Phe Trp Asp Asn
65              70              75              80

Leu Glu Lys Glu Thr Glu Gly Leu Arg Gln Glu Met Ser Lys Asp Leu
                85              90              95

Glu Glu Val Lys Ala Lys Val Gln Pro Tyr Leu Asp Asp Phe Gln Lys
            100             105             110

Lys Trp Gln Glu Glu Met Glu Leu Tyr Arg Gln Lys Val Glu Pro Leu
        115             120             125

Arg Ala Glu Leu Gln Glu Gly Ala Arg Gln Lys Leu His Glu Leu Gln
    130             135             140

Glu Lys Leu Ser Pro Leu Gly Glu Glu Met Arg Asp Arg Ala Arg Ala
145             150             155             160

His Val Asp Ala Leu Arg Thr His Leu Ala Pro Tyr Ser Asp Glu Leu
            165             170             175

Arg Gln Arg Leu Ala Ala Arg Leu Glu Ala Leu Lys Glu Asn Gly Gly
        180             185             190

Ala Arg Leu Ala Glu Tyr His Ala Lys Ala Thr Glu His Leu Ser Thr
        195             200             205

Leu Ser Glu Lys Ala Lys Pro Ala Leu Glu Asp Leu Arg Gln Gly Leu
    210             215             220

Leu Pro Val Leu Glu Ser Phe Lys Val Ser Phe Leu Ser Ala Leu Glu
225             230             235             240

Glu Tyr Thr Lys Lys Leu Asn Thr Gln
                245
```

```
<210>   42
<211>   285
<212>   PRT
<213>   Homo sapiens
```

<400> 42

```
Ser Thr Gln Asp Glu Val Ala Ala Ser Ala Ile Leu Thr Ala Gln Leu
1               5               10              15

Asp Glu Glu Leu Gly Gly Thr Pro Val Gln Ser Arg Val Val Gln Gly
            20              25              30

Lys Glu Pro Ala His Leu Met Ser Leu Phe Gly Gly Lys Pro Met Ile
        35              40              45

Ile Tyr Lys Gly Gly Thr Ser Arg Glu Gly Gly Gln Thr Ala Pro Ala
    50              55              60

Ser Thr Arg Leu Phe Gln Val Arg Ala Asn Ser Ala Gly Ala Thr Arg
65              70              75              80

Ala Val Glu Val Leu Pro Lys Ala Gly Ala Leu Asn Ser Asn Asp Ala
            85              90              95

Phe Val Leu Lys Thr Pro Ser Ala Ala Tyr Leu Trp Val Gly Thr Gly
            100             105             110

Ala Ser Glu Ala Glu Lys Thr Gly Ala Gln Glu Leu Leu Arg Val Leu
        115             120             125

Arg Ala Gln Pro Val Gln Val Ala Glu Gly Ser Glu Pro Asp Gly Phe
    130             135             140

Trp Glu Ala Leu Gly Gly Lys Ala Ala Tyr Arg Thr Ser Pro Arg Leu
145             150             155             160

Lys Asp Lys Lys Met Asp Ala His Pro Pro Arg Leu Phe Ala Cys Ser
            165             170             175

Asn Lys Ile Gly Arg Phe Val Ile Glu Glu Val Pro Gly Glu Leu Met
            180             185             190

Gln Glu Asp Leu Ala Thr Asp Asp Val Met Leu Leu Asp Thr Trp Asp
        195             200             205

Gln Val Phe Val Trp Val Gly Lys Asp Ser Gln Glu Glu Glu Lys Thr
    210             215             220

Glu Ala Leu Thr Ser Ala Lys Arg Tyr Ile Glu Thr Asp Pro Ala Asn
225             230             235             240

Arg Asp Arg Arg Thr Pro Ile Thr Val Val Lys Gln Gly Phe Glu Pro
            245             250             255

Pro Ser Phe Val Gly Trp Phe Leu Gly Trp Asp Asp Asp Tyr Trp Ser
            260             265             270
```

Val Asp Pro Leu Asp Arg Ala Met Ala Glu Leu Ala Ala
        275             280                 285


<210> 43
<211> 782
<212> PRT
<213> Homo sapiens

<400> 43

Met Ala Pro His Arg Pro Ala Pro Ala Leu Leu Cys Ala Leu Ser Leu
1               5                   10                  15

Ala Leu Cys Ala Leu Ser Leu Pro Val Arg Ala Ala Thr Ala Ser Arg
            20                  25                  30

Gly Ala Ser Gln Ala Gly Ala Pro Gln Gly Arg Val Pro Glu Ala Arg
            35                  40                  45

Pro Asn Ser Met Val Val Glu His Pro Glu Phe Leu Lys Ala Gly Lys
        50                  55                  60

Glu Pro Gly Leu Gln Ile Trp Arg Val Glu Lys Phe Asp Leu Val Pro
65                  70                  75                  80

Val Pro Thr Asn Leu Tyr Gly Asp Phe Phe Thr Gly Asp Ala Tyr Val
                85                  90                  95

Ile Leu Lys Thr Val Gln Leu Arg Asn Gly Asn Leu Gln Tyr Asp Leu
            100                 105                 110

His Tyr Trp Leu Gly Asn Glu Cys Ser Gln Asp Glu Ser Gly Ala Ala
            115                 120                 125

Ala Ile Phe Thr Val Gln Leu Asp Asp Tyr Leu Asn Gly Arg Ala Val
        130                 135                 140

Gln His Arg Glu Val Gln Gly Phe Glu Ser Ala Thr Phe Leu Gly Tyr
145                 150                 155                 160

Phe Lys Ser Gly Leu Lys Tyr Lys Lys Gly Gly Val Ala Ser Gly Phe
                165                 170                 175

Lys His Val Val Pro Asn Glu Val Val Val Gln Arg Leu Phe Gln Val
            180                 185                 190

Lys Gly Arg Arg Val Val Arg Ala Thr Glu Val Pro Val Ser Trp Glu
            195                 200                 205

Ser Phe Asn Asn Gly Asp Cys Phe Ile Leu Asp Leu Gly Asn Asn Ile
        210                 215                 220

His Gln Trp Cys Gly Ser Asn Ser Asn Arg Tyr Glu Arg Leu Lys Ala

```
        225                  230                  235                  240

Thr Gln Val Ser Lys Gly Ile Arg Asp Asn Glu Arg Ser Gly Arg Ala
                245                  250                  255

Arg Val His Val Ser Glu Glu Gly Thr Glu Pro Glu Ala Met Leu Gln
            260                  265                  270

Val Leu Gly Pro Lys Pro Ala Leu Pro Ala Gly Thr Glu Asp Thr Ala
            275                  280                  285

Lys Glu Asp Ala Ala Asn Arg Lys Leu Ala Lys Leu Tyr Lys Val Ser
    290                  295                  300

Asn Gly Ala Gly Thr Met Ser Val Ser Leu Val Ala Asp Glu Asn Pro
305                  310                  315                  320

Phe Ala Gln Gly Ala Leu Lys Ser Glu Asp Cys Phe Ile Leu Asp His
                325                  330                  335

Gly Lys Asp Gly Lys Ile Phe Val Trp Lys Gly Lys Gln Ala Asn Thr
                340                  345                  350

Glu Glu Arg Lys Ala Ala Leu Lys Thr Ala Ser Asp Phe Ile Thr Lys
            355                  360                  365

Met Asp Tyr Pro Lys Gln Thr Gln Val Ser Val Leu Pro Glu Gly Gly
        370                  375                  380

Glu Thr Pro Leu Phe Lys Gln Phe Phe Lys Asn Trp Arg Asp Pro Asp
385                  390                  395                  400

Gln Thr Asp Gly Leu Gly Leu Ser Tyr Leu Ser Ser His Ile Ala Asn
                405                  410                  415

Val Glu Arg Val Pro Phe Asp Ala Ala Thr Leu His Thr Ser Thr Ala
            420                  425                  430

Met Ala Ala Gln His Gly Met Asp Asp Asp Gly Thr Gly Gln Lys Gln
            435                  440                  445

Ile Trp Arg Ile Glu Gly Ser Asn Lys Val Pro Val Asp Pro Ala Thr
    450                  455                  460

Tyr Gly Gln Phe Tyr Gly Gly Asp Ser Tyr Ile Ile Leu Tyr Asn Tyr
465                  470                  475                  480

Arg His Gly Gly Arg Gln Gly Gln Ile Ile Tyr Asn Trp Gln Gly Ala
                485                  490                  495

Gln Ser Thr Gln Asp Glu Val Ala Ala Ser Ala Ile Leu Thr Ala Gln
        500                  505                  510
```

Leu Asp Glu Glu Leu Gly Gly Thr Pro Val Gln Ser Arg Val Val Gln
        515                 520             525

Gly Lys Glu Pro Ala His Leu Met Ser Leu Phe Gly Gly Lys Pro Met
    530             535             540

Ile Ile Tyr Lys Gly Gly Thr Ser Arg Glu Gly Gly Gln Thr Ala Pro
545             550             555             560

Ala Ser Thr Arg Leu Phe Gln Val Arg Ala Asn Ser Ala Gly Ala Thr
            565             570             575

Arg Ala Val Glu Val Leu Pro Lys Ala Gly Ala Leu Asn Ser Asn Asp
        580             585             590

Ala Phe Val Leu Lys Thr Pro Ser Ala Ala Tyr Leu Trp Val Gly Thr
        595             600             605

Gly Ala Ser Glu Ala Glu Lys Thr Gly Ala Gln Glu Leu Leu Arg Val
    610             615             620

Leu Arg Ala Gln Pro Val Gln Val Ala Glu Gly Ser Glu Pro Asp Gly
625             630             635             640

Phe Trp Glu Ala Leu Gly Gly Lys Ala Ala Tyr Arg Thr Ser Pro Arg
            645             650             655

Leu Lys Asp Lys Lys Met Asp Ala His Pro Pro Arg Leu Phe Ala Cys
            660             665             670

Ser Asn Lys Ile Gly Arg Phe Val Ile Glu Glu Val Pro Gly Glu Leu
        675             680             685

Met Gln Glu Asp Leu Ala Thr Asp Asp Val Met Leu Leu Asp Thr Trp
    690             695             700

Asp Gln Val Phe Val Trp Val Gly Lys Asp Ser Gln Glu Glu Glu Lys
705             710             715             720

Thr Glu Ala Leu Thr Ser Ala Lys Arg Tyr Ile Glu Thr Asp Pro Ala
            725             730             735

Asn Arg Asp Arg Arg Thr Pro Ile Thr Val Val Lys Gln Gly Phe Glu
            740             745             750

Pro Pro Ser Phe Val Gly Trp Phe Leu Gly Trp Asp Asp Asp Tyr Trp
            755             760             765

Ser Val Asp Pro Leu Asp Arg Ala Met Ala Glu Leu Ala Ala
    770             775             780

94

```
<210>  44
<211>  182
<212>  PRT
<213>  Homo sapiens

<400>  44

Glu Arg Asp Cys Arg Val Ser Ser Phe Arg Val Lys Glu Asn Phe Asp
1               5               10              15

Lys Ala Arg Phe Ser Gly Thr Trp Tyr Ala Met Ala Lys Lys Asp Pro
            20              25              30

Glu Gly Leu Phe Leu Gln Asp Asn Ile Val Ala Glu Phe Ser Val Asp
        35              40              45

Glu Thr Gly Gln Met Ser Ala Thr Ala Lys Gly Arg Val Arg Leu Leu
    50              55              60

Asn Asn Trp Asp Val Cys Ala Asp Met Val Gly Thr Phe Thr Asp Thr
65              70              75              80

Glu Asp Pro Ala Lys Phe Lys Met Lys Tyr Trp Gly Val Ala Ser Phe
            85              90              95

Leu Gln Lys Gly Asn Asp Asp His Trp Ile Val Asp Thr Asp Tyr Asp
            100             105             110

Thr Tyr Ala Val Gln Tyr Ser Cys Arg Leu Leu Asn Leu Asp Gly Thr
        115             120             125

Cys Ala Asp Ser Tyr Ser Phe Val Phe Ser Arg Asp Pro Asn Gly Leu
        130             135             140

Pro Pro Glu Ala Gln Lys Ile Val Arg Gln Arg Gln Glu Glu Leu Cys
145             150             155             160

Leu Ala Arg Gln Tyr Arg Leu Ile Val His Asn Gly Tyr Cys Asp Gly
                165             170             175

Arg Ser Glu Arg Asn Leu
            180
```